(19) Europäisches Patentamt
European Patent Office
Office européen des brevets

(11) EP 1 642 970 A1

(12) # EUROPEAN PATENT APPLICATION

published in accordance with Art. 158(3) EPC

(43) Date of publication:
**05.04.2006 Bulletin 2006/14**

(21) Application number: **04747556.1**

(22) Date of filing: **08.07.2004**

(51) Int Cl.:
***C12N 15/09*** *(1990.01)* ***C12N 5/10*** *(1990.01)*
***A01K 67/027*** *(1990.01)*

(86) International application number:
**PCT/JP2004/010090**

(87) International publication number:
**WO 2005/003342 (13.01.2005 Gazette 2005/02)**

(84) Designated Contracting States:
**AT BE BG CH CY CZ DE DK EE ES FI FR GB GR HU IE IT LI LU MC NL PL PT RO SE SI SK TR**

(30) Priority: **08.07.2003 WOPCT/JP03/08681**

(71) Applicant: **Japan Science and Technology Agency**
**Kawaguchi-shi,**
**Saitama 332-0012 (JP)**

(72) Inventors:
- **TAKEDA, Junji,**
  **Osaka University**
  **2-2, Yamadaoka, Suita-shi, Osaka 5650871 (JP)**
- **HORIE, Kyoji,**
  **Osaka University**
  **Suita-shi, Osaka 5650871 (JP)**

(74) Representative: **Bates, Philip Ian et al**
**Reddie & Grose**
**16 Theobalds Road**
**London WC1X 8PL (GB)**

(54) **METHOD OF PREPARING TRANSGENIC ORGANISM WITH USE OF METHYLATION AND SYSTEM THEREFOR**

(57) A technique for efficiently introducing a foreign gene into cells with the use of transposons. In particular, a technique for efficiently preparing a transgenic organism with the use of a transposon having its transposition activity strikingly enhanced through methylation of a sequence containing the transposon. The methylation is retained even after incorporation in a genome, and now can be utilized in actual gene incorporation in a genome. This technique can realize strikingly efficient gene transformation as compared with the a method of preparing a transgenic organism with the use of conventional transposons.

EP 1 642 970 A1

**Description**

TECHNICAL FIELD

The present invention relates to a system, a kit and a composition for introducing a foreign nucleic acid into a cell. More specifically, the present invention relates to the production of a transgenic organism and a composition, a kit and a system therefor.

BACKGROUND ART

Transgenic organisms are an important technology, which is currently of note at present, due to its broad range of applications. However, methods for efficiently producing transgenic organisms are not yet well developed, and therefore, development of such methods for efficiently producing transgenic organisms are of note.

Recently, production of transgenic biological organisms using transposons has been attempted. A transposon (or alternatively called "transposable element") is a nucleic acid molecule or sequence with repeated sequences in parallel. Transposase is an enzyme promoting integration of a different nucleic acid into a nucleic acid molecule. Usually, the sequence for transposase exists in the transposon.

Transposons are found in a relatively broad range of biological organisms, and the phenomenon attained thereby is believed to be ubiquitous. Transposition is achieved by cut-and-paste mechanism. Transposons have also been found in vertebrates (Radice, A.D. et al., 1994.Mol. Gen. Genet. 244,606-612). A number of transposons have been isolated from a variety of organisms such as fish, amphibia, mammals and the like, including those belonging to the super families Tc1/mariner hAT (hobo/Ac/Tam) and the like (Oosumi et al . , 1995. Nature 378, 873; Ivics et al., 1995. Mol. Gen. Genet. 247, 312-322; Koga et al. 1996. Nature 383, 30; Lam et al., 1996. J. Mol. Biol. 257,359-366, and Lam, W. L. et al., Proc. Natl. Acad. Sci. USA 93, 10870-10875). Transposases are known to catalyze or facilitate excision from the original location of a transposon and reintegration thereof (Plasterk, RHA., 1999, TIG 15: 326-332; Plasterk RHA., 1996 Curr. Top. Microbiol. Immunol. 204, 125-143). An autonomous element of transposons can express active transposases, which are a transacting factor, and thus have the property of the transposon per se and can also transpose. A non autonomous element may be a cis-acting element, and this may be called as inverted terminal repeat sequence. Some inverted repeat sequences may include one or more tandem repeat sequences. Such a sequence is included in a terminal inverted repeat sequence and may be used for transposition from another element in the presence of a complementary transposase.

Such a system has been used to attempt introduction of a foreign gene into a variety of organisms.

In plants, transposable elements such as Ac/Ds, Spm superfamilies and the like have been routinely used (Osborne and Baker, 1995 Curr. Opin. Cell Biol. 7, 406-413). Recently, attempts have been made in animals, however, such elements have species specificity, and thus it is said that very few attempts have been successful. Use of P element from Drosophila melanogaster for genetic transformation of a cell of a non-Drosophila insect, Zebra fish, mammals and the like, has not been successful to date (Handler et al., 1993. Arch. Insect Biochem. Physiol. 22, 373-384; Gibbs et al. Mol. Mar. Biol. Biotech. 3, 317-326; and Rio et al . , 1988 J. Mol. Biol. 200, 411-415). The Tc1/mariner superfamily member has little species specificity and thus is of note today, and has been tried in mammals, such as a human. Minos and TcE have also been attempted for such applications.

Sleeping Beauty (SB) has been specified as having an activity for promoting transposition of a salmon type Tc1 like transposon using molecular lineage classification data. A transposase gene common sequence has been deduced from eight types of inactive elements of the salmon subfamily elements from fish, and engineered to eliminate the mutations responsible for inactivation. The transposase has been constructed to identify its functional domain, and tested individually with respect to the biochemical functions thereof and from the points of the full length transposase. The transposase binds to two binding sites in an inverted repeat sequence of the salmon element and has substrate specificity, and thus allows prevention from cross-transposition between fish element subfamily members in a closely related species. SB transposase enhances not only fish, but also significantly enhances chromosomal integration of transposons in murine and human cells. The requirement for a specific motif in a transposase and specific sequences in a target transposon have established SB transposase as the first active DNA transposon system for insertion mutagenesis, in addition to activity in fish and mammalian cells and the like. In an aspect of the present invention, the present invention is related to a nucleic acid fragment, which comprises at least a nucleic acid sequence located between two inverted repeat sequences, and the inverted repeat sequences can bind to SB protein, and the nucleic acid fragment can be integrated into the DNA of the cell. In an embodiment, the cells of the present invention may be obtained from, for example, invertebrate or vertebrate. Preferable embodiments of invertebrates include, but are not limited to, for example, shrimps, scallop, lobsters, clam oysters and the like. Preferable embodiments of vertebrates include fish, birds, and mammals, including mice, Ungulata, sheep, pigs, humans and the like. Cellular DNA may be of a cellular genome or epichromosomal DNA, including episomes, plasmids and the like.

A method for introducing DNA into a cell is known, and includes for example, DNA aggregation reagents (e.g. calcium phosphate, polyethylene glycol and the like), lipid-containing reagents (e.g., liposome, multi-lamellar vesicle and the like) , and virus mediated methods, and the like. These methods have their own deficiencies. For example, DNA aggregation reagents and virus mediated methods, have deficiencies where the size of the DNA is limited, and the amount of nucleic acids to be introduced is limited. The promotion of integration of the transgene is not always good.

There is still a demand for a method for introducing DNA into a cell, in particular, efficient integration of a nucleic acid fragment of a variety of sizes into the nucleic acid of a cell, specifically a method for promoting integration of DNA into the genome of a cell.

Z. Ivics et al. (Cell, 91, 501-510 (1997)) reported the expression of a transposon in a cultured cell, in which no effect of the transposon system had been confirmed in an adult mammalian individual or an organ thereof or the like. For animal cells, a mariner transposon has been isolated from Drosophila mauritiana, which is a subspecies of fruit fly. This mariner transposon was used to construct a vector.

For example, it has been attempted to incorporate a P-element transposon of Drosophila melanogaster into chromosomal DNA of various heterologous organisms. However, the function of the P-element transposon was not maintained due to species specificity. In experiments using flies, such as Muscidae, Sphaeroceridae, Phoridae, or the like, the transposition activity of the P-element was not maintained (Handler et al. ; Arch. Insect Biochem. Physiol., 22: 373-384 (1993) ) . In the case of a transgenic zebra fish having an incorporated P-element and reporter gene, genetic expression was not stably obtained (Gibbs et al.; Mol. Mar. Biol. Biotech., 3:317-326 (1994)) . It is known that when Tc1/mariner transposons, which are the most studied eukaryotic transposons, are used in heterologous organisms, transposition is likely to occur since the species specificity of these transposons is relatively low (Z. Ivics et al. ; Cell, 91: 501-510 (1997) ) . An example of a transposon system comprising a transposon, which is reconstructed from such a Tc1/mariner-like transposon, and a transposase, is the above-described Sleeping Beauty (SB) transposon system comprising an SB transposon and an SB transposase. The following examples have been reported: the SB transposon was introduced into human HeLa cells and mouse LMTK cells (Z. Ivics et al.; Cell, 91:501-510 (1997) ) ; the SB transposon was introduced into mouse embryonic stem (ES) cells (G. Luo et al . ; Proc. Natl. Acad. Sci. USA, 95:10769-10773 (1998)); the activity of a Caenorhabditis elegans-derived Tc1 transposon, which was introduced into human cultured cells, was observed (G. Schouten et al.; Nucleic Acids Res., 26:3013-3017 (1998)). However, for example, in the case of the above-described example in which the SB transposon was introduced into the mouse ES cells, the frequency of transposition of the transposon was $3.5 \times 10^{-5}$ per cell, having the introduced exogenous gene at the maximum, which is considerably low. In this case, a large amount of cells had to be used in order to obtain a desired cell. The example where the SB transposon was introduced into human HeLa cells cannot be applied to animal individuals. For introduction of transposons into mammals, mice having a transposed transposon have been reported, in which the SB transposon and an SB transposase gene were introduced into the genome of somatic cells via the blood (S.R. Yant et al. ; Nature Genetics, 25:35-41 (2000)). In this case, however, the transposition frequency of the transposon was as low as about 5 to 6% of the liver cells having the introduced gene. This method has such a poor efficiency for gene introduction that a transgenic animal line could not be obtained. In the case of conventional techniques, it is difficult to randomly introduce mutations into a number of genes of an animal individual in vivo. The expression frequency is also low. Therefore, it was necessary to design a general method for mutagenesis of genes.

Accordingly, as mentioned above, when using such SB system to introduce a gene at the cell level, there are many cases where successful transformation has not been achieved, and there is a need for increasing transformation efficiency.

As such, it is an object of the present invention to develop a transposon system to increase transformation efficiency and to efficiently achieve production of a transgenic biological organism.

## SUMMARY OF INVENTION

The above-mentioned object has been achieved by, in part, the unexpected discovery by the present inventors as a result of study and efforts, that methylation of at least a portion of a nucleic acid sequence to be introduced increases transformation efficiency, in view of the above-mentioned background.

The present invention is related to the efficient introduction of a foreign gene into a cell by means of a transposon. In particular, the present invention is related to technology for efficiently producing a transgenic biological organism by methylating a sequence comprising a transposon, thereby surprisingly improving the transposition activity of the transposon. The methylation has been maintained after the integration into the genome, thereby allowing one to use it to identify the actual integration of the gene into the genome. The use of the present invention allows extremely efficient transformation of a gene, more than that which can be achieved with conventional methods for producing a transgenic biological organism using a conventional transposon.

Accordingly, the present invention provides the following:

1. An isolated nucleic acid molecule having a nucleic acid sequence encoding a transposon, wherein the nucleic acid sequence has a methylation at at least one nucleotide.

2. An isolated nucleic acid molecule according to Item 1, further comprising a nucleic acid molecule encoding a desired gene.

3. An isolated nucleic acid molecule according to Item 1, wherein said methylation is present at least C in a CG sequence.

4. An isolated nucleic acid molecule according to Item 1, wherein said transposon is of a DNA-type.

5. An isolated nucleic acid molecule according to Item 1, wherein said transposon belongs to Tc1/mariner types.

6. An isolated nucleic acid molecule according to Item 1, wherein said transposon comprises *Sleeping Beauty.*

7. An isolated nucleic acid molecule according to Item 2, wherein said desired gene is operably linked to said transposon, or is capable of being operably linked to said transposon when intracellularly introduced.

8. An isolated nucleic acid molecule according to Item 1 for use in introducing a foreign gene into a host.

9. An isolated nucleic acid molecule according to Item 8, wherein said host comprises a eukaryote.

10. An isolated nucleic acid molecule according to Item 8, wherein said host comprises a mammal.

11. An isolated nucleic acid molecule according to Item 8, wherein said host comprises a rodent.

12. An isolated nucleic acid molecule according to Item 1, wherein a transposase functions at a location on a genome to which said nucleic acid molecule is inserted.

13. A gene cassette having a nucleic acid sequence encoding a transposon, wherein said nucleic acid sequence has a methylation at at least one nucleotide.

14. A vector having a nucleic acid sequence encoding a transposon and a nucleic acid sequence encoding a desired gene, wherein said nucleic acid sequence has a methylation at at least one nucleotide.

15. A vector according to Item 14, wherein said methylation is present at least C in a CG sequence.

16. A vector according to Item 14, wherein said transposon is of a DNA-type.

17. A vector according to Item 14, wherein said transposon belongs to Tc1/mariner types.

18. A vector according to Item 14, wherein said transposon comprises *Sleeping Beauty.*

19. A vector according to Item 14, wherein said desired gene is operably linked to said transposon, or is capable of being operably linked to said transposon when intracellularly introduced.

20. A vector according to Item 14 for use in introducing a foreign gene into a host.

21. A vector according to Item 20, wherein said host comprises a eukaryote.

22. A vector according to Item 20, wherein said host comprises a mammal.

23. A vector according to Item 20, wherein said host comprises a rodent.

24. A vector according to Item 14, wherein a transposase functions at a location on a genome to which said nucleic acid molecule is inserted.

25. A composition for <u>rendering a transposase to act on</u> a foreign nucleic acid molecule to be inserted in a genome,

wherein said composition comprises a nucleic acid sequence encoding a transposon and a nucleic acid sequence encoding a desired gene, wherein said nucleic acid sequence has a methylation at at least one nucleotide.

26. A cell comprising a nucleic acid sequence encoding a transposon and a nucleic acid sequence encoding a desired gene, wherein said nucleic acid sequence has a methylation at at least one nucleotide.

27. A cell according to Item 26, wherein said methylation is present at least C in a CG sequence.

28. A cell according to Item 26, wherein said transposon is of a DNA-type.

29. A cell according to Item 26, wherein said transposon belongs to Tc1/mariner types.

30. A cell according to Item 26, wherein said transposon comprises *Sleeping Beauty.*

31. A cell according to Item 26, wherein said desired gene is operably linked to said transposon.

32. A cell according to Item 26 for use in introducing a foreign gene into a host.

33. A cell according to Item 26, wherein said host comprises a eukaryote.

34. A cell according to Item 26, wherein said host comprises a mammal.

35. A cell according to Item 26, wherein said host comprises a rodent.

36. A tissue comprising a nucleic acid sequence encoding a transposon and a nucleic acid sequence encoding a desired gene, wherein said nucleic acid sequence has a methylation at at least one nucleotide.

37. A tissue according to Item 36, wherein said methylation is present at least C in a CG sequence.

38. A tissue according to Item 36, wherein said transposon is of a DNA-type.

39. A tissue according to Item 36, wherein said transposon belongs to Tc1/mariner types.

40. A tissue according to Item 36, wherein said transposon comprises *Sleeping Beauty.*

41. A tissue according to Item 36, wherein said desired gene is operably linked to said transposon.

42. A tissue according to Item 36 for use in introducing a foreign gene into a host.

43. A tissue according to Item 42, wherein said tissue comprises a eukaryotic tissue.

44. A tissue according to Item 42, wherein said tissue comprises a mammalian tissue.

45. A tissue according to Item 42, wherein said tissue comprises a rodent tissue.

46. A biological organism comprising a nucleic acid sequence encoding a transposon and a nucleic acid sequence encoding a desired gene, wherein said nucleic acid sequence has a methylation at at least one nucleotide.

47. A biological organism according to Item 46, wherein said methylation is present at least C in a CG sequence.

48. A biological organism according to Item 46, wherein said transposon is of a DNA-type.

49. A biological organism according to Item 46, wherein said transposon belongs to Tc1/marine types.

50. A biological organism according to Item 46,
wherein said transposon comprises *Sleeping Beauty.*

51. A biological organism according to Item 46, wherein said desired gene is operably linked to said transposon.

52. A biological organism according to Item 46, which comprises a eukaryote.

53. A biological organism according to Item 46, which comprises a mammal.

54. A biological organism according to Item 46, which comprises a rodent.

55. A biological organism according to Item 46, wherein said desired gene is not derived from said biological organism.

56. A method for producing a transgenic biological organism, comprising the steps of:

A) providing an isolated nucleic acid molecule having a nucleic acid sequence encoding a transposon;
B) transforming a germ cell of a desired biological organism with said nucleic acid molecule;
C) selecting an individual in which the germ cell has a methylation in a nucleic acid sequence encoding said transposon;
D) regenerating a biological organism using the transformed germ cell.

57. A method according to Item 56, wherein said biological organism comprises a eukaryote.

58. A method according to Item 56, wherein said biological organism comprises a mammal.

59. A method for producing a transgenic biological organism comprising the steps of:

A) providing an isolated nucleic acid molecule having a nucleic acid sequence encoding a transposon, wherein the nucleic acid sequence has a methylation at at least one nucleotide;
B) transforming a germ cell of a desired biological organism with said nucleic acid molecule;
C) regenerating a biological organism using the transformed germ cell.

60. A method according to Item 59, wherein said biological organism comprises a eukaryote.

61. A method according to Item 59, wherein said biological organism comprises a mammal.

62. A method according to Item 59, wherein said biological organism comprises a rodent.

63. A kit for producing a transgenic biological organism, comprising the steps of:

A) an isolated nucleic acid molecule having a nucleic acid sequence encoding a transposon, wherein the nucleic acid sequence has a methylation at at least one nucleotide; and
B) a transposase.

64. A kit according to Item 63, further comprising instructions indicating a method of use of said nucleic acid molecule and transposase.

65. Use of an isolated nucleic acid molecule having a nucleic acid sequence encoding a transposon, wherein the nucleic acid sequence has a methylation at at least one nucleotide for producing a transgenic biological organism.

66. A nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated.

67. A nucleic acid fragment according to Item 66, wherein the nucleic acid sequence comprises at least a portion of a foreign gene.

68. A nucleic acid fragment according to Item 66, wherein said nucleic acid sequence comprises at least one expression controlling region.

69. A nucleic acid fragment according to Item 68, wherein the expression controlling region is selected from the group consisting of a promoter, enhancer and silencer.

70. A nucleic acid fragment according to Item 66, further comprising at least a portion of a foreign gene, wherein the nucleic acid sequence is operably linked to a sequence encoding at least a portion of the foreign gene.

71. A nucleic acid fragment according to Item 66, wherein said cell is derived from an animal.

72. A nucleic acid fragment according to Item 71, wherein said cell is obtained from a vertebrate.

73. A nucleic acid fragment according to Item 72, wherein the vertebrate is a mammal.

74. A nucleic acid fragment according to Item 73, wherein the mammal is a primate or a rodent.

75. A nucleic acid fragment according to Item 66, the DNA of the cell is selected from the group consisting of a cellular genome, episome and plasmid.

76. A nucleic acid fragment according to Item 66, wherein said at least one inverted repeat sequence comprises the sequence set forth in SEQ ID NO: 20 or 21, or a portion thereof.

77. A nucleic acid fragment according to Item 66, wherein said transposase is SB protein.

78. A nucleic acid fragment according to Item 77, wherein the transposase has at least 80 % amino acid homology to the sequence set forth in SEQ ID NO: 3.

79. A nucleic acid fragment according to Item 66, wherein said at least one inverted repeat sequence comprises at least one tandem repeat sequence, and the tandem repeat sequence comprises a nucleotide sequence set forth in SEQ ID NO: 26 or that having at least 80% homology to the sequence set forth in SEQ ID NO: 26.

80. A nucleic acid fragment according to Item 66, wherein said at least one inverted repeat sequence comprises at least one tandem repeat sequence, wherein the tandem repeat sequence is selected from the group consisting of nucleic acid sequences set forth in SEQ ID NOs: 22-25.

81. A nucleic acid introduction system for introducing into a DNA of a cell another DNA, the system comprising:

A) a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; and
B) a transposase or a nucleic acid encoding a transposase.

82. A nucleic acid introduction system according to Item 81, therein the transposase is SB protein.

83. A nucleic acid introduction system according to Item 81, wherein said transposase has the amino acid sequence set forth in SEQ ID NO:3 or a variant thereof, or the nucleic acid sequence encoding the transposase has the nucleic acid sequence set forth in SEQ ID NO: 2 or a variant thereof.

84. A nucleic acid introduction system according to Item 81, wherein said nucleic acid encoding the transposase is incorporated into the cellular genome.

85. A nucleic acid introduction system according to Item 81, further comprising a plasmid or a virus vector, wherein said plasmid or virus vector comprises the nucleic acid fragment as a part thereof.

86. A nucleic acid introduction system according to Item 81, wherein nucleic acid fragment comprises at least a portion of a sequence encoding a foreign gene.

87. A nucleic acid introduction system according to Item 81, wherein the nucleic acid fragment is introduced into the cell by means of a method selected from the group consisting of particle bombardment; electroporation; microinjection; use of a gene introduction reagent; and a use of virus vector.

88. A method for producing a transgenic biological organism, comprising the steps of:

introducing a nucleic acid fragment and transposase into a pluripotent cell, wherein the nucleic acid fragment comprises a nucleic acid sequence located between at least two inverted repeat sequences, wherein the inverted repeat sequence have the capability of binding to a transposase, wherein the nucleic acid fragment is capable of being incorporated into a DNA in a cell, wherein at least one nucleotide thereof is methylated; and
growing the cell into a living body.

89. A method according to Item 88, wherein said pluripotent cell is selected from the group consisting of an oocyte, an embryo, an egg and a stem cell.

90. A method according to Item 88, wherein said biological organism is a rodent or a primate.

91. A method according to Item 89, wherein said biological organism is a mouse or a rat.

92. A method for introducing a nucleic acid into a DNA of a cell, comprising the steps of:

introducing a nucleic acid fragment into a cell, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated.

93. A method according to Item 92, further comprising the step of introducing a transposase into the cell.

94. A method according to Item 92, wherein the transposase has at least 80 % homology to the sequence set forth in SEQ ID NO: 3.

95. A method according to Item 92, wherein said cell comprises a nucleic acid encoding the transposase.

96. A method according to Item 95, wherein the nucleic acid encoding the transposase is incorporated into the cellular genome.

97. A method according to Item 95, wherein the transposase is stably expressed in the cell.

98. A method according to Item 95, wherein the transposase is operably linked such that it is under the control of an inducible promoter.

99. A method according to Item 92, wherein the nucleic acid sequence encodes a protein.

100. A method according to Item 92, wherein the nucleic acid sequence encodes a marker protein.

101. A method for tranposing a nucleic acid sequence in a cell, comprising the steps of:

introducing a transposase into a cell comprising a nucleic acid fragment, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated;
wherein the transposase transposes the nucleic acid sequence from a first location in a DNA of the cell to a second location of the DNA.

102. A method according to Item 101, wherein the DNA of the cell is a genomic DNA.

103. A method according to Item 101, wherein the first location is an extrachromosomal DNA.

104. A method according to Item 101, wherein the second location is an extrachromosomal DNA.

105. A method according to Item 101, wherein the transposase introduces a nucleic acid into the cell.

106. A method for identifying a gene in a cell, comprising the steps of:

introducing into a cell, a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated, and a transposase;
digesting the DNA in the cell with a restriction endonuclease capable of digesting the nucleic acid sequence;
identifying the inverted repeat sequence;
determining a sequence of a nucleic acid having a similar sequence to the inverted repeat sequence; and
comparing the sequence with sequence information in a sequence information database.

BRIEF DESCRIPTION OF THE DRAWINGS

Figure **1** depicts that CpG methylation is involved in a high frequency of transposition.

Figure **1A** depicts the introduction of a single copy of the SB transposon into Sptlc2 locus by insertion-type homologous recombination and the detection of transposon excision by PCR. White square: exon, solid triangle: loxP site, gray and white arrows: nested PCR primers, less dense line: backbone sequence of the plasmid, CAP: CAP promoter, IR/DR-R and IR/DR-L: right IR/DR and left IR/DR, respectively, Xb: XbaI, B: BamHI, K: KpnI.

Figure **1B** depicts excision of SB transposon in Sptlc2 locus of an ES cell. ES 115 clone (shown in panel A) having SB transposon inserted therein by targeting Sptlc2 locus, was transfected with sSB10 expressing the wild type of the SB transposase (transposase +) or pSB10-DDE expressing the inactive SB transposase with deletion of DDE box (transposase -) (Ivics, Z., P. B. Hackett, R. H. Plasterk, and Z. Izsvak.1997;Cell 91:501-510) in a serial dilution. As shown in Panel A, the resultant was screened with nested PCR. Four independent transfections for each of the dilution factors of pSB10 were conducted, and eight independent PCR with 1 μg of genomic DNA per reaction to conduct screening of 8μg of genomic DNA per transfection. Typical PCR results with the maximum amount of pSB10 (2μg) are shown in the left panel. The right panel shows the results of the average number of positive PCR for each transfection and the RT-PCR analysis at the expression level. Actb: beta-actin.
Figure **1C** shows Southern blot analysis of methylation state in the transposon sequences of male mouse germ line cell and ES cell. EGFP was used as a probe. Genomic DNA derived from the germ line cell has not been digested by HpaII. This shows that the site has mostly been methylated. On the other hand, the genomic DNA from the ES115 clone has been substantially completely digested. The presence of minor band (shown as asterisk) shows that a small fraction of HpaII site has been methylated in an ES cell. Solid circle: HpaII (H) or MspI (M) site; X: XhoI site; 0.5 kb bands derived from HpaII-MspI fragment in KM, KXM and KXH lanes are shown with arrow.
Figure **1D** shows a result of an assay of temporary transposon excision.

a) Experimental procedure is shown. Transposon DNA (pTransCX-EGFP : neo, Horie et al. , Proc. Natl. Acad. Sci. USA 2001) has been methylated in advance with SssI CpG methylase. Murine erythroleukemia cell (MEL cell) was introduced with transposon DNA and Sleeping Beauty (SB) transposase. Total DNA was extracted from the cell, and PCR was conducted with the plasmid vector using primers, and 358 bp PCR products detected, which are amplified when the excision reaction is caused.
b) The result of the PCR is shown. A cell in which methylated transposon has been introduced with a transposition enzyme, showed excision reactions more often than a cell with unmethylated transposons. Abbreviation: CAG: CAG promoter, EGFP: green fluorescent protein, pA: poly addition signal, L: left IR/DR, R: right IR/DR, M: methylation, N: non-methylation, NC: negative control (MEL cell, genomic DNA).

Figure **2** shows a figure of establishment of a cell having a methylated or unmethylated transposon in the same genetic locus of a murine genome. Figure **2A** depicts a flow chart of an exemplary experiment. Figure **2B** shows a result of Southern blotting analysis. Figure **2C-D** show examples of exemplary results for confirming maintenance of methylation. Figure **2D** shows production of cells having methylated SB transposon or non-methylated SB transposon in the predetermined locus of the genome.
Figure **2E** shows a fluorescent activated cell sorting (FACS) for EGFP expression in a target clone. Gray region: wild-type cell. Light line: target clone.
Figure **3A** is an example showing the effects of DNA methylation on the reaction of transposon excision in the murine genome.
Figure **3B-C** depicts the details of frequent excision of methylated SB transposon in a predetermined locus of the genome. A portion thereof overlaps with Figure **3A.**

One microgram or 10 nanograms of clones shown in Figure 2 have been used as a template for nested PCR. For each clone, 10 rounds of PCR have been performed. (B) Direction D; (C) Direction I. NC: genomic DNA which has not been transfected as a negative control, M: 100 bp ladder. Right panel shows methylation state of the transposon region and the parent clone.

Figure **4** depicts an example showing the effects of insertion of a transposon into the genome. A depicts an exemplary plasmid construct used herein. B shows methylation (left) and non-methylation (right).

Figure **4B** depicts effects of CpG methylation on the SB transposition. Average values of G418 resistant colony per culture dish against three independent transfections are shown on the right panel. Error bar shows standard deviation.

Figure **4C** depicts the structure of a gene trapped and transposon insertion site. The insertion site is shown with black arrows. Clones M1-M4 are derived from transfection with methylated transposon, and Clones N1-N6 are derived from non-methylated transposon. The number of chromosomes, ensemble gene designator, and the gene nomenclature are also shown. Except for Clone M2, in which two insertion sites have been characterized (called M2S and M2L), one insertion site has been identified for each clone. Correct splicing in some clones (M2S, M2L, M4 and N1) have been confirmed with RT-PCR using primers to the upstream exon (white arrow) and transposon. Solid square: exon. Scale bar for 5kb is shown on the right hand side.

Figure **4D** shows the effect of methylation on Tc transposition. Construct for the trapping vector is shown in the upper panel. TIR: terminal inverted repeat. Three independent transfections are shown in the lower panel with respect to the average number of G418 resistant colonies per dish.

Figure **5** shows an alignment of typical transposon sequences. Multiple alignment with X01005 (1-1610), Z29098 (15-1787), Z29102 (15-1787), U11641 (188-1451), U11652 (146-1442) and L48685 (1-1455) are shown (the numerals in parentheses show the range of base sequence in the sequence of each Accession number)

Figure **6** shows invariant affinity of SB transposon DNA binding domain against naked methylated IR/DR.

Figure **6A** shows an exemplary drawing of CpG sites in the IR/DR and transposase binding sites. Solid circle: CpG sites.

Figures **6B-E** shows an electrophoretic mobility shift assay (EMSA) in the recombinant SB transposase peptide (N123).

Figures **6B** and **6C** show results obtained in the external binding site of IR/DR-L 34 bp, and Figures **6D** and **6E** show the results obtained by IR/DR-L fragment of 300 bp.

As shown in Figure **6B**, a concentration of N123 peptide sufficient for enhancing non-methylated or methylated external binding sites (1600 to 100 dilution of the purified peptide) mixed therewith to form a nucleoprotein complex.

As shown in Figure **6C,** non-methylated external binding site has been labeled for use as a probe, and non-methylated external binding site or methylated external binding site have been used as a competitive substance in an increased concentration (10-50 folds molar excessive probes).

As shown in Figure **6D,** non-methylated IR/DR-L fragment or methylated IR/DR-L fragment have been mixed with an increased concentration of N123 peptides (5100-160 fold dilution of the purified peptides) for formation of nucleotprotein complex.

As shown in Figure **6E,** non-methylated IR/DR-L fragment was labeled for use as a probe, and non-methylated or methylated IR/DR-L fragments have been used as a competitive substance in an increased concentration (500-8000 fold molar excess of probes). Unmet: non-methylated, Met: methylated, F: free probe, C: complex. Complexes 1 (C1) and 2 (C2) indicate one and two molecule(s) of N123 peptides per IR/DR, respectively (Ivics, Z., P. B. Hackett, R. H. Plasterk, and Z. Izsvak. 1997; Cell 91: 501-510) .

Figure 7 shows ChIP assay of methylated transposon or non-methylated transposon in a predetermined locus.

Figure **7A** shows SB transposon region, which was analyzed in the ChIP assay. PCR amplification region is shown as light line under each component. Solid triangle: lox 511 site.

Figures **7B** and **7C** show PCR analysis of precipitated DNA. As shown in Figures **3** and **4,** Clones 5M3 and 5U3 (B) and 6M11 and 6U1 (C) are derived from parent clones RL5 and RL6, respectively. Input and precipitated DNA have been analyzed in a five-fold stepwise dilution. AcH3: anti-acetylated H3, MeH3K9; anti-trimethylated H3K9, no Ab: control having no antibodies. Amylase 2.1 and beta-small globin have been used as a typical controls against heterochrome regions and euchromatin regions. Intensity of bands of amylase 2.1 and beta-small globin are the same in the anti-acetyl H3 fraction, whereas the higher amplification efficiency than amylase 2.1 sequence in the input DNA, indicates that beta-small globin is enriched compared with the amylase 2.1 sequence in this fraction. Minor bands found in the control lanes without antibodies are derived from non-specific binding of the genomic DNA to the protein. Agarose beads are used for preparation. Determination was conducted twice. Representative results are shown.

In the Figures, "GFP" refers to the GFP gene. As used herein, the transposon used in the Sleeping Beauty (SB) transposon system are SB transposon, and the transposase is the SB transposase. In the figures, "SB" indicates the Sleeping Beauty transposase gene.

DESCRIPTION OF SEQUENCE LISTING

SEQ ID NO: 1: DNA sequence of the transposon SB (Sleeping Beauty) (GENBANK Accession No.: L48685)
SEQ ID NO: 2: Nucleic acid sequence of SB transposase
SEQ ID NO: 3: Polypeptide sequence of SB transposase polypeptide
SEQ ID NO: 4: Nucleic acid sequence of left outer repeat sequence
SEQ ID NO: 5: Nucleic acid sequence of left internal repeat sequence
SEQ ID NO: 6: Nucleic acid sequence of TgTP-1U
SEQ ID NO: 7: Nucleic acid sequence of TgTP-2L
SEQ ID NO: 8: Nucleic acid sequence of TgTP-2U
SEQ ID NO: 9: Nucleic acid sequence of TgTP-3L
SEQ ID NO: 10: Nucleic acid sequence of transposable element Tc1derived from Caenorhabditis elegans (GENBANK Accession X01005)
SEQ ID NO: 11: Amino acid sequence of transposable element derived from Caenorhabditis elegans (GENBANK Accession X01005)
SEQ ID NO: 12: Nucleic acid sequence of Minos-2 derived from Drosophila hydei (GENBANK Accession Z29098)
SEQ ID NO: 13: Drosophila hydei Minos-2 amino acid sequence (GENBANK Accession No.: Z29098)
SEQ ID NO: 14: Drosophila hydei Minos-3 nucleic acid sequence (GENBANK Accession No.: Z29102)
SEQ ID NO: 15: Drosophila hydei Minos-3 amino acid sequence (GENBANK Accession No.: Z29102)
SEQ ID NO: 16: Haematobia irritans Hi2 mariner nucleic acid sequence (GENBANK Accession No.: U11641)
SEQ ID NO: 17: Haematobia irritans Hi2 mariner amino acid sequence (GENBANK Accession No.:1641)
SEQ ID NO: 18: Chrysoperla plorabunda mariner nucleic acid sequence (GENBANK Accession No.: U11652)
SEQ ID NO: 19: Chrysoperla plorabunda mariner amino acid sequence (GENBANK Accession No.: U11652)
SEQ ID NO: 20: IR/DR-R sequence is shown:

```
aattccatcacaaagctctgacctcaatcctatagaaaggaggaatgagccaa
aattcacccaacttattgtgggaagcttgtggaaggctactcgaaatgtttga
cccaagttaaacaatttaaaggcaatgctaccaaatactaattgagtgtatgt
taacttctgacccactgggaatgtgatgaaagaaataaaagctgaaatgaatc
attctctctactattattctgatatttcacattcttaaaataaagtggtgatc
ctaactgaccttaagacagggaatctttactcggattaaatgtcaggaattgt
gaaaaagtgagtttaaatgtatttggctaaggtgtatgtaaacttccgacttc
aactgta
```

```
ccttgaaatacatccacaggtacacctccaattgactcaaatgatgtcaatta
gtctatcagaagcttctaaagccatgacatcattttctggaattttccaagct
gtttaaaggcacagtcaacttagtgtatgtaaacttctgacccactggaattg
tgatacagtgaattataagtgaaataatctgtctgtaaacaattgttggaaaa
atgacttgtgtcatgcacaaagtagatgtcctaactgacttgccaaaactatt
gtttgttaacaagaaatttgtggagtagttgaaaaacgagttttaatgactcc
aacttaagtgtatgtaaacttccgacttcaactgta
```

SEQ ID NO: 21: IR/DR-L sequence is shown:
SEQ ID NO: 22: example of repetitive portion of an inverted repeat sequence: gttcaagtcg gaagtttaca tacacttag
SEQ ID NO: 23: example of repetitive portion of an inverted repeat sequence: cagtgggtca gaagtttaca tacactaagg
SEQ ID NO: 24: example of repetitive portion of an inverted repeat sequence: cagtgggtca gaagttaaca tacactcaat t
SEQ ID NO: 25: example of repetitive portion of an inverted repeat sequence: agttgaatcg gaagtttaca tacaccttag

SEQ ID NO: 26: example of common portion of repetitive portion of an inverted repeat sequence: caktgrgtcr gaagtttaca tacacttaag
SEQ ID NO: 27: example of left outer repeat
SEQ ID NO: 28: example of left inner repeat
SEQ ID NO: 29: forward sequence used for PCR amplification from pCMV-SB
SEQ ID NO: 30: reverse sequence used for PCR amplification from pCMV-SB
SEQ ID NO: 31: primer EGFP-1U
SEQ ID NO: 32: primer EGFP-1L
SEQ ID NO: 33: primer HYG-1U
SEQ ID NO: 34: primer TK-1L
SEQ ID NO: 35: primer M13F
SEQ ID NO: 36: primer RMCE-DL1
SEQ ID NO: 37: primer RRMCE-IL-1
SEQ ID NO: 38: primer TgTP-2L
SEQ ID NO: 39: primer neo-U1
SEQ ID NO: 40: primer neo-L1
SEQ ID NO: 41: fluorescence labeled probe for excision production
SEQ ID NO: 42: fluorescence labeled probe for excision production
SEQ ID NO: 43: fluorescence labeled probe for neo fragment SEQ ID NO: 44: fluorescence labeled probe for neo fragment SEQ ID NO: 45: PCR primer for excision product
SEQ ID NO: 46: PCR primer for excision product
SEQ ID NO: 47: PCR primer for neo fragment
SEQ ID NO: 48: PCR primer for neo fragment
SEQ ID NO: 49: primer LCB2XL2
SEQ ID NO: 50: primer PGK2
SEQ ID NO: 51: primer LCB2XL1
SEQ ID NO: 52: primer PGK4
SEQ ID NO: 53: forward primer for amplification of SB transposase gene
SEQ ID NO: 54: reverse primer for amplification of SB transposase gene
SEQ ID NO: 55: forward primer for amplification of beta-actin transposase gene
SEQ ID NO: 56: reverse primer for amplification of beta-actin transposase gene
SEQ ID NO: 57: primer β-geo
SEQ ID NO: 58: primer specific for M2S
SEQ ID NO: 59: primer specific for M2L
SEQ ID NO: 60: primer specific for M4
SEQ ID NO: 61: primer specific for N1
SEQ ID NO: 62:Unmet-U
SEQ ID NO: 63:Unmet-L
SEQ ID NO: 64:Met-U
SEQ ID NO: 65:Met-L
SEQ ID NO: 66: forward primer for amylase 2.1 gene
SEQ ID NO: 67: reverse primer for amylase 2.1 gene
SEQ ID NO: 68: forward primer for beta-small globin gene SEQ ID NO: 69: reverse primer for beta-small globin gene SEQ ID NO: 70: forward primer sequence for IR/DR-L
SEQ ID NO: 71: reverse primer sequence for IR/DR-L
SEQ ID NO: 72: forward primer sequence for ID/DR-R
SEQ ID NO: 73: EGFP-1U primer
SEQ ID NO: 74: EGFP-1L primer
SEQ ID NO: 75: SB-2U primer
SEQ ID NO: 76: SB-1L primer

BEST MODE FOR CARRYING OUT THE INVENTION

Hereinafter the present invention is described.

It should be understood throughout the present specification that expression of a singular form includes the concept of their plurality unless otherwise mentioned. Specifically, articles for a singular form (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned. Thus, unless otherwise defined, all scientific and technical terms have the same meanings as those generally used by those skilled in the art to which the present invention pertain. If there is contradiction, the present specification (including the definition) precedes.

(Definition and Description of Terms)

Hereinafter, the definition of terms specifically used herein are listed.
Transposon as used herein refers to a nucleic acid molecule or nucleic acid sequence which is capable of moving (transposition) from one site to another on a chromosome. Typically, transposon is a DNA segment (DNA transposon). DNA transposons (hereinafter simply referred to as "transposons") are activated by a transposase and are then transposed. Transposons include, but are not limited to, for example, SB transposon (Acc. No. L48685; SEQ ID NO: 1) , and those included in the sequences set forth in SEQ ID NOs: 10-19, and the like.
As used herein, the term "transposase" refers to an enzyme having catalytic activity of recognizing a transposon and transposing the transposon. Transposases include, but are not limited to, for example, SB transposases (SEQ ID NOs : 2, 3) and those included in the sequences set forth in SEQ ID NOs: 10-19, and the like.
In the present invention, any transposon sequences and transposase genes used in a transgenic biological organism, regardless of being internal or external, preferably external transposon sequences and transposase genes, can be used.
Transposons usually have a repeated sequence (hereinafter referred to as a "transposon sequence") on each end thereof. This repeated sequence is a transposase recognition site. Examples of such repeated sequences include, but are not limited to, for example, SEQ ID NO: 22-26. The transposon sequence may include an imperfect repeated portion if the transposon can be transposed by action of a transposase. An insertion recognition site having a length specific to transposons within DNA into which a transposon is to be inserted is called a target sequence. For example, in the case of the Sleeping Beauty (SB) transposon system (Z. Ivics et al. ; Cell, 91:501-510 (1997)), the target sequence is TA. After a transposon is inserted, the sequence is TA-transposon-TA. Accordingly, as used herein, transposon sequence refers to a sequence recognized by a transposase, and transposable natural or artificial transposon sequence in a desired organism.
As target sequences of transposons, for example, TA, ATAT, TATATA, TACA, and the like are known. Regardless of the type of target sequences, the object which enhances efficiency of gene introduction by a transposon has been achieved by methylation of the transposon in the present invention.
Transposons include mainly those self-completing type, internally including enzymatic transposase which can catalyze transposition *per se*, and those self-incompleting type, lacking transposase activity. Biological organisms, each having a transposon sequence and a transposase gene are bred. When biological organisms having a transposon sequence and a transposase gene are obtained, or when biological organisms having a transposon sequence whose transposition is fixed, (without a transposase gene) are obtained, non-self completed types of transposons are used. When biological organisms including both transposons and transposases or those comprising signature and lacking a transposon and a transposase gene are obtained, both self completing type and non-self completing type transposons can be used. The self completing type transposon may be converted to a non-self completing type by excising a transposase, by using the Cre loxP system, which is described below, or breeding a biological organism having the loxP sequence at both sides of the transposase gene and non biological organisms having Cre. Further, transposons of host-dependent type and host-independent type both can be used. Generally, the host-independent type can be used.
In the present invention, transposon only and/or transposase only may be excised from the transposon system comprising a transposon and a transposase gene for use, or transposase may be inactivated to make the transposon the non-self completing type for use. A transposon system useful for practicing the present invention, includes any sequence which is transposable in a biological cell, and preferably a mariner superfamily member may be used. Examples thereof include for example, transposon family such as Tc1 SB, Minos, Txr, Tc; mariner transposon family including Caenorhabditis elegans, Mos1, Hyalophora cecropia and the like; Pogo transposon family such as Pogo, Tigger,"4 and the like (R.H. Plasterk et al . ; Trends in genetics, 15: No. 8: 326-332 (1999)). Most preferably, the SB (Sleeping Beauty) transposon may be used. Non-self completing transposon system may be obtained by removing or inactivating a transposase gene in a self completing type transposon.
As used herein, "DNA type" transposon refers to a transposon for use in transposing a DNA. Usual transposons are of DNA type. In typical embodiments, the present invention is practiced by using a DNA-type transposon.
As used herein, the term "Tc1/mariner type transposon" refers to a transposon similar to Tc1/mariner. Transposons belonging to Tc1/mariner type include, but are not limited to: minos, SB, Tcl, mariner and the like. This superfamily is believed to be derived from a vertebrate genome (Radice et al., 1994; Smit and Riggs, 1996 Proc. Natl. Acad. Sci. USA 93, 1443-1448).
As used herein the term "SB" and "Sleeping Beauty" refer to a transposon of the Tc1/mariner type, and which has transposon activity in a mammal or a mammalian cell (Ivics et al . , Cell 91 :501-510, 1997) . As used herein the term SB may be used as SB polypeptide, SB transposon and the like.

Portions sandwiched or inserted between transposon sequences may be a variety of DNA sequences (for example, marker genes, gene expression regulatory sequences, desired genes and the like). A variety of transposon constructs combining a variety of elements as necessary, in addition to a transposon sequence, can be constructed. In the present invention, a target cell for introduction of a transposon construct or transposase gene, may be a cell having the potential to differentiate into an individual of a biological organism, preferably a non-human organism, and such cells include, for example, stem cells or fertilized cells.

The transgenic biological organism of the present invention includes founder (not only the first generation but also those lineages established based on the founder are of course encompassed by the present invention) having either or both a transposon construct and a transposase. Further, organs, tissues, eggs, sperms and fertilized eggs derived from the transgenic biological organism lineage of the present invention, established cell lines established from a lineage of the transgenic biological organism, cloned individuals produced from the transgenic biological organisms, are also encompassed by the present invention. Transposon constructs of the present invention may be constructed by combining a transposon sequence with a variety of other elements and introduced into a stem cell or fertilized egg or the like.

(Development of transposon vectors having high gene disruption efficiency)

Hereinafter, the transposon system development which the inventors have conducted is described. Transposon system as used in the present invention usually consists of two elements of a transposon sequence, a DNA sequence transposing on the genome, and a transposase, an element catalyzing the transposition. If there is a gene in the place to be transposed, the function of the gene may be disrupted. However, most of the genes are composed of introns, and thus even if it is inserted into inside the gene, there is a possibility of elimination of the transposon sequence when splicing. In order to prevent this elimination, it is possible to construct a construct having a splice acceptor inside the structure. A transcription product of an internal gene is stopped in the transposon, and as a result, it is expected that the function of the internal gene is efficiently disrupted. In order to investigate this hypothesis, for three lineages of mice which have been inserted with transposons inside the gene, homozygous mice are obtained, and tail RNA are used to conduct RT-PCR using primers corresponding to exons upstream and downstream of insertion sites, to confirm elimination of bands corresponding to the normal gene product. Furthermore, it is possible to confirm the expression of the lacZ gene or the like for the existence of expected splicing. As such, verification of extremely high gene disruption efficiency of the produced vector can be confirmed. Moreover, when tested for determination of transposition of 200 or more sites for transposition distribution, it can be confirmed that about 80 % of the transpositions are in the identical chromosome and the remaining 20 % are transposed to other chromosomes. Accordingly, in order to introduce exhaustively mutation into the entire genome, it is desirable to produce a mouse having a transposon in a variety of chromosomes. Based on this idea, to date, a number of lineages of mice having transposons at different sites on the genome have been established. In fact, the present inventors have established more than 20 lineages. In the present procedures, it is important that the transposon insertion site *per se,* before transposition, have no effects on phenotypes, and thus systems satisfying these conditions are identified. On the other hand, when transposition happens in an identical chromosome, the transposition appears to be concentrated in the distance of within 3Mb before the transposition. This suggests the possibility of introduction of a variety of mutations onto a specific genetic locus. In fact, in some lineages of mice, insertion of transposons into the Neurexin 3 gene is identified in four mice. From the Neurexin 3 gene, different promoters are used to produce alpha and beta types of two proteins, and the present inventors have also found that it is recognized that the four mice have identified two out of four species the alpha-type only, and the remaining two types of both alpha and beta types have been introduced at locations with mutations. Site specific mutagenesis of this type is believed to be a specific property of transposons, and is expected to have future applications.

In the present invention, transposons are methylated in vitro, and introduced to an ES cell together with a transposase expression vector, resulting in significant increase in the transposition efficiency into the genome. Further, in order to assay transposition efficiency in the genome, a methylated transposon or a transposon in the state of methylation, is inserted into a specific site of the genome. After methylation of the transposon and the state of methylation has been confirmed to be maintained after insertion into the genome, when a transposase is expressed in the cell, methylation introduction is greater than 100 times more efficient than transposition using a transposon having no methylation. It was also found that the transposon region has been heterochromatinated, which indicates the possibility of enhancing transposition frequency of modifying the state of the genome of the transposon region.

As used herein the term "methylation" refers to the addition of a methyl group to a nucleic acid molecule . If similar effects are found, the methyl group may be a similar group such as lower alkyl group. Methylation is catalyzed by a methylation enzyme (methylase) in vivo. As used herein methyl group is provided by a substrate via a methyl group transposition response.

One example is that a methyl group is produced as 5-methyl tetrahydrofolate via enzymatic reduction of 5,10-methylene tetrahydrofolate, and transferred to a homocysteine via a type of cobamide enzyme to result in a methionine. The methionine is converted to S-adenosyl methionine via action of ATP, which then acts as methyl donor which is used for production of a variety of methyl products. Betaine, which is an oxidated type of choline, may also act as a methyl donor. Several tens of methyl group transferase specific to each compound have been elucidated, and as necessary, an appropriate enzyme may be selected by those skilled in the art, and may be used in the present invention. Alternatively, methylation may be added by chemical reaction in addition to the use of an enzyme.

As used herein, the confirmation of methylation may be conducted by means of any conventional technology known in the art. Such a method includes, but is not limited to for example, confirmation of changes in physical behavior by methylation, bisulfite modified sequencing and the like (Gitan RS, et al., Genome Res. Jan; 12: 158-64 . 2002; Lilischkis R., et al. Diagn. Mol. Pathol. 2000; 9: 165-71.).

In a system such as those using transposon like SB, each inverted repeat typically comprises at least one direct repeat. The gene transposition system of the present invention includes, therefore two constructs: transposase and cloned non-self completing (i.e. non-self insertion) salmon type element or a transposon including transposon substrate DNA of an inverted repeat. When mixing together, these two constructs provide active transposon activity.

As used herein the term "inverted repeat sequence" is a sequence acting in a transposon, and has often a structural feature of having an inverted repeat of 15-40 base pairs at both termini, such as those of SEQ ID NOs: 22-26. Typical sequences used herein are set forth in SEQ ID NOs: 20-21. These inverted repeat sequences are insertion sequences, and one of transposition elements. Each inverted repeat sequence preferably comprises at least one tandem repeat sequence (i.e. called IR/DR) . Transposon elements may be a linear nucleic acid fragment (for convenience, from the 5' terminus to the 3' terminus) which can be used as a linear fragment, or for example, circular in a plasmid. In a preferable embodiment, two direct repeat sequences occur in each inverted repeat sequence.

Preferable inverted repeat sequences herein include, but are not limited to, for example, the inverted repeat sequence of the Tc1/mariner type transposon and the SB transposon, and the like. Amongst these, those binding to SB include but are not limited to, the inverted repeat sequence of the SB transposon.

Preferable direct repeat sequences herein include, but are not limited to, for example, direct repeat sequences of Tc1/mariner type transposon and SB transposon and the like. Amongst these, those binding to SB include but are not limited to, direct repeat sequence of SB transposon.

The direct repeat sequences are typically about 25 to about 35 base pairs in length, and preferably about 29 to about 31 base pairs in length. However, regardless of this, an inverted repeat may include the sole direct repeat. In these cases, it is not an actual repeat, but as described below in detail, these are nucleotide sequences having at least about 80 % identity to the consensus direct repeat sequence. The transposon element may be linear chain of nucleic acid fragments, which may be used as a linear fragment or a circular fragment, for example, a plasmid (conventionally, extending from the 5' terminus to the 3' terminus).

In a preferred embodiment of the transposon, there are two direct repeats in each inverted repeat sequence. The direct repeats (which number, in this embodiment, is four) have similar nucleotide sequences, as described in more detail below. An inverted repeat on the 5' or "left-hand" side of a nucleic acid fragment of this embodiment typically comprises a direct repeat (i.e., a left outer repeat), an intervening region, and a second direct repeat (i.e., a left inner repeat). An inverted repeat on the 3' or "right-hand" side of a nucleic acid fragment of this embodiment comprises a direct repeat (i.e . a right inner repeat) , an intervening region, and a second direct repeat (i.e. a right outer repeat).

Because these are inverted with respect to each other on the nucleic acid fragment, the direct repeats in the 5' inverted repeat of the nucleic acid fragment are in a reverse orientation compared to the direct repeats in the 3' inverted repeat of the nucleic acid fragment. The intervening region within an inverted repeat is generally at least about 150 base pairs in length, preferably at least about 160 base pairs in length. The intervening region is preferably no greater than about 200 base pairs in length, more preferably no greater than about 180 base pairs in length. The nucleotide sequence of the intervening region of one inverted repeat may or may not be similar to the nucleotide sequence of an intervening region in another inverted repeat.

Most transposons have perfect inverted repeats, whereas the inverted repeats that bind SB protein generally have at least about 80% identity to a consensus direct repeat, preferably about 90% identity to a consensus direct repeat. Preferable consensus direct repeats, include but are not limited to, sequences such as SB transposon sequence and the like.

Sites which are hypothesized to be the core binding site of the SB protein, are present in the 123 amino acids from the N-terminus of SEQ ID NO: 3. Nucleotide identity is determined by homology search between the sequence set forth in SEQ ID NO: 1 or the like and the direct repeat.

Examples of direct repeat sequences that bind to SB protein include but are not limited to those sequence of SB transposon. An example of a left outer repeat includes, but is not limited to: 5' gttgaagtcggaagtttacatacacttag-3' (SEQ ID NO: 27). An example of a left inner repeat includes, but is not limited to: 5'-cagtgggtcagaagtttacatacactaagg-3' (SEQ ID NO: 28). Examples of a right inner repeat includes, but is not limited to those similar to left inner repeat (for example, having identity of 90 % or greater). Examples of right outer repeat include, but are not limited to those similar to left outer repeat sequence (for example having identity of 90 % or greater).

The inverted repeat may include the poly (A) signal AATAAA. This poly (A) signal can be utilized by a coding sequence present in the nucleic acid fragment to result in addition of a poly (A) tail to an mRNA. The addition of a poly (A) tail to an mRNA typically results in increased stability of that mRNA relative to the same mRNA without the poly (A) tail. Preferably, the inverted repeat is present on the 3' of a nucleic acid fragment that comprises two direct repeats in each inverted repeat sequence.

The inverted repeat will be flanked by a nucleic acid sequence to be inserted in the DNA in a cell. The nucleic acid sequence may comprise the entire or partial open reading frame of the gene (i.e., a portion of the gene encoding a protein) , alone or with one or more expression regulatory sequences (i.e. regulatory sequence in the nucleic acid) with the entire or partial of the open reading frame. Preferable expression regulatory sequences include those elements including, but not limited to promoters, enhancers, boarder regulatory elements, genetic locus regulatory regions or silencers. In a preferable embodiment, the nucleic acid sequence includes a promoter operably linked to a portion of the open reading frame.

Transposons may transpose from the first location to the second location on the DNA in the presence of transposase (also called mobility). Any mobile cut-and-paste type transposon includes two basic elements, and the origin of active transposase and a mobile DNA sequence which is recognized by the transposase. Mobility of the DNA sequence allows transposition of intervening nucleic acids between the recognized DNA sequences.

As used herein the term "foreign gene" refers to a gene which is intended to be introduced via gene transfer of the present invention or a nucleic acid molecule encoding the same. Such a foreign gene is derived from a host of different origin from the host which the introduction is intended or the same host. As long as introduction is intended, the nucleic acid sequence encoding the foreign gene may encode any protein. In one embodiment the protein encoded by the nucleic acid sequence is a marker protein such as GFP, chloramphenicol acetyltransferase (CAT), β-galactosidase (lacZ), and luciferase (LUC). In another embodiment, the protein encoded by the nucleic acid is a growth hormone, for example to promote growth in a transgenic animal, or insulin-like growth factors (IGFs). Alternatively, the foreign gene may not encode a protein.

In one embodiment of a transgenic animal, the protein encoded by the nucleic acid fragment is a product for isolation from a cell. Transgenic animals as bioreactors are known. Protein can be produced in quantity in milk, urine, blood or eggs. Promoters are known that promote expression in milk, urine, blood or eggs and these include, but are not limited to, the casein promoter, the mouse urinary protein promoter, beta-globin promoter and the ovalbumin promoter, respectively. Recombinant proteins are produced by means of other methods for producing a protein in a cell. Nucleic acids encoding these or other proteins can be incorporated into the nucleic acid fragment of this invention and introduced into a cell. Efficient incorporation of the nucleic acid fragment into the DNA of a cell occurs when a composition of the present invention is present. Where the cell is part of a tissue or part of a transgenic animal, large amounts of recombinant protein can be obtained.

(Cells and Biology)

The term "cell" is herein used in its broadest sense in the art, referring to a structural unit of tissue of a multicellular organism, which is capable of self replicating, has genetic information and a mechanism for expressing it, and is surrounded by a membrane structure which isolates the living body from the outside. Cells used herein may be either naturally-occurring cells or artificially modified cells (e.g., fusion cells, genetically modified cells, etc.) . Cells used herein may be either naturally-occurring cells or artificially modified cells (e.g. , fusion cells, genetically modified cells, etc.), as long as the cell has a chemical receptor or is capable of having such a nucleic acid molecule introduced therein. Examples of cell sources include, but are not limited to, a single-cell culture; the embryo, blood, or body tissue of normally-grown transgenic animal; a mixture of cells derived from normally-grown cell lines; and the like. Preferably, a cell which is easily transformed or transfected is used. Cells used in the present invention are preferably cells which easily introduces a nucleic acid thereinto.

Cells used herein may be derived from any organism (e.g., any unicellular organisms (e.g., bacteria and yeast) or any multicellular organisms (e.g., animals (e.g., vertebrates and invertebrates), plants (e.g., monocotyledons and dicotyledons, etc.)). For example, cells used herein are derived from a vertebrate (e.g., Myxiniformes, Petronyzoniformes, Chondrichthyes, Osteichthyes, amphibian, reptilian, avian, mammalian, etc.), more preferably mammalian (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, etc.). In one embodiment, cells derived from Primates (e.g., chimpanzee, Japanese monkey, human) are used. Particularly, without limitation, cells derived from a human are used. The above-described cells may be either stem cells or somatic cells. Also, the cells may be adherent cells, suspended cells, tissue forming cells, and mixtures thereof.

Any organ may be targeted by the present invention. A tissue or cell targeted by the present invention may be derived from any organ. As used herein, the term "organ" refers to a morphologically independent structure localized at a particular portion of an individual organism in which a certain function is performed. In multicellular organisms (e.g., animals, plants), an organ consists of several tissues spatially arranged in a particular manner, each tissue being composed of a number of cells. An example of such an organ includes an organ relating to the vascular system. In one embodiment, organs targeted by the present invention include, but are not limited to, skin, blood vessel, cornea, kidney, heart, liver, umbilical cord, intestine, nerve, lung, placenta, pancreas, brain, peripheral limbs, retina, and the like. In plant, "organ" includes, but is not limited to: callus, root, caulome, stem, stalk, leaf, flower, seed, embryo, germ, fruit, albumen and the like.

As used herein, the term "tissue" refers to an aggregate of cells having substantially the same function and/or form in a multicellular organism. "Tissue" is typically an aggregate of cells of the same origin, but may be an aggregate of cells of different origins as long as the cells have the same function and/or form. Therefore, when stem cells of the present invention are used to regenerate tissue, the tissue may be composed of an aggregate of cells of two or more different origins. Typically, a tissue constitutes a part of an organ. Animal tissues are separated into epithelial tissue, connective tissue, muscular tissue, nervous tissue, and the like, on a morphological, functional, or developmental basis. Plant tissues are roughly separated into meristematic tissue and permanent tissue according to the developmental stage of the cells constituting the tissue. Alternatively, tissues may be separated into single tissues and composite tissues according to the type of cells constituting the tissue. Thus, tissues are separated into various categories.

As used herein, the term "stem cell" refers to a cell capable of self replication and pluripotency. Typically, stem cells can regenerate an injured tissue. Stem cells used herein may be, but are not limited to, embryonic stem (ES) cells or tissue stem cells (also called tissular stem cell, tissue-specific stem cell, or somatic stem cell). Accordingly, a stem cell may be directly used in the present invention.

As used herein, the term "somatic cell" refers to any cell other than a germ cell, such as an egg, a sperm, or the like, which does not transfer its DNA to the next generation. Typically, somatic cells have limited or no pluripotency. Somatic cells used herein may be naturally-occurring or genetically modified.

As used herein, the term "isolated" means that naturally accompanying material is at least reduced, or preferably substantially completely eliminated, in normal circumstances. Therefore, the term "isolated cell" refers to a cell substantially free from other accompanying substances (e.g., other cells, proteins, nucleic acids, etc.) in natural circumstances. The term "isolated" in relation to nucleic acids or polypeptides means that, for example, the nucleic acids or the polypeptides are substantially free from cellular substances or culture media when they are produced by recombinant DNA techniques; or precursory chemical substances or other chemical substances when they are chemically synthesized. Isolated nucleic acids are preferably free from sequences naturally flanking the nucleic acid within an organism from which the nucleic acid is derived (i.e., sequences positioned at the 5' terminus and the 3' terminus of the nucleic acid).

As used herein, the term "established" in relation to cells refers to a state of a cell in which a particular property (pluripotency) of the cell is maintained and the cell undergoes stable proliferation under culture conditions. Therefore, established stem cells maintain pluripotency. In the present invention, such an established cell is preferably used since such a cell provides a stabilized result.

As used herein, the term "differentiated cell" refers to a cell having a specialized function and form (e.g., muscle cells, neurons, etc.). Unlike stem cells, differentiated cells have no or little pluripotency. Examples of differentiated cells include epidermal cells, pancreatic parenchymal cells, pancreatic duct cells, hepatic cells, blood cells, cardiac muscle cells, skeletal muscle cells, osteoblasts, skeletal myoblasts, neurons, vascular endothelial cells, pigment cells, smooth muscle cells, fat cells, bone cells, cartilage cells, and the like.

As used herein the term "a living body" refers to a form of a biological organism which may be present as a single individual capable of existing as a living organism.

(Biochemistry and Molecular Biology)

As used herein, the term "gene" refers to an element defining a genetic trait. A gene is typically arranged in a given sequence on a chromosome. A gene which defines the primary structure of a protein is called a structural gene. A gene which regulates the expression of a structural gene is called a regulatory gene (e.g., promoter). Genes herein include structural genes and regulatory genes unless otherwise specified. Therefore, the term "cyclin gene" typically includes the structural gene of cyclin and the promoter of cyclin. As used herein, "gene" may refer to "polynucleotide", "oligonucleotide", "nucleic acid", and "nucleic acid molecule" and/or "protein", "polypeptide", "oligopeptide" and "peptide". As used herein, "gene product" includes "polynucleotide", "oligonucleotide", "nucleic acid" and "nucleic acid molecule" and/or "protein", "polypeptide", "oligopeptide" and "peptide", which are expressed by a gene. Those skilled in the art understand what a gene product is, according to the context. Accordingly, gene used herein usually includes not only double-stranded DNA but also each single-stranded DNA such as sense chain and antisense chain constituting thereof. Therefore, the genes of the present invention include any of double-stranded DNA including human genome DNA, and single-stranded DNA (sense chain) including cDNA, as well as a single stranded DNA (antisense) having a sequence complementary to the sense chain, as well as fragments thereof.

As used herein, the term "homology" in relation to a sequence (e.g., a nucleic acid sequence, an amino acid sequence, etc.) refers to the proportion of identity between two or more gene sequences. Therefore, the greater the homology between two given genes is, the greater is the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other. As used herein, the term "similarity" in relation to a sequence (e.g., a nucleic acid sequence, an amino acid sequence, or the like) refers to the proportion of identity between two or more sequences when conservative substitution is regarded as positive (identical) in the above-described homology. Therefore, homology and similarity differ from each other in the presence of conservative substitutions. If no conservative substitutions are present, homology and similarity have the same value.

The similarity, identity and homology of amino acid sequences and base sequences are herein compared using FASTA with the default parameters. Alternatively, an identity search may be conducted, for example, using NCBI's BLAST 2.2.9 (published May 12, 2004) . As used herein, the value of identity usually refers to the value as a result of alignment with the BLAST as described above using the default parameter. If the change of parameters results in higher values, then the highest value is employed herein as the value of the identity. When a plurality of regions are evaluated for identity, the highest value is employed herein as the value of the identity.

The terms "protein", "polypeptide", "oligopeptide" and "peptide" as used herein have the same meaning and refer to an amino acid polymer having any length. This polymer may be a straight, branched or cyclic chain. An amino acid may be a naturally-occurring or non-naturally-occurring amino acid, or a variant amino acid. The term may include those assembled into a composite of a plurality of polypeptide chains. The term also includes a naturally-occurring or artificially modified amino acid polymer. Such modification includes, for example, disulfide bond formation, glycosylation, lipidation (acylation), acetylation, phosphorylation, or any other manipulation or modification (e.g., conjugation with a labeling moiety). This definition encompasses a polypeptide containing at least one amino acid analog (e.g., non-naturally-occurring amino acid, etc.), a peptide-like compound (e.g., peptoid), and other variants known in the art. Gene products, such as extracellular matrix proteins (e.g., fibronectin, etc.), are usually in the form of polypeptide, however, there may be a form of a polypeptide variant as long as it has the same function. Polypeptides having specific amino acid sequences include fragments, cognates, derivatives and variants thereof.

The terms "polynucleotide", "oligonucleotide", "nucleic acid molecule" and "nucleic acid" as used herein have the same meaning and refer to a nucleotide polymer having any length. This term also includes an "oligonucleotide derivative" or a "polynucleotide derivative". An "oligonucleotide derivative" or a "polynucleotide derivative" includes a nucleotide derivative, or refers to an oligonucleotide or a polynucleotide having linkages between nucleotides different from typical linkages, which are interchangeably used. Examples of such an oligonucleotide specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2' -methoxyethoxy ribose. Unless otherwise indicated, a particular nucleic acid sequence also implicitly encompasses conservatively-modified variants thereof (e.g. degenerate codon substitutions) and complementary sequences, as well as the sequence explicitly indicated. Specifically, degenerate codon substitutions may be produced by generating sequences in which the third position of one or more selected (or all) codons is substituted with mixed-base and/or deoxyinosine residues (Batzer et al. , Nucleic Acid Res. 19:5081 (1991) ; Ohtsuka et al., J. Biol. Chem. 260:2605-2608 (1985); Rossolini et al., Mol. Cell. Probes 8 : 91-98 (1994) ) . A gene encoding an extracellular matrix protein (e.g., fibronectin, etc.) or the like is usually in the form of polynucleotide. A molecule to be transfected is in the form of polynucleotide.

As used herein amino acids may be referred to with the generally known three-letter abbreviation or the one letter-abbreviation proposed by the IUPAC-IUB Biochemical Nomenclature Commission. Nucleotides may also be referred to with the generally known one-letter abbreviations which are generally accepted.

The letter codes are as follows:

Amino Acids:

| 3-letter | single-letter | reference |
|---|---|---|
| Ala | A | alanine |
| Cys | C | cysteine |
| Asp | D | aspartic acid |
| Glu | E | glutamic acid |
| Phe | F | phenylalanine |
| Gly | G | glycine |
| His | H | histidine |
| Ile | I | isoleucine |
| Lys | K | lysine |
| Leu | L | leucine |
| Met | M | methionine |
| Asn | N | asparagine |
| Pro | P | proline |
| Gln | Q | glutamine |
| Arg | R | arginine |
| Ser | S | serine |
| Thr | T | threonine |
| Val | V | valine |
| Trp | W | tryptophane |
| Tyr | Y | tyrosine |
| Asx | | asparatic acid or |
| asparagine | | |
| Glx | | glutamine or glutamic acid |
| Xaa | | unknown or other amino acid |

Base (Nucleotide)

| abbreviation | reference |
|---|---|
| a | adenine |
| g | guanine |
| c | cytosine |
| t | thymine |
| u | uracyl |
| r | guanine or adenine purine |
| y | thymine/uracil or cytosine purimidine |
| m | adenin or cytocine amino group |
| k | guanine or thymine uracil keto group |
| s | guanin or cytosine |
| w | adenine or thymine/uracil |
| b | guanine or cytocine or thymine/uracil |

d adenine or guanine or thymine/uracil
h adenine or cytosine or thymine/uracil
v adenine or guanine or cytosine
n adenine or guanine or cytosine or

thymine/uracil, unknown or other base

As used herein, the similarity, identity and homology of amino acid sequences and base sequences are herein compared using BLAST, a sequence analysis tool, with the default parameters.

As used herein the term "nucleotide" refers to a nucleoside in which the sugar moiety is phosphate ester, and includes DNA, RNA and the like, and may be naturally occurring or non-naturally occurring. Nucleoside refers to a compound in which a base and a sugar are bound via N-glycoside bonding. "Nucleotide derivative" or "nucleotide analog" are interchangeably used herein to refer to a derivative or an analog which is different from a naturally occurring nucleotide but has a similar function as that of such a nucleotide. Such a nucleotide derivative and nucleotide analog is well known in the art. Examples of such a nucleotide derivative and nucleotide analog include, for example, but are not limited to phosphorothioate, phosphoramidate, methyl phosphonate, chiral methyl phosphonate, 2-O-methyl ribonucleotide, peptide-nucleic acid (PNA). DNA includes cDNA, genomic DNA, and synthetic DNA.

In an embodiment, the variant refers to a naturally occurring allelic variant, non-naturally occurring variant, a variant having deletion, substitution, addition and addition, a polynucleotide sequence which does not substantially alter the function of the encoded polypeptide.

In an embodiment, variation such as mutation of such amino acid sequences may occur in nature such as natural mutation, post-translational modification and the like, but also may artificially made using a naturally occurring gene such as specific genes of the present invention.

In an embodiment, the polypeptide comprises the allelic variants, homologs, natural variants, having at least 70%, preferably at least 80%, more preferably at least 95 %, still more preferably at least 97 % homology with the naturally occurring polypeptide.

As used herein, the term "corresponding" amino acid or nucleic acid refers to an amino acid or nucleotide in a given polypeptide or polynucleotide molecule, which has, or is anticipated to have, a function similar to that of a predetermined amino acid or nucleotide in a polypeptide or polynucleotide as a reference for comparison. Particularly, in the case of enzyme molecules, the term refers to an amino acid which is present at a similar position in an active site and similarly contributes to catalytic activity. For example, in the case of the transposon sequence for a certain polynucleotide, the term refers to a similar portion in an ortholog corresponding to a particular portion of the transposon sequence.

As used herein, the term "corresponding" gene (e.g., a polypeptide or polynucleotide molecule) refers to a gene in a given species, which has, or is anticipated to have, a function similar to that of a predetermined gene in a species as a reference for comparison. When there are a.plurality of genes having such a function, the term refers to a gene having the same evolutionary origin. Therefore, a gene corresponding to a given gene may be an ortholog of the given gene. Therefore, genes corresponding to those such as murine transposon and murine transposase can be found in other animals. Such a corresponding gene can be identified by techniques well known in the art. Therefore, for example, a corresponding gene in a given animal can be found by searching a sequence database of the animal (e.g., human, rat, dog, cat) using the sequences such as murine transposon and murine transposase of a reference gene as a query sequence. Such corresponding genes can be readily obtained by those skilled in the art using genome databases. Methods for obtaining such genome sequences are well known in the art and described herein elsewhere. In the present invention, sequences obtained by such search can also be used.

As used herein, the term "fragment" with respect to a polypeptide or polynucleotide refer to a polypeptide or polynucleotide having a sequence length ranging from 1 to n-1 with respect to the full length of the reference polypeptide or polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more nucleotides. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit. As used herein, the length of polypeptides or polynucleotides can be represented by the number of amino acids or nucleic acids, respectively. However, the above-described numbers are not absolute. The above-described numbers as the upper or lower limit are intended to include some greater or smaller numbers (e.g., $\pm$10%), as long as the same function is maintained. For this purpose, "about" may be herein put before the numbers. However, it should be understood that the interpretation of numbers is not affected by the presence or absence of "about" in the present specification. In the present invention, it should be understood that any fragment can be used as long as the fragment functions as murine transposon, murine transposase and the like, i.e., has transposition activity.

As used herein, the term "biological molecule" refers to a molecule relating to an organism and an aggregation thereof.

As used herein, the term "biological" or "organism" refers to a biological organism, including, but being not limited to, an animal, a plant, a fungus, a virus, and the like. A biological molecule includes a molecule extracted from an organism and an aggregation thereof, though the present invention is not limited to this. Any molecule capable of affecting an organism and an aggregation thereof fall within the definition of a biological molecule. Therefore, low molecular weight molecules (e.g., low molecular weight molecule ligands, etc.) capable of being used as medicaments fall within the definition of biological molecule as long as an effect on an organism is intended. Examples of such a biological molecule include, but are not limited to, a protein, a polypeptide, an oligopeptide, a peptide, a polynucleotide, an oligonucleotide, a nucleotide, a nucleic acid (e.g., DNA such as cDNA and genomic DNA; RNA such as mRNA), a polysaccharide, an oligosaccharide, a lipid, a low molecular weight molecule (e.g., a hormone, a ligand, an information transmitting substance, a low molecular weight organic molecule, etc.) , and a composite molecule thereof (glycolipids, glycoproteins, lipoproteins, etc.), and the like. A biological molecule may include a cell itself or a portion of tissue as long as it is intended to be introduced into a cell. Preferably, a biological molecule may include a nucleic acid (DNA or RNA) or a protein. In another preferred embodiment, a biological molecule is a nucleic acid (e.g., genomic DNA or cDNA, or DNA synthesized by PCR or the like). In another preferred embodiment, a biological molecule may be a protein. Preferably, such a biological molecule may be a hormone or cytokine.

As used herein "chemical synthesized substance" refers to any substance which may be synthesized using an ordinary chemical technology. Accordingly, any chemical synthesized substance are within chemical substances. Substantially all chemical substances may be synthesized. Such synthetic technology is well known in the art, and those skilled in the art can produce chemical synthesized substance appropriately combining such technology.

As used herein, the term "biological activity" refers to activity possessed by an agent (e.g. , a polynucleotide, a protein, etc.) within an organism, including activities exhibiting various functions (e.g., transcription promoting activity, etc.). For example, when an agent is an antisense molecule, the biological activity thereof includes binding to a targeted nucleic acid molecule, suppression of expression thereby and the like. For example, when an agent is an enzyme, the biological activity thereof includes the enzymatic activity thereof. As for another example, when an agent is a ligand or a receptor, binding to the receptor or the ligand corresponding to the ligand or receptor, respectively, is included in the biological activity thereof. When the biological activity is transcriptional regulation activity, the activity refers to an activity for regulating transcriptional level or the variation thereof. For example, if an agent is a transposon, the biological activity thereof is transposition activity. Examples in which transposition activity is measured, include, for example, technologies described in the Examples, and such a biological activity may be determined by a well known technology in the art.

As used herein, "polynucleotides hybridizing under stringent conditions" refers to conditions commonly used and well known in the art. Such a polynucleotide can be obtained by conducting colony hybridization, plaque hybridization, Southern blot hybridization, or the like using a polynucleotide selected from the polynucleotides of the present invention. Specifically, a filter on which DNA derived from a colony or plaque is immobilized is used to conduct hybridization at 65°C in the presence of 0.7 to 1.0 M NaCl . Thereafter, a 0. 1 to 2-fold concentration SSC (saline-sodium citrate) solution (1-fold concentration SSC solution composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1 : Core Techniques, A Practical Approach, Second Edition, Oxford University Press (1995), and the like. Here, sequences hybridizing under stringent conditions exclude, preferably, sequences containing only A (adenine) or T (thymine). As used herein, "hybridizable polynucleotide" refers to a polynucleotide which can hybridize to other polynucleotides under the above-described hybridization conditions. Specifically, the hybridizable polynucleotide includes at least a polynucleotide having a homology of at least 60% to the base sequence of DNA encoding a polypeptide having an amino acid sequence as specifically set forth herein, preferably a polynucleotide having a homology of at least 80%, and more preferably a polynucleotide having a homology of at least 95%.

As used herein, the term "probe" refers to a substance for use in searching, which is used in a biological experiment, such as in vitro and/or in vivo screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90% or at least 95%.

As used herein, the term "search" indicates that a given nucleic acid sequence is utilized to find other nucleic acid base sequences having a specific function and/or property either electronically or biologically, or using other methods. Examples of an electronic search include, but are not limited to, BLAST (Altschul et al . , J. Mol. Biol . 215:403-410 (1990) ) , FASTA (Pearson & Lipman, Proc. Natl. Acad. Sci., USA 85:2444-2448 (1988)), Smith and Waterman method (Smith and Waterman, J. Mol. Biol. 147:195-197 (1981)), and Needleman and Wunsch method (Needleman and Wunsch, J. Mol. Biol. 48:443-453 (1970)), and the like. Examples of a biological search include, but are not limited to, a macroarray in which genomic DNA is attached to a nylon membrane or the like or a microarray (microassay) in which genomic DNA is attached to a glass plate under stringent hybridization, PCR and in situ hybridization, and the like.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength, and the concentration of denaturing agents such as formamide. Examples of "highly stringent conditions" for hybridization and washing are 0.0015 M sodium chloride, 0.0015 M sodium citrate at 65-68°C or 0.015 M sodium chloride, 0.0015 M sodium citrate, and 50% formamide at 42°C. See Sambrook, Fritsch & Maniatis, Molecular Cloning: A Laboratory Manual (2nd ed., Cold Spring Harbor Laboratory, N.Y., 1989); Anderson et al., Nucleic Acid Hybridization: A Practical Approach Ch. 4 (IRL Press Limited) (Oxford Express). More stringent conditions (such as higher temperature, lower ionic strength, higher formamide, or other denaturing agents) may be optionally used. Other agents may be included in the hybridization and washing buffers for the purpose of reducing non-specific and/or background hybridization. Examples are 0.1% bovine serum albumin, 0.1%polyvinylpyrrolidone, 0.1% sodium pyrophosphate, 0.1% sodium dodecylsulfate ($NaDodSO_4$ or SDS) , Ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are ordinarily carried out at pH 6.8-7.4; however, at typical ionic strength conditions, the rate of hybridization is nearly independent of pH. See Anderson et al., Nucleic Acid Hybridization: A Practical Approach Ch. 4 (IRL Press Limited, Oxford UK).

Agents affecting the stability of DNA duplex include base composition, length, and degree of base pair mismatch. Hybridization conditions can be adjusted by those skilled in the art in order to accommodate these variables and allow DNAs of different sequence relatedness to form hybrids. The melting temperature of a perfectly matched DNA duplex can be estimated by the following equation:

$$Tm\ (^{\circ}C) = 81.5 + 16.6\ (\log[Na^{+}]) + 0.41\ (\%\ G{+}C) - 600/N - 0.72\ (\%\ formamide)$$

where N is the length of the duplex formed, $[Na^+]$ is the molar concentration of the sodium ion in the hybridization or washing solution, % G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015 M sodium chloride, 0.0015 M sodium citrate at 50-65°C or 0.015 M sodium chloride, 0.0015 M sodium citrate, and 20% formamide at 37-50°C. By way of example, "moderately stringent conditions" of 50°C in 0.015 M sodium ion will allow about a 21% mismatch.

It will be appreciated by those skilled in the art that there is no absolute distinction between "highly stringent conditions" and "moderately stringent conditions". For example, at 0.015 M sodium ion (no formamide) , the melting temperature of perfectly matched long DNA is about 71°C. With a wash at 65°C (at the same ionic strength) , this would allow for approximately a 6% mismatch. To capture more distantly related sequences, those skilled in the art can simply lower the temperature or raise the ionic strength.

A good estimate of the melting temperature in 1 M NaCl for oligonucleotide probes up to about 20 nucleotides is given by:

$$Tm = (2^{\circ}C\ per\ A\text{-}T\ base\ pair) + (4^{\circ}C\ per\ G\text{-}C\ base\ pair).$$

Note that the sodium ion concentration in 6X salt sodium citrate (SSC) is 1 M. See Suggs et al., Developmental Biology Using Purified Genes 683 (Brown and Fox, eds., 1981).

A naturally-occurring nucleic acid encoding a protein (e.g., transposon, transposase or a variant or fragment thereof, or the like) may be readily isolated from a cDNA library having PCR primers and hybridization probes containing part of a nucleic acid sequence indicated by, for example, SEQ ID NO. 1, 2, 10, 12, 14, 16, 18 or the like. A preferable nucleic acid encoding chemical receptors, or variants or fragments thereof, or the like is hybridizable to the whole or part of a sequence as set forth in SEQ ID NO: 1, 2, 10, 12, 14, 16, 18 or the like under low stringency conditions defined by hybridization buffer essentially containing 1% bovine serum albumin (BSA) ; 500 mM sodium phosphate ($NaPO_4$) ; 1mM EDTA; and 7% SDS at 42°C, and wash buffer essentially containing 2xSSC (600 mM NaCl; 60 mM sodium citrate) ; and 0.1% SDS at 50°C, more preferably under low stringency conditions defined by hybridization buffer essentially containing 1% bovine serum albumin (BSA); 500 mM sodium phosphate ($NaPO_4$); 15% formamide; 1 mM EDTA; and 7% SDS at 50°C, and wash buffer essentially containing 1xSSC (300 mM NaCl; 30 mM sodium citrate); and 1% SDS at 50°C, and most preferably under low stringency conditions defined by hybridization buffer essentially containing 1% bovine serum albumin (BSA); 200 mM sodium phosphate ($NaPO_4$) ; 15% formamide; 1 mM EDTA; and 7% SDS at 50°C, and wash buffer essentially containing 0.5xSSC (150 mM NaCl; 15 mM sodium citrate); and 0.1% SDS at 65°C.

As used herein, the term "probe" refers to a substance for use in searching, which is used in a biological experiment, such as in vitro and/or *in vivo* screening or the like, including, but not being limited to, for example, a nucleic acid molecule having a specific base sequence or a peptide containing a specific amino acid sequence.

Examples of a nucleic acid molecule as a common probe include one having a nucleic acid sequence having a length of at least 8 contiguous nucleotides, which is homologous or complementary to the nucleic acid sequence of a gene of interest. Such a nucleic acid sequence may be preferably a nucleic acid sequence having a length of at least 9 contiguous nucleotides, more preferably a length of at least 10 contiguous nucleotides, and even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 20 contiguous nucleotides, a length of at least 25 contiguous nucleotides, a length of at least 30 contiguous nucleotides, a length of at least 40 contiguous nucleotides, or a length of at least 50 contiguous nucleotides. A nucleic acid sequence used as a probe includes a nucleic acid sequence having at least 70% homology to the above-described sequence, more preferably at least 80%, and even more preferably at least 90% or at least 95%.

As used herein, the term "primer" refers to a substance required for initiation of a reaction of a macromolecule compound to be synthesized, in a macromolecule synthesis enzymatic reaction. In a reaction for synthesizing a nucleic acid molecule, a nucleic acid molecule (e.g., DNA, RNA, or the like) which is complementary to part of a macromolecule compound to be synthesized may be used.

A nucleic acid molecule which is ordinarily used as a primer includes one that has a nucleic acid sequence having a length of at least 8 contiguous nucleotides, which is complementary to the nucleic acid sequence of a gene of interest. Such a nucleic acid sequence preferably has a length of at least 9 contiguous nucleotides, more preferably a length of at least 10 contiguous nucleotides, even more preferably a length of at least 11 contiguous nucleotides, a length of at least 12 contiguous nucleotides, a length of at least 13 contiguous nucleotides, a length of at least 14 contiguous nucleotides, a length of at least 15 contiguous nucleotides, a length of at least 16 contiguous nucleotides, a length of at least 17 contiguous nucleotides, a length of at least 18 contiguous nucleotides, a length of at least 19 contiguous nucleotides, a length of at least 20 contiguous nucleotides, a length of at least 25 contiguous nucleotides, a length of at least 30 contiguous nucleotides, a length of at least 40 contiguous nucleotides, and a length of at least 50 contiguous nucleotides. A nucleic acid sequence used as a primer includes a nucleic acid sequence having at least 70% homology to the above-described sequence, more preferably at least 80%, even more preferably at least 90%, and most preferably at least 95%. An appropriate sequence as a primer may vary depending on the property of the sequence to be synthesized (amplified) . Those skilled in the art can design an appropriate primer depending on the sequence of interest. Such a primer design is well known in the art and may be performed manually or using a computer program (e.g., LASERGENE, Primer Select, DNAStar).

As used herein, the term "epitope" refers to an antigenic determinant whose structure is clear. Therefore, the term "epitope" includes a set of amino acid residues which are involved in recognition by a particular immunoglobulin, or in the context of T cells, those residues necessary for recognition by T cell receptor proteins and/or Major Histocompatibility Complex (MHC) receptors. This term is also used interchangeably with "antigenic determinant" or "antigenic determinant site". In the field of immunology, in vivo or in vitro, an epitope is the feature of a molecule (e.g., primary, secondary and tertiary peptide structure, and charge) that forms a site recognized by an immunoglobulin, T cell receptor or MHC (e.g. HLA) molecule. An epitope including a peptide comprises 3 or more amino acids in a spatial conformation which is unique to the epitope. Generally, an epitope consists of at least 5 such amino acids, and more ordinarily, consists of at least 6, 7, 8, 9 or 10 such amino acids. The greater the length of an epitope, the more the similarity of the epitope to the original peptide, i.e., longer epitopes are generally preferable. This is not necessarily the case when the conformation is taken into account. Methods of determining the spatial conformation of amino acids are known in the art, and include, for example, X-ray crystallography and two-dimensional nuclear magnetic resonance spectroscopy. Furthermore, the identification of epitopes in a given protein is readily accomplished using techniques well known in the art. See, also, Geysen et al., Proc. Natl. Acad. Sci. USA (1984) 81: 3998 (general method of rapidly synthesizing peptides to determine the location of immunogenic epitopes in a given antigen) ; U.S. Patent No. 4,708,871 (procedures for identifying and chemically synthesizing epitopes of antigens); and Geysen et al., Molecular Immunology (1986) 23: 709 (technique for identifying peptides with high affinity for a given antibody). Antibodies that recognize the same epitope can be identified in a simple immunoassay. Thus, methods for determining an epitope including a peptide are well known in the art. Such an epitope can be determined using a well-known, common technique by those skilled in the art if the primary nucleic acid or amino acid sequence of the epitope is provided.

Therefore, an epitope including a peptide requires a sequence having a length of at least 3 amino acids, preferably at least 4 amino acids, more preferably at least 5 amino acids, at least 6 amino acids, at least 7 amino acids, at least 8 amino acids, at least 9 amino acids, at least 10 amino acids, at least 15 amino acids, at least 20 amino acids, and at least 25 amino acids. Epitopes may be determined by those skilled in the art by using commercially available kit, such as PepSet™ (Kurabo). In the present invention, presenting a protein epitope playing a role in signal transduction may be used as a system for measuring signal transduction.

As used herein, the term "agent binding specifically to" a certain nucleic acid molecule or polypeptide refers to an agent which has a level of binding to the nucleic acid molecule or polypeptide equal to or higher than a level of binding to other nucleic acid molecules or polypeptides. Examples of such an agent include, but are not limited to, when a target is a nucleic acid molecule, a nucleic acid molecule having a complementary sequence of a nucleic acid molecule of interest, a polypeptide capable of binding to a nucleic acid sequence of interest (e.g., a transcription agent, etc.), and the like, and when a target is a polypeptide, an antibody, a single chain antibody, either of a pair of a receptor and a ligand, either of a pair of an enzyme and a substrate, and the like. As used herein, such an agent specifically binding to (such as an agent specifically binding to calcium, an antibody against a specific gene product and the like), can be used in measuring signal transduction.

(Variation of polypeptides or polynucleotides)

In the present invention, when using a functional polypeptide such as a transposon, transposase and the like, a variant thereof (as used herein it is called "functional variant") may be used as long as the variant can attain similar functions, such as transposition activity and the like.

A given amino acid may be substituted with another amino acid in a protein structure, such as a cationic region or a substrate molecule binding site, without a clear reduction or loss of interactive binding ability. A given biological function of a protein is defined by the interactive ability or other property of the protein. Therefore, a particular amino acid substitution may be performed in an amino acid sequence, or at the DNA code sequence level, to produce a protein which maintains the original property after the substitution. Therefore, various modifications of peptides as disclosed herein and DNA encoding such peptides may be performed without clear losses of biological usefulness.

When the above-described modifications are designed, the hydrophobicity indices of amino acids may be taken into consideration. Hydrophobic amino acid indices play an important role in providing a protein with an interactive biological function, which is generally recognized in the art (Kyte, J. and Doolittle, R.F., J. Mol. Biol. 157(1):105-132, 1982). The hydrophobic property of an amino acid contributes to the secondary structure of a protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4) ; threonine (-0.7); serine (-0.8); tryptophan (-0.9); tyrosine (-1.3); proline (-1.6) ; histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within ±2, more preferably within ±1, and even more preferably within ±0 . 5 . It is understood in the art that such an amino acid substitution based on hydrophobicity is efficient.

A hydrophilicity index is also useful for modification of an amino acid sequence of the present invention. As described in US Patent No. 4,554,101, amino acid residues are given the following hydrophilicity indices: arginine (+3.0); lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within ±2, more preferably ± 1, and even more preferably ±0.5.

For example, it is well known in the art that the following RNA codon (therefore, in the corresponding DNA codon, T is replaced with U), can be interchangeably used for encoding each of the particular amino acids: phenylalanine (Phe or F) : UUU or UUC; leucine (Leu or L) : UUA, UUG, CUU, CUC, CUA or CUG; isoleucine (Ile or I): AUU, AUC or AUA; methionine (Met or M) : AUG; valine (Val or V) : GUU, GUC, GUA or GUG; serine (Ser or S) : UCU, UCC, UCA, UCG, AGU or AGC; proline (Pro or P): CUU, CCC, CCA or CCG; threonine (Thr or T) : ACU, ACC, ACA or ACG; alanine (Ala or A): GCU, GCG, GCA or GCC; tyrosine (Tyr or Y): UAU or UAC; histidine (His or H): CAU or CAC; glutamine (Gln or Q) : CAA or CAG; asparagine (Asn or N) : AAU or AAC; lysine (Lys or K) : AAA or AAG; asparatic acid (Asp or D) : GAU or GAC; glutamic acid (Glu or E) : GAA or GAG; cystein (Cys or C): UGU or UGC; arginine (Arg or R): CGU, CGC, CGA, CGG, AGA or AGC; glycine (Gly or G): GGU, GGC, GGA or GGG; termination codon: UAA, UAG or UGA. Further, a specific DNA sequence is modified to employ a preferential codon for a specific cell type. For example, preferential codon usage of E. coli, is known in the art, as is the preferential codon usage of an animal and a human. Such a modification is well known in the art, and constitutes a part of the present invention.

Variants thus produced are also within the scope of the present invention, and any of such variants are used in the present invention.

(Antigen and antibody)

As used herein, the term "antibody" encompasses polyclonal antibodies, monoclonal antibodies, human antibodies, humanized antibodies, polyfunctional antibodies, chimeric antibodies, and anti-idiotype antibodies, and fragments thereof (e.g., F (ab') 2 and Fab fragments), and other recombinant conjugates. These antibodies may be fused with an enzyme (e.g., alkaline phosphatase, horseradish peroxidase, a-galactosidase, and the like) via a covalent bond or by recombination.

As used herein, the term "monoclonal antibody" refers to an antibody composition having a group of homologous antibodies. This term is not limited by the production manner thereof. This term encompasses all immunoglobulin molecules and Fab molecules, F(ab')2 fragments, Fv fragments, and other molecules having an immunological binding property of the original monoclonal antibody molecule. Methods for producing polyclonal antibodies and monoclonal antibodies are well known in the art, and will be more sufficiently described below.

Monoclonal antibodies are prepared by using the standard technique well known in the art (e.g. , Kohler and Milstein, Nature (1975) 256:495) or a modification thereof (e.g., Buck et al. (1982) In vitro 18:377). Representatively, a mouse or rat is immunized with a protein bound to a protein carrier, and boosted. Subsequently, the spleen (and optionally several large lymph nodes) is removed and dissociated into a single cell suspension. If desired, the spleen cells may be screened (after removal of nonspecifically adherent cells) by applying the cell suspension to a plate or well coated with a protein antigen. B-cells that express membrane-bound immunoglobulin specific for the antigen bind to the plate, and are not rinsed away with the rest of the suspension. Resulting B-cells, or all dissociated spleen cells, are then induced to fuse with myeloma cells to form hybridomas. The hybridomas are used to produce monoclonal antibodies.

As used herein, the term "antigen" refers to any substrate to which an antibody molecule may specifically bind. As used herein, the term "immunogen" refers to an antigen capable of initiating activation of the antigen-specific immune response of a lymphocyte. Accordingly, chemical receptors or products of the downstream thereof may be used as an antigen or immunogen and uses antibody-antigen response to realize the sensor of the present invention.

(Gene Engineering)

As used herein, the term " gene cassette" refers to a nucleic acid sequence comprising a DNA encoding a gene, a nucleic acid sequence comprising a gene promoter operably linked thereto (such that it can control the expression of the DNA) and a promoter, and optionally a heterologous gene operably linked thereto (i.e., in frame) . It is intended that the use of this cassette optionally in combination of another regulatory element is encompassed in the present invention. Preferably expression cassettes are those which are amenable to a specific restriction enzymatic digestion and feasible recovery.

When a gene is mentioned herein, the term "vector" or "recombinant vector" refers to a vector transferring a polynucleotide sequence of interest to a target cell. Such a vector is capable of self-replication or incorporation into a chromosome in a host cell (e.g., a prokaryotic cell, yeast, an animal cell, a plant cell, an insect cell, an individual animal, and an individual plant, etc.), and contains a promoter at a site suitable for transcription of a polynucleotide of the present invention. A vector suitable for performing cloning is referred to as a "cloning vector". Such a cloning vector ordinarily contains a multiple cloning site containing a plurality of restriction sites. Restriction enzyme sites and multiple cloning sites as described above are well known in the art and can be used as appropriate by those skilled in the art depending on the purpose in accordance with publications described herein (e.g., Sambrook et al., *supra).*

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes, and enhancers. It is well known in the art that a type of an expression vector of a living organism such as an animal and a species of a regulatory element used may vary depending on the type of host cell used.

Examples of "recombinant vectors" for prokaryotic cells include, but are not limited to, pcDNA3 (+) , pBluescript-SK(+/-), pGEM-T, pEF-BOS, pEGFP , pHAT, pUC18, pFT-DEST™42GATEWAY (Invitrogen), and the like.

Examples of "recombinant vectors" for animal cells include, but are not limited to, pcDNAI/Amp, pcDNAI, pCDM8 (all commercially available from Funakoshi), pAGE107 [Japanese Laid-Open Publication No. 3-229 (Invitrogen), pAGE103 [J. Biochem., 101, 1307(1987)], pAMo, pAMoA [J. Biol. Chem., 268, 22782-22787(1993)], a retrovirus expression vector based on a murine stem cell virus (MSCV), pEF-BOS, pEGFP, and the like.

Examples of recombinant vectors for plant cells include, but are not limited to, pPCVICEn4HPT, pCGN1548, pCGN1549, pBI221, pBI121, and the like.

As used herein, the term "terminator" refers to a sequence which is located downstream of a protein-encoding region of a gene and which is involved in the termination of transcription when DNA is transcribed into mRNA, and the addition of a poly-A sequence. It is known that a terminator contributes to the stability of mRNA, and has an influence on the amount of gene expression.

As used herein, the term "promoter" refers to a base sequence which determines the initiation site of transcription of a gene and is a DNA region which directly regulates the frequency of transcription. Transcription is started by RNA polymerase binding to a promoter. Accordingly, a portion having promoter function of a gene herein refers to "promoter moiety" . A promoter region is usually located within about 2 kbp upstream of the first exon of a putative protein coding region. Therefore, it is possible to estimate a promoter region by predicting a protein coding region in a genomic base sequence using DNA analysis software. A putative promoter region is usually located upstream of a structural gene, but depending on the structural gene, i.e., a putative promoter region may be located downstream of a structural gene. Preferably, a putative promoter region is located within about 2 kbp upstream of the translation initiation site of the first exon. Promoters include, but are not limited to for example, constitutive promoters, specific promoters and inductive promoters.

As used herein, the term "enhancer" refers to a sequence which is used so as to enhance the expression efficiency of a gene of interest. One or more enhancers may be used, or no enhancer may be used.

As used herein, the term "silencer" refers to a sequence which has a function of suppressing and arresting the expression of a gene. Any silencer which has such a function may be herein used. No silencer may be used.

As used herein, the term "operably linked" indicates that a desired sequence is located such that expression (operation) thereof is under control of a transcription and translation regulatory sequence (e.g., a promoter, an enhancer, and the like) or a translation regulatory sequence. In order for a promoter to be operably linked to a gene, typically, the promoter is located immediately upstream of the gene. A promoter is not necessarily adjacent to a structural gene. "Operably linked" also refers ,when herein used to refer to signal transduction, to that each signal transduction molecule interacts directly or indirectly via another molecule to contribute to the signal transduction.

As used herein, technologies for introducing a nucleic acid molecule into a cell may be of any type, and include, for example, transformation, transduction, transfection and the like. Such a technology for introducing a nucleic acid molecule is well known in the art and is routinely used, and includes, for example, those described in Ausubel F. A. et al. ed. (1988), Current Protocols in Molecular Biology, Wiley, New York, NY; Sambrook J. et al. (1987) Molecular Cloning: A Laboratory Manual , 2nd Ed. and the third version thereof, Cold Spring Harbor Laboratory Press, Cold Spring Harbor, NY, Suppln. Experimental Medicine "Gene Introduction & Expression Analysis Experimental Procedure", Yodosha 1997. Introduction of genes may be confirmed by means of those described herein, such as Northern blotting, Western blotting analysis and other well known and routinely used technologies.

Methods of introducing a vector is also achieved by any of the above-mentioned methods for introducing a DNA into a cell, and include for example, transfection, transduction, transformation and the like, such as calcium phosphate, liposome methods, DEAE dextran methods, electroporation methods, particle gun methods (gene gun) , and the like, lipofection, spheroplast Proc. Natl. Acad. Sci. USA, 84, 1929 (1978)], lithium acetate method [J. Bacteriol., 153, 163 (1983)], a method described in Proc. Natl. Acad. Sci. USA, 75, 1929 (1978) and the like.

As used herein, the term "gene introduction reagent" refers to a reagent which is used in a gene introduction method so as to enhance introduction efficiency. Examples of such a gene introduction reagent include, but are not limited to, cationic polymers, cationic lipids, polyamine-based reagents, polyimine-based reagents, calcium phosphate, and the like. Specific examples of a reagent used in transfection include reagents available from various sources, such as, without limitation, Effectene Transfection Reagent (cat. no. 301425, Qiagen, CA), TransFast™ Transfection Reagent (E2431, Promega, WI), Tfx™-20 Reagent (E2391, Promega, WI) , SuperFect Transfection Reagent (301305, Qiagen, CA) , PolyFect Transfection Reagent (301105, Qiagen, CA), LipofectAMINE 2000 Reagent (11668-019, Invitrogen corporation, CA), JetPEI (x4) conc. (101-30, Polyplus-transfection, France) and ExGen 500 (R0511, Fermentas Inc., MD), and the like. In the present invention, such a gene introduction reagent may be used when introducing the nucleic acid molecule of the present invention into a cell.

Gene introduction efficiency may be calculated by measuring the cell number of introduction or expression the introduced foreign substance (introduced gene) (for example, gene product of a reporter gene, fluorescence protein GFP and the like) per unit area (for example, 1 $mm^2$ and the like); intensity of total signal (in case of fluorescence protein, fluorescence).

As used herein, the term "transformant" refers to the whole or a part of an organism, such as a cell or a tissue, which is produced by transformation. Examples of a transformant include a prokaryotic cell, yeast, an animal cell, a plant cell, an insect cell, and the like. Transformants may be referred to as transformed cells, transformed tissue, transformed hosts, or the like, depending on the subject, and may refer to any specific form depending on the context. Cells used in the present invention may be a transformant.

When a prokaryotic cell is used in genetic engineering in the present invention, prokaryotic cells include the following genera: Escherichia, Serratia, Bacillus, Brevibacterium, Corynebacterium, Microbacterium, Pseudomonas, for example, those species including Escherichia coli XL1-Blue, Escherichia coli XL2-Blue, Escherichia coli DH1, as examples. Alternatively, in the present invention, cells isolated from a naturally occurring substance may also be used.

Animal cells which can be used in genetic engineering or the like herein, include murine myeloma cells, rat myeloma cells, murine hybridoma cells, Chinese Hamster cells including CHO cell, BHK cell, African green monkey kidney cells, human leukemia cells, HBT5637 (see Japanese Laid-Open Publication 63-299), human colon cancer cell line and the like. Murine myeloma cells include ps20, NSO and the like; rat myeloma cells include YB2/0 and the like; human fetal kidney cells include HEK293 (ATCC: CRL-1573) and the like; human leukemia cells include BALL-1 and the like; African green monkey kidney cells include COS-1, COS-7 and the like; human colon cancer cell lines include HCT-15; human neuroblastoma cells include SK-N-SH, SK-N-SH-5Y and the like; murine neuroblastoma cells include Neuro2A and the like as examples. Alternatively, the present invention may use a primary cultured cell.

Plant cells which can be used in genetic engineering herein include callus, or a portion thereof and suspension culture cells, those cells from Solanaceae, Gramineae, Brassicaceae, Rosaceae, Leguminosae, Cucurbitaceae, Lamiacea, Liliaceae, Chenopodiaceae, Apiaceae and the like.

Gene expression (e.g., mRNA expression, polypeptide expression) maybe "detected" or "quantified" by an appropriate method, including mRNA measurement and immunological measurement. Examples of molecular biological measurement methods include Northern blotting methods, dot blotting methods, PCR methods, and the like. Examples of immunological measurement method include ELISA methods, RIA methods, fluorescent antibody methods, Western blotting methods, immunohistological staining methods, and the like, where a microtiter plate may be used. Examples of quantification methods include ELISA methods, RIA methods, and the like. A gene analysis method using an array (e.g. , a DNA array, a protein array, etc.) may be used. The DNA array is widely reviewed in Saibo-Kogaku [Cell Engineering], special issue, "DNA Microarray and Up-to-date PCR Method", edited by Shujun-sha. The protein array is described in detail in Nat Genet. 2002 Dec; 32 Suppl:526-32. Examples of methods for analyzing gene expression include, but are not limited to, RT-PCR methods, RACE methods, SSCP methods, immunoprecipitation methods, two-hybrid systems, in vitro translation methods, and the like in addition to the above-described techniques. Other analysis methods are described in, for example, "Genome Analysis Experimental Method, Yusuke Nakamura's Lab-Manual, edited by Yusuke Nakamura, Yodo-sha (2002) , and the like. All of the above-described publications are herein incorporated by reference.

As used herein, the term "expression" of a gene product, such as a gene, a polynucleotide, a polypeptide, or the like, indicates that the gene or the like is affected by a predetermined action in vivo to be changed into another form. Preferably, the term "expression" indicates that genes, polynucleotides, or the like are transcribed and translated into polypeptides. In one embodiment of the present invention, genes may be transcribed into mRNA. More preferably, these polypeptides may have post-translational processing modifications.

As used herein, the term "expression level" refers to the amount of a polypeptide or mRNA expressed in a subject cell. The term "expression level" includes the level of protein expression of a polypeptide evaluated by any appropriate method using an antibody, including immunological measurement methods (e.g., an ELISA method, an RIA method, a fluorescent antibody method, a Western blotting method, an immunohistological staining method, and the like, or the mRNA level of expression of a polypeptide evaluated by any appropriate method, including molecular biological measurement methods (e.g., a Northern blotting method, a dot blotting method, a PCR method, and the like). The term "change in expression level" indicates that an increase or decrease in the protein or mRNA level of expression of a polypeptide evaluated by an appropriate method including the above-described immunological measurement method or molecular biological measurement method.

Accordingly, as used herein, "reduction" of "expression" of a gene, a polynucleotide, a polypeptide or the like refers to when an agent of the present invention is subjected to an action, the amount of expression is significantly reduced compared to that when the agent is not subjected to an action. Preferably, the reduction of expression includes a reduction of the level of polypeptide expression. As used herein, the "increase" of "expression" of a gene, a polynucleotide, a polypeptide or the like refers to when an agent of the present invention is subjected to an action (or an agent relating to gene expression into a cell, for example, a gene to be expressed or an agent for regulating the same), the amount of expression is significantly increased compared to when the agent is not subjected to an action. Preferably, the increase of an expression includes an increase in the level of polypeptide expression. As used herein, the term "induction" of "expression" of a gene refers to an increase in the level of expression of the gene by acting an agent on a cell. Accordingly, the induction of expression encompasses the expression of the gene when no expression of the gene had been observed, and the increase in the level of expression of the gene when the level of the expression of the gene had already been observed.

As used herein, the term "specifically express (ing) " of a gene refers to expression in a different level (preferably in a higher level) in a specific site or period of time than that of the other site or period of time. Specific expression may refer to expression in a certain site (specific site) or may also refer to the expression including that in another site. Preferably, specific expression refers to the expression in the certain site only. A gene to be introduced into a biological organism by the present invention may be modified such that specific expression is achieved.

As used herein, the term "biological activity" refers to activity possessed by an agent (e.g. , a polynucleotide, a protein, etc.) within an organism, including activities exhibiting various functions such as transcription promoting activity.

When a collage interacts with the ligand thereof, the biological activity thereof encompasses formation of a conjugate or other biological change. In another embodiment, such a biological activity may be gene transposition activity and the like. Gene transposition activity may be determined by confirming the movement of a sequence encoding a gene of interest by any means. For example, when an agent is an enzyme, the biological activity thereof encompasses the enzymatic activity thereof. In another example, when an agent is a ligand, the activity encompasses the binding of the ligand to the receptor thereof. Such a biological activity may be determined by any well known technology in the art (see, for example, Molecular Cloning, Current Protocols, which is herein incorporated by reference).

As used herein, the term "kit" refers to a unit typically comprising two or more sections which provide portions (e.g., of a reagent, a particle, a cell, a nucleic acid and the like) . When components are not provided as a mixture and are preferably mixed immediately before use, this form of the kit is preferable. It is advantageous that such a kit preferably comprises instructions describing how to treat a portion to be presented (for example, a reagent, a particle and the like). Such instructions may be of any medium, and includes, but is not limited to, for example, paper-medium, transmitting medium, storage medium and the like. Transmitting media include, but are not limited to the internet, intranet, extranet, LAN and the like. Storage media include, but are not limited to CD-ROM, CD-R, flexible disk, DVD-ROM, MD, mini-disc, MO, memory stick and the like.

(Transgenic biological organism)

A general technique for producing transgenic mice is described in International Publication WO91-13150 (Ludwig Inst. Cancer Res.). US Patent No. 4,873,191 (Wagner et al.) teaches a mammal having an exogenous DNA, which was obtained by microinjection of the DNA into a mammalian zygote. Further, a method of efficiently producing mutants of an animal, a plant, or the like has been studied, in which a transposable genetic element (transposon) is inserted or transposed into an endogenous DNA so that the structure of the DNA is changed and the DNA is thus inactivated. Transposons have been available for introduction, addition, and the like of a particular gene into a chromosome.

In addition, a variety of methods for producing transgenic biological organisms include, but are not limited to those described in, for example: M.Markkula et al., Rev.Reprod., 1, 97-106 (1996); R. T. Wall et al., J. Dairy Sci., 80, 2213-2224 (1997); J. C. Dalton, et al . , Adv. Exp. Med. Biol., 411, 419-428 (1997) ; and H. Lubon et al., Transfus. Med. Rev., 10, 131-143 (1996), which are herein incorporated by reference, respectively.

In such circumstances, in the last ten years, analysis of transgenic (including knock-out, knock-in) animal via homologous recombination of embryonic stem (ES) cells is becoming of note for the purpose of analysis of gene functions.

In higher biological organisms, for example, efficient selection of recombinants by means of positive selection using the neomycin resistance gene and negative selection using the HSV thymidine kinase gene or the diphtheria toxin gene are known. Homologous recombinants are selected by PCR or Southern blotting method, i.e., a portion of a target gene is replaced with the neomycin resistance gene or the like for positive selection, and at the bottom thereof, targeting vectors in which the HSVTK gene or the like is linked for negative selection at the terminus, to introduce the same into an ES cell by electroporation, and selected in the presence of G418 and gancyclovir. The resultant colonies are isolated and selected for homologous recombinants by means of PCR or Southern blotting.

As such, a method for producing a transgenic (targeted gene recombination) mouse having substitution or disruption of a internal target gene, and having lost the function thereof or having altered the mutation, is useful since mutations are introduced only in the gene which is targeted for the analysis of the gene function. Use of the present invention further enhances the transposition efficiency by methylation and thus the analytical efficiency of a gene will be greatly enhanced.

After selection of a desired homologous recombinant, the resultant recombinant ES cell is mixed with a normal embryo by the blastcyst injection method or the collection chimeric method to produce a chimeric mouse between the ES cell and the host embryo. In the blastcyst injection method, ES cells are injected into the blastcyst by a glass pipette. In the collection chimeric method, the mass of ES cell and an embryo of the eight-cell phase, which has a removed clear zone, are fused. The blastcyst with the ES cell introduced therein is transferred to the uterus of a pseudo pregnant surrogate mother to produce a chimeric mouse. Since an ES cell has totipotency, it can differentiate in to any type of cell including a germline cell in vivo. When a chimeric mouse having the germline cell derived from the ES cell and a normal mouse are crossbred, then a mouse having the chromosome of ES cell in a heterologous manner, and a transgenic mouse having the modified chromosome of ES cell in a homologous manner shall be obtained by crossbreeding the mice to each other. In order to obtain a transgenic mouse having the modified chromosome in a homologous manner from the resultant chimeric mouse, a male chimeric mouse and a female wild-type mouse is crossbred to produce a F1 generation of heterozygous mouse, and the resultant male and female heterozygous mice are crossbred and select homozygous mouse in the F2 generation. Whether or not a desired gene mutation is introduced in each generation of F1 and F2, any methods routinely used in the art such as Southern blotting, PCR, sequencing, and the like as in the assays for recombinant ES cells.

However, the production technology of a transgenic animal being presently conducted has a defect in that it is difficult to selectively analyze a variety of gene functions. There is also the disadvantage in that transgenic biological organism cannot be readily produced.

Further, production of present transgenic animals requires disruption from initiation or disruption and replacement with respect to a desired gene after identification of such a desired gene as described above. Thus, it requires extreme labor and time, and even those researchers familiar with the technology do not always succeed. Accordingly, it is still a labor-intensive operation.

As such, in order to overcome the problem in which a variety of gene functions cannot be selectively analyzed, it is of note that Cre recombinase cell-type specific expression and Cre-loxP site specific recombination are combined. Transgenic mice using Cre-loxP are produced by introducing the neomycin resistance gene in a location, such that the expression of a target gene is not blocked, introducing a targeting vector into an ES cell, the vector containing the loxP sequence introduced therebetween in a manner such that the exon to be deleted is sandwiched, and isolating the homologous recombinant. The isolated clone is used to obtain a chimeric mouse, and a genetically modified mouse is produced. Next, when the mouse is cross bred with a transgenic mouse, which tissue-specifically expresses site specific recombinant enzyme Cre from P1 phage of *E. coli,* genes are disrupted only in the tissue expressing Cre (herein, Cre specifically recognizes loxP sequence (34bp) to raise recombination between the two lox P sequences, resulting in the disruption thereof. It is now possible to express Cre in an adult by crossbreeding the adult with a transgenic mouse having the Cre gene linked to an organ specific promoter or using a viral vector having the Cre gene. Further, the present invention is used to enhance the transposition efficiency by methylation, and thus the analytical efficiency of genes will be greatly enhanced.

The gene trapping (gene trap) method is of note as a method for analyzing a specific gene. In the gene trapping method, a reporter gene having no promoter is introduced into a cell, and once the gene is inserted into the genome in an accidental manner to express the reporter gene, which is used to isolate (trap) a novel gene. The gene trapping method is a method for efficient insertion mutation and identification of unknown gene, based on mouse primary embryo operation method, embryonic stem cell culture method and a gene targeting method via homologous recombination (Stanford WL., et al., Nature Genetics 2:756-768(2001)). The gene trap method allows introduction of a gene, selection of a mutant and analysis of the phenotype with relative ease.

In gene trap method, for example, a gene trapping vector having beta-geo, a fusion gene between lacZ and neo, has been linked between the splicing/acceptor sequence and the polyA addition signal, is introduced into an ES cell, and selected with G418 to allow selection of the clones which have accidentally trapped the gene expressed by the ES cell.

Production of a chimeic embryo from thus a obtained clone, a variety of X-gal stain patterns will be shown depending on the expression patterns of the genes. As such, in the gene trapping method, unknown genes will be isolated and analyzed for the gene expression patterns thereof or the gene is disrupted. The present invention is used for enhancing transposition efficiency by methylation and the analytical efficiency of genes will also be greatly enhanced.

According to the present invention, a "transgenic biological organism having a transposase gene" and a "transgenic biological organism containing a nonself-contained transposon" can be crossbred to obtain a "biological organism containing the transposase gene and the transposon". As used herein the term "non-self completing transposon" refers to one which is not transposable *per se.* The term "self-completing transposon" refers to one which can transpose *per se.* With this method, similar parents can be crossbred to obtain child mammals having an identical gene. This method makes it possible to know in advance the influence of introduction of only a transposon construct into a mammal on a phenotype of the mammal. Similarly, the method makes it possible to know in advance the influence of introduction of only a transposase construct into a mammal on a phenotype of the mammal. Alternatively, it is possible to obtain a "transgenic biological organism containing a transposase gene and a transposon" into which the transposase gene and the transposon are initially introduced without crossbreeding. This method does not require crossbreeding of parents, resulting in good efficiency in terms of labor, time, and cost. Accordingly, the present invention allows application of a production method for such a transgenic biological organism.

In this "transgenic biological organism containing a transposase gene and a transposon", since the transposon is contained in a state that allows the transposon to be transposed, the transposon can be transposed on a chromosome. This transposition can disrupt, lower, or activate gene function at any site on the chromosome.

Further, a "transgenic biological organism containing a transposase gene and a transposon" and a "biological organism containing no transposase" can be crossbred to obtain a "biological organism having a transposon but no transposase gene". If a transposase is interposed by loxPs, a biological organism containing Cre may be used for crossbreeding.

In a preferable embodiment, a "biological organism, in which substantially all cells thereof contain (i) at least one transposase gene and at least one nonself-contained transposon or self-contained transposon and (ii) at least one signature site" corresponds to a "transgenic biological organism having both a transposon sequence (TP) containing GFP as an arbitrary component and a transposase gene (SB) (hereinafter also referred to as a "TP-SB mammal") " . This transgenic biological organism is obtained by crossbreeding a "biological organism having a transposon sequence (TP) containing GFP as an arbitrary component but no transposase gene (SB) (hereinafter also referred to as a "TP (biological) organism mammal")" and a "biological organism having a transposase gene (SB) but no transposon sequence (hereinafter also referred to as an "SB (biological) organism")".

In one embodiment, the biological organism of the present invention is induced from a stem cell or a fertilized egg having a transposon or a signature site. Therefore, essentially all cells contain a transposase gene. In fact, a transposon may not leave a signature site when the transposon is excised and a transposon may not be transposed. Therefore, item 2 describes "substantially all cells". "Substantially all cells" is meant all cells except for such a particular cell (s). In each cell of the above-described biological organism, a transposon is randomly transposed. For this reason, no uniform mutation is found in a whole individual among genetic mutations introduced by the transposon.

In another embodiment, of the transgenic biological organisms of the present invention, a transgenic biological organism obtained by crossbreeding a "TP-SB biological organism" and a "biological organism containing no transposase" is a transgenic biological organism in which a genetic mutation based on a signature sequence of the "TP-SB biological organism" has already been present since the fertilized egg stage and the common signature site is contained in substantially all cells of the biological organism individual.

In the present invention, a desired transgenic biological organism may be obtained by prescreening. As a prescreening method, a gene trap method can be used, for example (Zambrowicz et al . , ; Nature, 392:608-611 (1998); Gossler, A. et al.; Science, 244:463-465 (1989); Skarnes, W.C. et al.; Genes Dev, 6:903-918 (1992); and Friedrich, G. et al.; Genes Dev, 5:1513-1523 (1991)).

Thus, pre-screening is performed to select in advance transgenic biological organism desirable for clarification of gene function. Thereafter, crossbreeding over two or more generations or other appropriate means can be performed to obtain a transgenic biological organism in which both genes on a pair of chromosomes are mutated.

A method of analyzing the phenotype of a gene by disrupting the gene is an effective means for clarifying gene function. There are two big problems to be overcome in order to analyze phenotypes by exhaustive gene disruption for a mammal individual, particularly a mouse. The first problem is that there is no satisfactory technique for exhaustively disrupting genes so as to investigate gene function from phenotypes, i.e., so-called forward genetics. The second problem is that since there are a pair of genes (both alleles) , a phenotype does not appear if only one member of the pair of genes is disrupted. Currently, individuals having one disrupted member of a pair of genes are crossbred in order to introduce a mutation into both alleles. In other words, a long time is required for crossbreeding to obtain an individual in which a mutation is introduced into both alleles.

The first problem can be overcome by a transposon system newly developed in the present invention. The second problem can be overcome by a method of rapidly introducing a mutation into both alleles. As a specific method for overcoming the second problem, a Bloom gene knockout mouse, in which cells having a mutation in both alleles frequently appear, can be used (G. Luo et al.; Nature Genetics, 26:424-429 (2000) ) . Note that a perfect Bloom gene knockout mouse is fatal (N. Chester et al.; Gene and Dev., 12:3382-3393 (1998)), and therefore, the second problem may not be overcome. The present inventors are producing a mouse, in which expression of the Bloom gene can be arbitrarily adjusted, using a tetOFF system (CT. Bond et al.; Science, 289:1942-1946 (2000)). The Bloom gene encodes DNA helicase. If the activity of DNA helicase is lost, sister chromatid exchange (SCE) occurs, and at the same time, exchange with another chromatid occurs. Therefore, the lack of the bloom gene causes recombination in tetraploids. In this case, a cell in which both members of a pair of genes are mutated may occur in a part of an individual.

For example, if the Bloom gene is switched ON/OFF in a tetracycline dependent manner (A. Kistner et al. ; Proc. Natl. Acad. Sci. USA, 93:10933-10938 (1996)), by adjusting the time of supplying tetracycline or the time of supplying no tetracycline, recombinations can be more frequently induced in a time-specific manner so that a cell having a pair of mutated genes is likely to occur. Therefore, a non-human mammal having a pair of mutated genes can be obtained without repetition of crossbreeding. As a method of introducing mutation in a time-specific manner, for example, pellets are continuously administered orally into a non-human mammal so that a fetus having a pair of mutated genes can be obtained.

A means for regulatably expressing the Bloom gene (e.g., a tetracycline regulatable unit) is introduced in combination with a transposon system. For example, before crossbreeding, a means for regulatably expressing the Bloom gene is introduced into a fertilized egg or the like, into which a transposon construct, a transposase, a self-contained transposon, or the like is to be introduced. The obtained mouse having an introduced transposon transposition site is treated with a means for inhibiting expression of the Bloom gene (e.g., administration of tetracycline) so that a genetic mutation obtained by a transposon system is introduced into both alleles, thereby making it possible to rapidly determine phenotype. In the present invention, when no selectable marker gene is used, DNA may be extracted from cells of a non-human mammal and may be then screened by investigating the presence or absence of transposition by southern blotting. According to the present invention, it is possible to achieve efficient transposition of a transposon sequence in animals in vivo. Non-human mammals having various phenotypes can be efficiently and randomly obtained by a method of introducing mutation using a transposon, as compared to other methods. The transgenic non-human mammal of the present invention provides a considerably useful tool for clarifying complex life processes in gene function research since various genetic mutations can be introduced.

The frequency of expression of a transposon in cells is 3.5x10-$^{5}$ per cell at maximum, which is considerably low, as described in Proc. Natl. Acad. Sci. USA, vol. 95:10769-10773 (1998). In contrast, according to the present invention, the frequency of transposon expression among individuals, for example, was 42% of all mice and 80% (at maximum) of GFP gene-positive mice in an Example below. These values are significantly high. The present invention is the first to find that the transposition efficiency of a transposon was dramatically increased by the transposon expression system made of an aggregate of cells, such as animals or tissue, organs, and the like thereof.

According to one embodiment of the present invention, it is possible to screen transgenic non-human mammals having an introduced transposon construct for individuals having a randomly introduced mutation using a marker or other means. This is useful as means for clarifying gene function. For exhaustive analysis of gene function, it is necessary to cause a transposon to be transposed to a greater number of sites on a genome. According to item 2 of the present invention, it is possible to obtain a "transgenic non-human mammal, in which substantially all cells thereof contain (i) at least one transposase gene and at least one nonself-contained transposon or self-contained transposon and (ii) at least one signature site". For example, according to an example of the present application using mice, a signature site could be introduced at a rate of at least one per 10 cells, resulting in "seed mice" having a variety of cells in a mosaic pattern. Therefore, by producing mutated mice from different seed mice, it is possible to exhaustively introduce a mutation into substantially all genes the number of which is believed to be at least about 30,000. Therefore, in analysis of non-human mammals having mutations, since the present invention can achieve a considerably high level of expression frequency of genetic mutations, a number of functional changes by mutations can be simultaneously analyzed from if a single non-human mammal individual having a plurality of mutations is obtained. Thus, gene function can be highly efficiently clarified. When mice are used for introduction of mutations, for example, only one transgenic mouse is obtained from a single cell by a conventional method of introducing a mutation into an ES cell. The present invention improves transfer efficiency by methylation, and thus dramatically increases the level of gene analysis efficiency.

On the other hand, according to the transgenic non-human mammal production method of the present invention, when a "transgenic non-human mammal (e.g., a mouse) , in which substantially all cells thereof contain at least one selected from the group consisting of at least one nonself-contained transposon and at least one signature site" is used as a seed mouse, it is possible for the single individual to give birth to 100,000 types of transgenic individuals. In other words, the present invention has merit in that a single mouse can give birth to offspring having a huge number of types of transpositions. Thus, it is possible to obtain various mutated individuals for clarification of life processes.

According to the present invention, by obtaining and crossbreeding transgenic non-human mammals, it is possible to obtain non-human mammals having fixed transpositions, which are useful for clarification of gene function. As used herein, "fixed transposition" means that the number of signature sites produced by transposition of a transposon is not increased due to the lack of an active transposase. Specifically, this indicates either the case where at least one signature site and a transposon are present but no or an inactivated transposase (s) is present or the case where at least one signature site is present but no transposon is present. If such a transgenic mammal individual is obtained, a type of gene function can be simply analyzed by investigating a corresponding single individual. It is also possible to investigate an individual having a particular mutation about the influence of the mutation on the individual during the course of it's growth. Among the above-described non-human mammals, a non-human mammal lacking a certain function can be used to confirm a causative gene involved in a particular function as follows. For example, a transposon sequence containing a splice acceptor is used to add a transposase into a fertilized egg of the non-human mammal or crossbreed the non-human mammal with a non-human mammal having a transposase so as to remove the transposon sequence. It is then determined whether the function can be recovered as a result of the removal of the transposon sequence. In the present invention, mutations are introduced by transposons. Therefore, mutation introduction sites can be easily detected by an appropriate method, such as PCR or the like, using a signature sequence or a sequence derived from a transposon construct, as compared to when a mutation is introduced using a mutation inducing substance or the like. In an embodiment of the present invention, by introducing a genetic mutation into a non-human mammal individual, but not culture cells, it is possible to analyze gene function in individuals. It is also possible to introduce a genetic mutation into in vivo tissue of a non-human mammal individual, which is difficult to handle while the non-human mammal individual remains alive, without external manipulation. Further, transposition sites differ even within the same tissue, so that there are genetically different cells. Therefore, the lineage of cells, such as proliferation, differentiation, and the like, can be systematically investigated in any tissue and organs, such as the blood system, the immune system, and the like.

According to the present invention, a novel biological organism (particularly, a mouse) of the present invention provides a model system useful for clarification of gene function. This embodiment of the present invention may provide a model system of disease for studies on genetic disease in in vivo animal models. In the system, examples of disease genes to be introduced into animal models include human disease causative genes, homologous genes of biological organisms with the human disease causative genes, full-length cDNA genes, cDNA gene fragments, full-length genomic DNA genes, and genomic DNA gene fragments. Such a disease causative gene is not particularly limited. Any disease causative gene can be used as long as it can be introduced into biological organisms and the resultant transgenic biological organisms can be studied as animal models of human disease. Human disease causative genes are preferable. According to one embodiment of the present invention, when a transposon containing various enhancers is transposed near proto-oncogenes, cancer is eventually expressed in cells containing these genes. Therefore, it is possible to perform screening for proto-oncogenes. In particular, when a transgenic biological organism containing a transposon sequence and a transposase gene is used, cancer undergoes metastasis over the whole body as well as tissue since proto-oncogenes are clonally expressed. At the same time, reduction, disruption, or activation of gene function due to transposition randomly proceed in each animal cell. It is expected that a plurality of cancers occur in the same individual. Therefore, clarification of gene function involved in cancer can be efficiently developed. Further, when a plurality of cancers are confirmed in the same individual, it is possible to investigate whether or not cancerous cells are derived from the same cell by investigating whether or not the insertion site of a transposon vector is the same for the cancerous cells. Thus, the present invention may contribute to research on the mechanism of cancer metastasis.

According to a third embodiment, the transgenic biological organism of the present invention may be used as a donor for organ transplantation. Examples of organs which are considered to be used as donors for heterograft to a human, include neurons, heart, lung, liver, pancreas, kidney, cornea, skin, and the like. In this case, as an introduced gene, a gene having a function of possibly reducing rejection or a gene having a function of expectably increasing acceptance are preferable in heterograft, for example.

For production of transgenic biological organisms, refer also to: those references including, but not limited to: US patent Nos.: 5,464,764; 5,487,992; 5,627,059; Japanese Laid-Open Publication 2001-54337; Gossler, A. et al. (1989), Science 244, 463-465; Wurst, W. et al. (1995), Genetics 139, 889-899; Zambrowicz, B. P. et al. (1998), Nature 392, 608-611 Proc.Natl. Acad. Sci. USA, Vol. 86, 8932-8935, 1989; Nature, Vol. 342, 435-438, 1989; M. Muramatsu and M. Yamamoto ed. "Jikken Igaku Bessatsu, Shin-tei, Idenshi Kogaku Handobukku Kaitei Daisanhan" (Experimental Medicine, Suppl. New Revision, Gene Engineering Handbook, Third Edition" (1999, Yodosha) , in particular, pages 239-256; S. Aizawa (1995) Jikken Igaku "j iin taagettingu - ES saibo wo mochiita hen' i mausu no sakusei" (Experimental Medicine, Gene Targeting - production of mutant mouse using ES cell) and the like.

As used herein the term "knock out", when referring to a gene, refers to rendering the disruption (deletion) of the gene or rendering function the gene deficient. Accordingly, the concept of knock out is encompassed by transgenic.

As used herein, the term "knock-out biological organism" refers to a biological organism (for example, mouse) in which a gene is knocked out. Accordingly, the concept of knock-out biological organism is encompassed by a transgenic biological organism.

As used herein the term "biological organism" which is the object of the transgenic biological organism, encompasses any biological organism for which a transposon acts, and in which such a transgenic system can function. Such a biological organism includes, but is not limited to an animal, a plant, a bacteria and the like.

As used herein the term "animal" refers to any animal, which can be targeted by the introduction of a nucleic acid sequence (preferably a foreign sequence encoding a gene). Accordingly, an animal includes a vertebrate and invertebrate. An animal includes for example, mammals (for example, mouse, dos, cat, rat, monkey, pig, cattle, sheep, rabbit, dolphin, whale, goat, horse and the like), birds (for example, chicken, quail and the like) , amphibian (for example, frog and the like) , reptiles, insects (for example, Drosophila and the like) , and the like. Preferably, an animal may be a mammal, and preferably, an animal, which is amenable to production of a knock-out biological organism (for example, mouse) . In another preferable embodiment, an animal may be an animal which is known to be appropriate as a human model animal (for example, monkey). In an embodiment, an animal may be, but is not limited to: non-human animal or non-human mammal. An animal may be, for example, pig, monkey, cattle, horse, goat, sheep, cat, dog, rabbit, mouse, rat, or hamster and the like, and more preferably, mouse or rat. As used herein, the biological organism of the present invention, unless otherwise stated, includes not only mammalian individuals, but also a part of an individual, or organs or tissue possessed by an individual. These may be useful as a human disease model or a donor for organ transplantation.

As used herein the term "plant" collectively refers to an organism belonging to the kingdom of *Plantae* and is typically characterized in chlorophyl, hard cell wall, presence of abundant permanent embryonal cells, and incapability of movement or the like. Typically, plant refers to *Phanerogamae* having formatino of cell wall an anabolism action by chlorophyll. "Plant" encompasses both monocotyledonous plant and dicotyledonous plant. Preferably plants include, but are not limited to, for example, monocotyledonous plants belonging to Gramineae such as rice, wheat, maize, barley, sorghum, and the like. Preferably, plant may be rice. Rice includes but is not limited to *japonica* and *indica* variants. More preferably, rice may be *japonica* variant. As used herein variants of rice include but are not limited to, for example, Nipponbare, Nihonmasari, Kinmaze, Norin No. 22, Chiseiasahi, Koshihikari, Akitakomachi, Dontokoi, Hinohikari and the like. *Indica* variants include, but are not limited to Tetep, Basmati, IR8, Hunanzao, and the like. Preferable plants are not limited crops, but also flowers, trees, turfs, weeds and the like. Unless otherwise stated, plant refers to any of plant body, plant organ, plant tissue, plant cell, and seed. Examples of plant organs include root, leaf, stem and flower and the like. Examples of plant cells include callus and suspended culture cells.

Examples of Gramicear plants include plants belonging to Oryza, Hordenum, Secale, Scccharum, Echinochloa, or Zea, and include rice, barley, rye, Japanese millet, sorghum, maize and the like.

Plants used for a method for production according to the present invention are preferably monocotyledonous plant, and more preferably Gramineae plant. More preferably, it may be rice.

In the above-mentioned organisms, introduction technology of a gene includes a method selected from the group consisting of microinjection, a combination of a nucleic acid fragment and a cationic lipid vesicle or DNA aggregation reagent, and introduction of a nucleic acid fragment to a viral vector followed by contact with a cell with the virtual vector, and particle bombardment and electroporation.

Viral vectors which may be used herein, include but are not limited to: retroviral vector, adenovirus vector, herpes virus, and adeno-associated vector and the like.

As used herein the term "retrovirus" refers to a virus which has a genetic information in the form of RNA, and synthesize a DNA from the information of the RNA via reverse transcripitase. Accordingly, "retroviral vector" refers to a form of a retrovirus which is used as a vector for a gene. "Retroviral vectors" as used herein include, but are not limited to, for example, retroviral type expression vector based on Moloney Murine Leukemia Virus (MMLV), Murine Stem Cell Virus(MSCV) and the like.

Preferably, retroviral vectors include, but are not limited to: pGen-, pMSCV and the like.

As used herein the term "gene trap (method) " refers to a method for identification of a gene using the fact that a desired cell is introduced with a reporter gene lack of promoter, for example, and reporter activity is only detected when the reporter gene is inserted downstream of a promoter in an activated form in the chromosome. Such a gene trap is achieved by introducing a "gene trap vector" into the host chromosome of a eukaryotic organism and disrupting the host gene. A gene which was introduced with a reporter gene, expresses a complex protein with a reporter, and thus it is capable of identifying a gene by monitoring the protein. Accordingly, a reporter gene is incorporated into the original locus as in the homologous recombination, it is possible to produce a complete reporter system with respect to the transcription regulation. By means of these methods, it is possible to identify a gene which cannot be obtained by a method for isolation of a mutants via gene disruption. Accordingly, the present invention can use of these gene trapping method.

As used herein the term "gene trap vector" refers to a vector for selection of a vector inserted into a gene, using a phenomenon in which in the process of mRNA of a eukaryotic organism gene is matured into a mature mRNA, splicing mechanism is taken place. Gene trap vectors include, but are not limited to (1) a vector comprising a coding region of a reporter gene having no promoter, and a DNA sequence comprising a splice-acceptor sites, or (2) a vector comprising a coding region of a reporter gene having promoter, and a DNA comprising a splice-donor sites, and (3) a vector comprising the DNA sequence of both (1) and (2), and the like.

Gene trapping vectors comprising splice/acceptor sequence as described above, may comprise polyA addition signal as necessary. A gene trapping vector comprising a splice/donor sequence may comprise enhancer region, and/or mRNA instability region, as necessary. PolyA addition signal includes, but is not limited to: "AATAAA".

Promoters used in the present invention include but are not limited to: MC1 promoter, RNA pol II promoter and the like.

Enhancers used in the present invention include but are not limited to polyoma viral enhancer (PYF441) and the like.

Splice donor sequence used in the present invention include but are not limited to murine hprt gene exon 8 splice donor.

Splice acceptor sequence used in the present invention include, but are not limited to human bcl-2 gene exon 3 splice acceptor.

As used herein the term "reporter" molecule or "reporter" gene refer to a molecule (e.g. polypeptide) or gene which can be used as an indicative of gene expression in a cell. Such a molecule may be of known reporter protein, and includes, but is not limited to, for example, chloramphenicol acetyl transferase (CAT), beta-glucuronidase (GUS), beta-D-galactosidase, luciferase, green fluorescence protein (GFP) , or aequorin and the like As used herein, a method for introducing a gene *per se* may be achieved by means of desired material using known technology in the art. In such a case, for example, an embryonic stem cell of interest was introduced with a reporter gene free of a promoter (e.g., luciferase, green fluorescence gene, beta-galactosidase gene (lacZ), alkaline phosphatase gene, Cre recombinase gene and the like), and reporter activity will only be detected when inserted downstream of an activated promoter on the chromosome. Vectors used may include, for example, the presently mentioned reporter gene, selectable marker gene (e.g., neomycin resistant gene, hygromycin resistant gene, puromycin resistant gene, rescue marker gene (e.g., ampicillin resistant gene and collicin E1 replication origin) and the like. Selectable marker gene is used for selecting a host with the vector. Rescue marker gene is used for rescuing a vector (see Joyner, A. L. ed. "Gene Targeting, 2nd edition"(Oxford University Press,2000)). Using technologies as described above, an embryonic stem cell is produced. The modified embryonic stem cell has trapped a gene. As used herein the term "trap" refers to the state where an internal gene is disrupted by insertion of a trapping vector into the genome, and the gene disrupted by the gene is marked at the same time.

Preparation of an oligonucleotide having a specific sequence may be achieve by any well known technology in the art and include, but are not limited to: e.g. those described in Joyner, A. L. ed. "Gene Targeting, 2$^{nd}$ edition"(Oxford University Press,2000). Oligonucleotides are labeled as necessary with a fluorescence, radiolable and the like. Such a labeling method are well known in the art, and described in the references herein cited.

(Screening)

As used herein, the term "screening" refers to selection of a target, such as an organism, a substance, or the like, a given specific property of interest from a population containing a number of elements using a specific operation/evaluation method. For screening, a method or system of the present invention may be used. In the present invention, as a variety of transgenic biological organisms are produced, any nucleic acid molecule and a functional regulation agent may be screened.

In the present invention, any nucleic acid molecules may be screened by means of a nucleic acid molecule, a method or a system of the present invention. The present invention is also intended to comprise chemicals identified by the screening or the combination thereof.

A transposon system according to the present invention may be used in a variety of fields. For example, 1) the present invention is used to efficiently insert genetic material into a chromosome of a biological organism; 2) transposon is used as an insertion mutation agent to identify, isolate and characterize the genes relating to growth, maintenance, regulation and development of an organism (e.g. Kaiser et al., 1995 "Eukaryotic transposable, elements as tools to study gene structure and function" Mobile Genetic Elements, IRL Press,pp.69-100) ; 3) it is possible to identify, isolate and characterize the transcriptional regulatory factors relating to growth, maintenance, regulation and development of an organism (e.g., Anderson et al., 1996, Mol. Mar. Biol. Biotech., 5, 105-113). As an example, a method and system of the present invention may be used to produce a germ-free transgenic mouse. Litter-mates having an activated gene are crossbred to allow production of germ-free ascendants for biological containment or maximizing the growth rate.

(Genetic Therapy)

Use of the present invention includes incorporation of a gene for genetic therapy to a cell by modifying nucleic acid fragment. Such a gene is located under the control of a tissue-specific promoter or universal promoter, or under the control of one or more other expression controlling regions for expression of a gene in a cell requiring the gene. Genes used for genetic therapy include but are not limited to, for example, CFTR gene for cystic fibrosis, alpha-1-antitrypsin for lung diseases, adenosinaminase (ADA) for immunological diseases, Factor IX and interleukin-2 (IL-2) for blood cell diseases, and tumor necrosis factor (TNF) for cancer treatment and the like.

Gene sequence possibly used for genetic therapy can be obtained by searching a known database such as GenBank, DDBJ, EMBL and the like.

Further, the present invention may be used for operating or screening a library or a part thereof, evaluating a function of a sequence, or screening a protein expression, evaluating effects of a particular protein or a particular expression controlling region on a particular cell type. In one embodiment, libraries of recombinant sequences, for example, those products of combinatorial library or gene shuffling can be incorporated in to the nucleic acid fragments of the present invention to produce a library of nucleic acid fragment having a variety of nucleic acid sequences located between certain inverted repeat sequences. Next, this library is introduced into a cell with a transposase such as the SB protein as described above.

The advantages of the present invention is that the complete use of the advantages in the transposon transgenic biological organism production system which is free of size limitations of a mediated nucleic acid sequence located between the inverted repeat sequences. This is extremely enhanced by methylation. The SB protein is used for incorporation of a transposon of 1.3 kilobases (kb) to about 5.0kb. The mariner transposase was used to transpose about 13kb. There is no known limitation to the size of the nucleic acid sequence which can be incorporated into a cell of a DNA by means of SB protein.

BEST MODE FOR CARRYING OUT THE INVENTION

Hereinafter, preferable embodiments for carrying out the present invention are described. The embodiments provided below are only intended for better understanding of the present invention, and thus it should be understood that the scope of the present invention should not be limited to the description of the following section.

In an aspect, the present invention provides an isolated nucleic acid molecule having a nucleic acid sequence encoding a transposon, wherein the nucleic acid sequence has a methylation at at least one nucleotide. Such a nucleic acid molecule, unexpectedly retains transposon activity and can be activated in the host, which are significant effects. In the present invention such an effect allows production of a transgenic biological organism for any type of organism, which is a significant utility.

In a preferable embodiment, the nucleic acid of the present invention has a nucleic acid sequence encoding a desired gene. As used herein such a desired gene is any gene, and may be appropriately selected depending on the application of the nucleic acid molecule to be used.

In a preferable embodiment, it may be advantageous but is not limited to, that the methylation in the nucleic acid molecule used in the present invention is present at least C in a CG sequence. Although not wishing to be bound by any theory, specific presence of methyl group at a CG sequence causes a phenomenon of heterochromatinization of the chromosome, resulting in rendering greater effects on the transposition efficiency of a transposon.

Transposons used in the present invention may be in any form, and preferably, of DNA-type. If DNA-type is used, effects of methylation are amenable for achievement. Preferably, transposons used in the present invention belongs to Tc1/mariner type. Although not wishing to be bound by any theory, it is believed that transposons belonging to this type may heterochromatinize the chromosome when it is integrated into the chromosome, resulting in rendering greater effects on the transposition efficiency of a transposon.

In the most preferable embodiment, the transposon used in the present invention comprises Sleeping Beauty. This particular transposon allows, as described elsewhere herein, utilization across the wall of species.

Preferably, in the nucleic acid molecule of the present invention, a desired gene may be operably linked to a transposon, or constructed in a manner such that it is operably linked to a transposon when introduced into a cell. In order that a transposon is operably linked, for example, appropriate location of an inverted repeat sequence and the like is performed, but it is not limited to this.

In a preferable embodiment, the nucleic acid molecule of the present invention is used for introducing a foreign gene into a host. The introduction of a foreign gene into a host is an unexpected effect even if the nucleic acid molecule of the present invention has observed an effect *in vitro.* In addition, in the circumstances where methylation mechanisms are unclear, there is no key for expectation, and thus the efficient introduction of a foreign gene by methylation of the present invention is a significant effect.

As used herein, the biological organism (host) targeted by the present invention is any biological organism as long as a transposon operates, and includes eukaryotes but is not limited thereto. Preferably, the host targeted by the present invention includes, but is not limited to mammals (for example, rodents such as mouse, rat; primates and the like) . Whether or not the functions of a transposon can be confirmed by conducting an animal test.

In a preferable embodiment, with respect to the nucleic acid molecule of the present invention, it is advantageous that a transposase acts on the location on the genome for which the nucleic acid molecule is inserted. Such a constitution allows smooth transposition of a gene.

In another aspect, the present invention is directed to a gene cassette having a nucleic acid sequence encoding a transposon, wherein said nucleic acid sequence has a methylation at at least one nucleotide. The nucleic acid molecules to be included may have the features as described herein above. As used herein the term "gene cassette" may attach another element.

In another aspect, the present invention provides a vector having a nucleic acid sequence encoding a transposon and a nucleic acid sequence encoding a desired gene, wherein said nucleic acid sequence has a methylation at at least one nucleotide. The sequence encoding a transposon and methylation are described herein elsewhere. A vector has usually circular shape but is not limited thereto. A vector may include elements for regulating transcription, translation or expression. Such an element is preferably operably linked.

Preferably, the vector uses introduction of a foreign gene into a host. The vector of the present invention achieved an effect which allows production of a transgenic biological organism of a species for which it was impossible or difficult to produce. In particular, in mammals which had been believed that no transgenic biological organisms to date, SB allows production of a transgenic biological organism using a form of vector, which means that a system in which the foreign gene can be readily inserted is readily available. Accordingly, such a system has great utility.

The vector of the present invention can be constituted such that a transposase acts on the location of the genome for which a nucleic acid sequence is inserted.

As used herein, the term "signature site" refers to a site which emerges as a result of excision and transposition of a transposon. For example, when an SB transposon is used in the present invention, a signature site comprises a sequence "TAcagTA" or "TActgTA" where three bases of the terminus sequence of the transposon is inserted into a TA repeat of a target sequence. Note that even when a transposon is moved, a resultant signature site may not have the above-described perfect specific sequence. In the present invention, a site having such an imperfect sequence is regarded as a signature site.

In another embodiment, in the present invention, a vector is constructed such that a transposase can act on the site on the genome to which the nucleic acid of the present invention is inserted. Such construct may be achieved by methylation of at least a portion of the sequence encoding a transposon in the present invention.

In another embodiment, the present invention provides a composition for acting a transposase onto a foreign nucleic acid molecule to be inserted into the genome. The composition is characterized in that nucleic acid sequence encoding a transposon and the foreign nucleic acid molecule are included, and that the sequence encoding the transposon is methylated. As used herein, the foreign nucleic acid molecule may or may not be methylated

In another aspect, the present invention provides a cell comprising a nucleic acid molecule having a nucleic acid sequence encoding a transposon and a nucleic acid sequence encoding a desired gene, wherein said nucleic acid sequence has a methylation at at least one nucleotide. Such a cell allows ready production of a transgenic biological organism in the present invention. Accordingly, the present invention has significant utility. The nucleic acid molecule which can be encompassed by the cell of the present invention may have any sequence as long as the sequence comprises those encoding a transposon as described herein above. Preferably, such a sequence functions in the cell to be retained. Preferably, such a cell is used for introducing a foreign gene into a host. Such a host may preferably but no necessarily be of the same species as the cell.

The cell of the present invention may of any type, and preferably include but is not limited to eukaryotic cells, and more preferably includes but is not limited to mammalian cell, and still more preferably includes but is not limited to rodent cells. In a more preferable embodiment, it is useful to use a model animal such as a mouse, rat and the like. The cell of the present invention should be determined in relation to the property of the nucleic acid molecule to be introduced, object, and the host into which the nucleic acid molecule is introduced. The nucleic acid molecule to be included in the cell of the present invention may be the vector of the present invention.

In another aspect, the present invention provides a tissue comprising a nucleic acid sequence encoding a transposon and a nucleic acid sequence encoding a desired gene, wherein said nucleic acid sequence has a methylation at at least one nucleotide. Such a tissue allows ready production of a transgenic biological organism in the present invention. Accordingly, the present invention has significant utility. The nucleic acid molecule which can be encompassed by the tissue of the present invention may have any sequence as long as the sequence comprises those encoding a transposon as described herein above. Preferably, such a sequence functions in the tissue to be retained. Preferably, such a tissue is used for introducing a foreign gene into a host. Such a host may preferably but no necessarily be of the same species as the tissue.

The tissue of the present invention may of any type, and preferably includes, but is not limited to eukaryotic tissues, and more preferably includes, but is not limited to mammalian tissue, and still more preferably includes, but is not limited to rodent tissues. In a more preferable embodiment, it is useful to use a model animal such as a mouse, rat and the like. The tissue of the present invention should be determined in relation to the property of the nucleic acid molecule to be introduced, object, and the host into which the nucleic acid molecule is introduced. The nucleic acid molecule to be included in the tissue of the present invention may be the vector of the present invention.

In another aspect, the present invention provides a biological organism comprising a nucleic acid sequence encoding a transposon and a nucleic acid sequence encoding a desired gene, wherein said nucleic acid sequence has a methylation at at least one nucleotide. Such a biological organism allows ready production of a transgenic biological organism in the present invention. Accordingly, the present invention has significant utility. The nucleic acid molecule which can be encompassed by the biological organism of the present invention may have any sequence as long as the sequence comprises those encoding a transposon as described herein above. Preferably, such a sequence functions in the biological organism to be retained. Preferably, such a biological organism is used for introducing a foreign gene into a host. Such a host may preferably but not necessarily be of the same species as the biological organism.

The biological organism of the present invention may of any type, and preferably includes, but is not limited to eukaryotic biological organisms, and more preferably includes but is not limited to mammalian biological organism, and still more preferably includes but is not limited to rodent biological organisms. In a more preferable embodiment, it is useful to use a model animal such as a mouse, rat and the like. The biological organism of the present invention should be determined in relation to the property of the nucleic acid molecule to be introduced, object, and the host into which the nucleic acid molecule is introduced. The nucleic acid molecule to be included in the biological organism of the present invention may be the vector of the present invention.

In a preferable embodiment, the biological organism of the present invention is preferably not derived from the biological organism from which the desired gene is derived. In such a case, the desired gene is called a foreign gene. Those to be introduced as a foreign gene may be of any type and may vary depending on the gene of interest.

In another aspect, the present invention is related to a method for producing a transgenic biological organism, comprising the steps of: A) providing an isolated nucleic acid molecule having a nucleic acid sequence encoding a transposon; B) transforming a germ cell of a desired biological organism with said nucleic acid molecule; C) selecting an individual in which the germ cell has a methylation in a nucleic acid sequence encoding said transposon; and D) regenerating a biological organism using the transformed germ cell.

Provision of an isolated nucleic acid molecule having nucleic acid sequence encoding a transposon is known in the art, and may be achieved by means of well known technology. Transformation of a germ line cell of the desired biological organism with the nucleic acid molecule is also well known in the art and may be achieved by any methods including those gene recombination technology described herein. Individuals having the nucleic acid sequence encoding a transposon methylated in a germ line cell can also be selected by means of any well known technology in the art. Specifically, nucleic acid molecule (for example, DNA) is removed from the germ line cell so as not to disrupt methylation, and the nucleic acid molecule is subjected to demethylation and confirm whether the mass is changed or not and optionally the sequence of the methylated portion may be determined. Regeneration of the biological organism using the transformed germ line cell may also be conducted and can be achieved depending on the organism used for an appropriate method.

In a preferable embodiment, the biological organism targeted by the transgenic biological organism of the present invention is eukaryotic. This is because the effects of methylation of transposon in the present invention is more amenable.

In another preferable embodiment, the biological organism to be targeted by the transgenic biological organism of the present invention includes mammals. This is because the effects of methylation of transposon in the present invention is more amenable. More preferably, the mammals may be rodent, and still more preferably, a model animal such as mouse, rat and the like.

In another aspect, the present invention provides a method for producing a transgenic biological organism comprising the steps of: A) providing an isolated nucleic acid molecule having a nucleic acid sequence encoding a transposon, wherein the nucleic acid sequence has a methylation at at least one nucleotide; B) transforming a germ cell of a desired biological organism with said nucleic acid molecule; and C) regenerating a biological organism using the transformed germ cell.

Provision of an isolated nucleic acid molecule having nucleic acid sequence encoding a transposon which is methylated at at least one nucleotide is described elsewhere herein and sufficiently described and may be achieved by means of well-known technology.

In the method of the present invention, transformation of a germ line of the desired biological organism with a nucleic acid molecule is also achieved by means of well-known technology in the art.

In the method of the present invention, regeneration of the biological organism using the transformed germ line cell may also be readily achieved by those skilled in the art by selection of an appropriate method depending on the organism.

In another preferable embodiment, the biological organism to be targeted by the transgenic biological organisms of the present invention includes mammals. This is because the effects of methylation of transposon in the present invention is more amenable. More preferably, the mammals may be rodent, and still more preferably, a model animal such as mouse, rat and the like. The present invention allows production of transgenic animal of a model animal in a simple and automatic method having high probability. As such, the present invention has unexpected significant effects which has not been achieved by the conventional technologies.

In another aspect, the present invention provides a kit for producing a transgenic biological organism, comprising A) an isolated nucleic acid molecule having a nucleic acid sequence encoding a transposon, wherein the nucleic acid sequence has a methylation at at least one nucleotide; and B) a transposase.

The nucleic acid molecule to be included in the kit of the present invention are methylated at the portion encoding the transposon as described above, and may be a naturally-occurring one or may be produced in an artificial manner by means of methylation enzyme. Further, any transposase may be used as long as it has an activity to the transposon to be included in the kit.

In an embodiment, the kit of the present invention comprises an indication describing methods of using the nucleic acid molecule and transposase. The indication may be of paper medium, but may also be of transmitting medium such as information on a network. The indication describes handling of nucleic acid molecules, transformation methods, culture methods, regeneration methods, transposon incubation methods and the like, and a variety of protocols relating to transgenic biological organisms. The description may be of monolingual, but two or more languages may also be described.

In another aspect, the present invention is related to use of an isolated nucleic acid molecule having a nucleic acid sequence encoding a transposon, wherein the nucleic acid sequence has a methylation at at least one nucleotide for producing a transgenic biological organism. Such use of a methylated nucleic acid molecule for transgenic biological organism was not known in the art, and not obvious therefrom. Description relating to such a nucleic acid molecule is as described herein above, and a variety of modification is possible.

In another aspect, the present invention provides a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated. Inverted repeat sequences may be of any type, and for example, those set forth in SEQ ID NOs: 22-26. The nucleic acid sequence maybe of any type and include for example, a portion or the entire part of a foreign gene. Preferably, the nucleic acid sequence comprises at least one expression regulatory region. Such an expression regulatory region includes, but is not limited to for example, promoters, enhancers, or silencers and the like.

In an embodiment, the nucleic acid fragment of the present invention further includes at least a portion of a foreign gene, wherein the nucleic acid sequence included in the nucleic acid fragment is operably linked to a sequence encoding at least a portion of the foreign gene.

In an embodiment, the cell may be of any type, and preferably of an animal. In a preferable embodiment, cells used in the present invention is of vertebrate (for example, mammals such as primate or rodent (for example, rat, mouse and the like)).

DNA of the cell used herein may be DNA present in any cell such as cellular genome, episome and plasmid and the like.

At least one inverted repeat sequence used in the present invention may comprise sequence set forth in SEQ ID NO: 20 or 21 or a portion thereof.

In one embodiment, the transposase used in the present invention is SB protein. Transposases used in the present invention may have at least 80 %, preferably at least 90 %, more preferably at least 95 % amino acid homology to SEQ ID NO: 3. Alternatively, the nucleic acid molecule encoding the transposase used in the present invention is a sequence hybridizable to SEQ ID NO: 2 under stringent conditions, or hybridizable to the nucleic acid sequence encoding the amino acid sequence set forth in SEQ ID NO: 3 under stringent conditions. Alternatively, the transposase used in the present invention may have one or more amino acid substitutions, additions or deletions against the amino acid sequence set forth in SEQ ID NO: 3.

In a preferable embodiment, the at least one inverted repeat sequence included in the nucleic acid fragment of the present invention includes at least one direct repeat sequence, and the direct repeat sequence includes the nucleotide sequence set forth in SEQ ID NO: 26 or at least 80 % homologous nucleotide sequence thereto.

In another preferable embodiment, the at least one inverted repeat sequence included in the present invention comprises at least one direct repeat sequence, and preferably the direct repeat sequence may consist of the nucleic acid sequence set forth in or selected from the group consisting of SEQ ID NO: 22-25.

In another aspect, the present invention provides a nucleic acid introduction system for introducing into a DNA of a cell another DNA, the system comprising A) a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; and B) a transposase or a nucleic acid encoding a transposase.

In a preferable embodiment, the transposase used in the system of the present invention may be SB nucleic acid molecule or SB protein, but is not limited thereto, and it is understood that any transposase used in the nucleic acid fragment of the present invention may also be used in the system. Alternatively, the transposase used in the present invention may have the amino acid sequence set forth in SEQ ID NO: 3 or a variant thereof, or the nucleic acid sequence encoding the transposase may be the nucleic acid sequence set forth in SEQ ID NO: 2 or a variant thereof.

As used herein the nucleic acid encoding the transposase used in the present invention may preferably incorporated into the cellular genome.

In an embodiment, the system of the present invention further comprises a plasmid or viral vector, and the plasmid or the viral vector includes the nucleic acid fragment as a part thereof.

The nucleic acid fragment included in the system of the present invention may include at least a portion of the sequence encoding a foreign gene.

In a preferable embodiment, the nucleic acid fragment included in the system of the present invention is introduced into the cell by means of a method selected from the group consisting of particle bombardment; electroporation; microinjection; use of gene introduction reagents; and use of virus vectors. It should be understood that particle bombardment; electroporation; microinjection; use of gene introduction reagents; and use of virus vectors and the like are known in the art and any known technology may be used.

In another aspect, the present invention provides a method for producing a transgenic biological organism, comprising the steps of:

> introducing a nucleic acid fragment and transposase into a pluripotent cell, wherein the nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; and growing the cell into a living body. As used herein the introduction of the nucleic acid fragment and the transposase into a cell, may be achieved by means of any well-known technology of introducing a nucleic acid or a peptide. It should be understood that a method for growing a cell into a biological organism may also be achieved by means of an appropriate method such as the use of differentiation agents. Alternatively, it is understood that a pluripotent cell such as those selected from the group consisting of oocyte, embryonic cell, egg and stem cell may be used for obtaining a biological organism by growing the same under a normal condition.

Preferably, the biological organism used may be a rodent such as mouse or rat and a primate.

In another aspect, the present invention provides a method for introducing a nucleic acid into a DNA of a cell, comprising the step of: introducing a nucleic acid fragment into a cell, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated.

In an embodiment, the present invention may further comprise the step of introducing a transposase into the cell. The transposase used herein may have at least 80 % homology to the sequence set forth in SEQ ID NO: 3 or any other variant. Such an example of variants are described and exemplified herein elsewhere.

In an embodiment, the cell used in the present invention comprises a nucleic acid encoding transposase. The inclusion of such a nucleic acid avoids necessity of introduction of a foreign transposase. Preferably, the nucleic acid is incorporated in to the cellular genome. This is because it enables permanent expression thereof. Accordingly, such a transposase may preferably be stably expressed in the cell. Alternatively, the transposase may be operably linked to a inductive promoter in a controllable manner, and temporarily expressed. It should be understood that stable or temporal expression of the nucleic acid may be appropriately selected for an appropriate use depending on the experiments of interest.

In the present invention, the sequence to be inserted preferably encodes a protein. Such a protein includes but is not limited to, for example, a marker protein, fluorescence protein, a physiological protein and the like.

In another aspect, the present invention provides a method for transposing a nucleic acid sequence in a cell, comprising the step of: introducing a transposase into a cell comprising a nucleic acid fragment, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; wherein the transposase transposes the nucleic acid sequence from a first location in a DNA of the cell to a second location of the DNA. The transposition of the gene in the cell is a function which is advantageously achieved by the transposon system, and the methylation significantly enhances the mobility efficiency of the transposon system.

In the transposition method of nucleic acid sequence of the present invention, the DNA of the cell is preferably a genomic DNA, and the first and second locations may be at epichromosomal DNA, but the present invention is not limited thereto. Epichromosomal DNA involvement allows simple on-off switching of the gene. Transposase as used herein may preferably be introduced as a nucleic acid into the cell.

In another aspect, the present invention provides a method for identifying a gene in a cell, comprising the steps of: introducing into a cell a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated. and a transposase; digesting the DNA in the cell with a restriction endonuclease capable of digesting the nucleic acid sequence; identifying the inverted repeat sequence; determining a sequence of a nucleic acid having similar sequence to the inverted repeat sequence; and comparing the sequence with sequence information in a sequence information database. It is understood that the introduction of the nucleic acid fragment and the transposase into a cell may be achieved by any well-known technology in the art. Endonucleases used herein may be of any type known in the art, and it is understood that the cleavage and digestion may be achieved by using, for example, a manual according to the manufacturer. It is understood that identification of an inverted repeat sequence may be achieved by means of identification of specific base length or restriction pattern, sequencing and the like. Once sequenced, it is possible to compare the determined sequence with a known database or a database which is produced by the practitioner using a method known in the art. As such, the function of a gene may be identified by such a comparison. It is understood that such identification may be achieved by, for example, a variety of biological assay or observation of phenotype thereof and the like.

References such as scientific literature, patents, patent applications and the like will be incorporated herein by reference as if the entirety thereof is specifically described herein.

Hereinafter, the present invention is described based on the Examples. The following Examples are provided only for the purpose of illustration. Accordingly, the scope of the claims of the present invention is not limited to the description described above or following examples, but only by the appended claims.

## EXAMPLES

Hereinafter the present invention is described by way of examples in detail, but the present invention is not limited to the following examples. Reagents used herein below in the Examples, are obtained from Sigma (St. Louis, USA), Wako Pure Chemical (Osaka, Japan) and the like unless otherwise stated. Handling of animals have been conducted under the provisions defined in Osaka University, Medical School. The method for producing an expression vector used in the present invention will be described in with specific examples. It will be readily conducted for those skilled in the art to replace elements, such as these start plasmid, promoters and the like with equivalents thereof.

The scope of the claims of the present invention is not limited to the description described above or following examples, but only by the appended claims.

(Materials and Methods)

(Construction of plasmids and methylation)

A targeting vector to introduce the SB transposon into the serine palmitoyltransferase long-chain base subunit 2 (Sptlc2) locus was generated by first cloning a 5-kb XhoI-KpnI fragment of the Sptlc2 gene containing exon 5 into the XhoI-KpnI site of pBluescript II (pBS;Stratagene), resulting in pBS-Sptlc2. A SalI-BamHI fragment of pCX-EGFPPigA (Horie, K., A.Kuroiwa, M.Ikawa, M.Okabe, G.Kondoh, Y.Matsuda, and J.Takeda. 2001; Proc. Natl. Acad. Sci. USA 98: 9191-9196) and a BamHI-NotI fragment of a 150-bp synthetic splice donor site (a gift from M. Ikawa) were then cloned into the SalI-NotI site of pBS, followed by the deletion of the BamHI site, to generate pCX-EGFP-SD. A blunted SalI-NotI fragment of pCX-EGFP-SD was inserted into the blunted EcoRI-BamHI site of pBS-IR/DR(R,L) to generate pTransCX-EGFP-SD. An EcoRI-NotI fragment of pTransCX-EGFP:Neo (Horie, K., A. Kuroiwa, M.Ikawa, M. Okabe, G. Kondoh, Y. Matsuda, and J. Takeda. 2001; Proc. Natl. Acad. Sci. USA 98:9191-9196) containing a PGK-neo cassette was cloned into pBS, after which the BamHI site was deleted to generate pBS-Neo. A NotI-KpnI fragment of pBS-Neo and a KpnI-XhoI fragment of pTransCX-EGFP-SD were inserted into the NotI-XhoI site of pBS-Sptlc2, resulting in the targeting vector.

A vector for recombinase-mediated cassette exchange (RMCE) was constructed in the following manner. To remove the loxP site downstream of the PGK-neo cassette of pTransCX-EGFP:Neo, a BamHI-AflIII fragment and a NotI-AflIII fragment were religated, resulting in pTransCX-EGFP:Neo-3'lox. After blunt ending and NotI linker ligation of all cleavage ends, a ClaI-BglII fragment of pL1CMVEGFP1L was replaced with a PvuI fragment of pTransCX-EGFP: Neo-3' lox containing the SB transposon and a about 200-bp flanking sequence on both sides, resulting in pL1TransCX-EGFP1L.

TransSAβ-geo and pTc3/SAβ-geo were constructed by cloning a blunt-ended XhoI fragment of pROSAβ-geo (Friedrich, G. and P. Soriano. 1991;Genes Dev. 5: 1513-1523) into the blunt-ended EcoRI-BamHI site of pTransCX-GFP (Horie, K., A. Kuroiwa, M. Ikawa, M. Okabe, G. Kondoh, Y. Matsuda, and J. Takeda.2001; Proc.Natl.Acad.Sci.USA 98: 9191-9196) and the blunt-ended BspEI-NcoI site of pRP790 (Fischer, S.E. , H.G. van Luenen, and R.H. Plasterk. 1999.; Mol. Gen. Genet.262:268-274.), respectively.

For the expression of a six-His-tagged 123-amino-acid N-terminal fragment of the SB transposase (N123), the N-terminal fragment was first PCR amplified from pCMV-SB (pSB10) (19) with primers 5'-CATGCCATGGGAAAATCAAAAGAAATC-3' (SEQ ID NO: 29) and 5'-CCGCTCGAGCAGTGGCTTCTTCCTTG-3' (SEQ ID NO: 30) and digested with NcoI, BsrGI , and XhoI. Then, the NcoI -BsrGI fragment and the BsrGI-XhoI fragment were cloned into NcoI- and XhoI-digested pET21d (Novagen), resulting in pET-N123.

For the DNA probe of IR/DR-L for the electrophoretic mobility shift assay (EMSA), the HindIII-KpnI fragment of pTransCX-EGFP:Neo was cloned into the HindIII-KpnI site of pBS, resulting in pBS-IR/DR-L.

Methylation of plasmid DNAs was performed with SssI CpG methylase (NEB) according to the manufacturer's protocol, followed by purification with a PCR purification kit (Qiagen) . Complete methylation was confirmed by resistance to digestion with methylation-sensitive enzymes.

(Cell culture and gene targeting)

Mouse ES cells were cultured in Dulbecco modified Eagle medium containing 20% fetal bovine serum, nonessential amino acids, sodium pyruvate, and 1,000 U of leukemia-inhibitory factor/ml on mitomycin C-treated mouse embryonic fibroblasts. Mouse erythroleukemia (MEL) cell clones RL5 and RL6 (8) were cultured in Dulbecco modified Eagle medium containing 10% fetal bovine serum.

Targeted integration of the SB transposon at the Sptlc2 locus was performed by means of insertional-type homologous recombination. Briefly, 25 $\mu$g of the targeting vector was linearized at the BamHI site located in the homologous region and introduced into $1.0 \times 10^7$ ES cells by electroporation (240 V, 500 $\mu$F) with a Gene Pulser II (Bio-Rad) . Cells were selected for 7 days with 150 $\mu$g of G418/ml, after which resistant clones were picked up, expanded, and analyzed with Southern blotting.

RMCE was performed as described previously (32). Briefly, 25 $\mu$g of methylated or unmethylated plasmids was introduced by electroporation (250 V, 1,070 $\mu$F) into MEL cell clone RL5 or RL6 together with 20 $\mu$g of Cre expression vector pBS185 and 200 $\mu$g of salmon sperm DNA. Cells were selected for 10 days, starting 4 days after electroporation, with 10 $\mu$M gancyclovir. Limiting dilution was performed for the isolation of independent clones, which were expanded and prescreened by PCR with the primers EGFP-1U (5'-CACCCTC GTGACCACCCTGACCTAC-3') (SEQ ID NO: 31) and EGFP-1L (5'-CTTGATGCCGTTCTTCTGCTTGTCG-3') (SEQ ID NO: 32) for the detection of enhanced green fluorescent protein (EGFP) and with the primers HYG-1U (5'-CGGGCGTATATGCTCCCCATTGGTCTTGAC-3') (SEQ ID NO: 33) and TK-1L (5'-TGGTGTAGATGTTCGCGATTGTCTCGGAAG-3') (SEQ ID NO: 34) for the detection of HYTK.

The orientation of the exchanged cassette was determined with the primers M13F (5'-ACGACGTTGTAAAACGACGGCCAGT-3') (SEQ ID NO: 35) and RMCE-DL1 (5'-GCATCGCCATGGGTCACGACGAGATCCTC-3') (SEQ ID NO: 36) for orientation D and with the primers M13F and RMCE-IL-1 (5'-AAGTGAGTTTAAATGTATTTGGCTAAGGTG-3') (SEQ ID NO: 37) for orientation I. Cassette exchange and methylation statuses were further confirmed by Southern blot analysis.

(Isolation of male germ cells)

Testes of a mouse carrying donor transposons (Horie, K., A. Kuroiwa, M. Ikawa, M. Okabe, G. Kondoh, Y. Matsuda, and J. Takeda. 2001; Proc. Natl. Acad. Sci. USA 98: 9191-9196) were decapsulated and chopped into about 1-mm2 fragments with a razor blade. Germ cells were released by repeated pipetting. After brief centrifugation to remove seminiferous tubules, the supernatant was collected and sedimented by further centrifugation at 1,500 x g. Then, DNA was extracted from the cell pellet and analyzed with Southern blotting.

(Sourthern blotting analysis)

Genomic DNA was digested with restriction enzymes, fractioned by 0.7 % agarose gel, and transferred to HyBond·N+ nylon membrane (Amersham). 0.7 kb EcoRI fragment of EGEP containing EGFP (Okabe M.et al., FEBS Lett. 407, 313-9 (1997)) was used as a probe for detecting a transposon-specific band. Hybridization and washing were conduced according to a standard manual (J. Sambrook et al. *supra)* (1989)). In order to evaluate the transposon copy number of the transgenic mice, tail DNA bands were compared to the genomic DNA derived from the ES cell clone comprising the sole copy of the transposon.

(PCR analysis)

For excision of the transposon, the following primer set was used for detection: TgTP-2L, 5'-ACACAGGAAACAGCTATGACCATGATTACG-3' (SEQ ID NO: 7) and TgTP-1U, 5'-GACCGCTTCCTCGTGCTTTACGGTATC-3' (SEQ ID NO: 6). Each primer used is present outside pTransCX-GFP:Neo IR/DR(R) and IR/DR(L). PCR was conducted using HotStarTaq system (Qiagen) using the following conditions:

95 degrees Celsius, 15 minutes, 50 cycles of: 94 degrees Celsius, one minute, 59 degrees Celsius, one minute, 72 degrees Celsius, one minute, followed by the final step: 72 degrees Celsius, one minute (one cycle).

Genotyping of the transgenic mice was conducted using the following primer set:

With respect to the GFP gene: EGFP-1U,5 ' -CACGCTGGTGACCAGCCTGACCTA3' (SEQ ID NO: 73) and EGFP-1L,5' -CTTGATGCCGTTCTCTGCTTGTCG-3' (SEQ ID NO: 74); and for the SBtransgene: SB-2U, 5'-TCCTAGAGATGAACGTACTTTGGT-3' (SEQ ID NO: 75) and SB-1L, 5' -ATCCAGATAATTTTCCTTGCTCATG-3' (SEQ ID NO: 76).

PCR conditions were the same as above, except for the annealing temperature of 55 degrees Celsius and the number of cycles to be 30. Resultant PCR product were 313 bp for the GFP gene, and 466 bp for the SB transgene. Flanking sequence in the novel inserted or integrated sites in the transposon were PCR amplified as described previously (Ivicz, Z.et al., Cell 91, 501-10 (1997) ) . PCR product was directly sequence using dye-terminator and ABI373A DNA sequencer (Applied Biosystems).

(Determination of GFP expression)

The tip of the tail of mice just after the birth was resected for observation using GFP· specified Filter (Olympus, Tokyo, Japan) and Olympus fluorescence inverted microscope. Mice having GFP expression in the entire tail was determined to be positive.

(Example 1: Transient transposon excision assay)

First, the present Example confirmed whether methylation of a sequence encoding a transposon enhances excision capability.

(Transient transposon excision assay)

Transposon DNA (pTransCX-EGFP: neo, Horie et al., PNAS 2001, Proc. Natl. Acad. Sci. USA.;98:9191-6) has been methylated in advance with SssI CpG methylase (New England Biolabs;50mM NaCl,10mMTris-HCl,pH7.9, 10mM

$MgCl_2$, 1mM DTT, 160$\mu$M SAM, 0.2U/$\mu$l SssI) . Methylation has been verified by observing non digestion of the sample by methylation sensitive NotI.

Next, Murine erythroleukemia cell MEL cell; J. Mol. Biol. 292: 779-785, 1999) was introduced with transposon DNA and Sleeping Beauty (SB) transposase (PCMV-SB, obtained from Dr. P.Hackett). Total DNA was extracted from the cell usingDNeasy Tissue Ki (QIAGEN), and PCR was conducted with the plasmid vector using primers (TgTP-1U, TgTP-2L(previously shown)) and 358 bp PCR product is detected, which is amplified when the excision reaction is caused. Detection was conducted by staining the agarose gel with ethidium bromide after the electrophoresis of the agarose gel. As a reference, size markers have also been electrophoresed. An example of the excision system is shown in Figure 1D(a).

(Excision assay of transposon temporarily transfected)

For excision assay of transposon temporarily transfected, $1.0\times10^6$ cells of MEL cells were transfected using TransFast (Promega) with 1.0 $\mu$g of methylated or unmethylated pTransCX-EGFP:Neo together with 1.0 $\mu$g of pSB10 or pBS. For excision assay of a transposon which has transiently transfected, $1.0\times10^6$ cells of MEL cell have been tranfected with methylated or unmethylated pTransCX-EGFP:Neo (1.0 $\mu$g) together with $\mu$g of pSB10 or pBS.

Fourty-eight hours after the transfection, DNeasy kit (QIAGEN) was used to extract genomic DNA. Four hundred ng and 80 ng of HindIII digested DNA were used to the amplified excised products and neo fragments using HotStarTaq systemn (QIAGEN) . neo fragment is present in pTransCX-EGFP:Neo vector, and was used as an internal standard for transfection efficiency. Primer TgTP-1U (5'-GACCGCTTCCTCGTGCTTTACGGTATC-3' (SEQ ID NO: 6)) and primer TgTP-2L (5'-ACACAGGAAACAGCT ATGACCATGATTACG-3' (SEQ ID NO: 38) ) was used to detect the excised products, and primer neo-U1(5'-GGGTGGAGAGGCTATTCGGCTATGA-3' (SEQ ID NO: 39)) and primer neo-L1(5'-TGGATACTTTCTCGGCAGGAGCAAG-3' (SEQ ID NO: 40)) were used to amplify neo fragments. The amplification of the resected products were conducted as follows: 95 degrees Celsius for fifteen minutes, followed by 35 cycles of 94 degrees Celsius for one minute, 63 degrees Celsius for one minute, and 72 degrees Celsius for one minute, and finally the final extension at 72 degrees Celsius for seven minutes. The same conditions were used for amplification of neo fragment except for that annealing temperature is 60 degrees Celsius and the number of cycles was 19.

The excised product and neo fragment, according to manufacturer's manual, LightCycler FastStart DNA Master hybridization probe kit (Roche Diagnostics) was used to conduct quantification from 40ng of DNA, LightCycler apparatus (Roche Diagnostics) for real-time PCR. Fluorescent labeled probes for the resected products are as follows: 5'-CGGCCGCTCTAGCGGTACCCTAC-FITC-3' (SEQ ID NO: 41) and 5'-LCRed640-GTAGGGGATCGACCTCGAGGGG-3' (SEQ ID NO: 42), and fluorescence labeled probes for fragments are 5'-GCTGTGCTCGACGTTGTCACTGAAG-FITC-3' (SEQ ID NO: 43) and '-LCRed640-GGGAAGGGACTGGCTGCTATTGGG-3' (SEQ ID NO: 44). PCR primers for the excised products are as follows: 5'-GTTGGGTCGTTTGTTCGGAT-3' (SEQ ID NO: 45) and 5'-CGCGCAATTAACCCTCACTA-3' (SEQ ID NO: 46). PCR primers for neo fragment are as follows: 5'-AATGAACTGCAGGACGAGGC-3' (SEQ ID NO: 47) and 5'-ATGGATACTTTCTCGGCAGG-3'(SEQ ID NO: 48). Four atto gram to 2.0pg, and 0.2pg to 2.0ng of control plasmid having a target sequence were used as a standard for the excised product and neo fragment, respectively. The amplification conditions were as follows: 95 degrees Celsius for ten minutes, followed by forty-five cycles of 95 degrees Celsius for ten seconds, 55 degrees Celsius for 10 seconds and 72 degrees Celsius for ten seconds. The excised product and neo fragments were quantified based on the standard curve, and the amount of excised product was divided by that of neo fragment for normalization.

(RESULTS)

Figures 1A to 1C show the results relating to that frequent Transposition is related to CpG methylation.

(A) The introduction of a single copy of SB transposon into Sptlc2 locus by insertion-type homologous recombination and the detection of transposon excision by PCR. White square: exon, solid triangle: loxP site, gray and white arrows: nestid PCR primers, less dense line: backbone sequence of the plasmid, CAP: CAP promoter, IR/DR-R and IR/DR-L: right IR/DR and left IR/DR, respectively, Xb: XbaI, B: BamHI, K: KpnI.

(B) Excision of SB transposon in Sptlc2 locus of an ES cell. ES 115 clone (shown in panel A) having SB transposon inserted therein by targeting Sptlc2 locus, was transfected with sSB10 expressing the wild type of SB transposase (transposase +) or pSB10-DDE expressing inactive SB transposase with deletion of DDE box (transposase -) (Ivics, Z., P. B. Hackett, R. H. Plasterk, and Z. Izsvak. 1997; Cell 91:501-510) in a serial dilution. As shown in Panel A, the resultant was screened with nested PCR. Four independent transfections for each of dilution factors of pSB10 were conducted, and eight independent PCR was with 1 $\mu$g of genomic DNA per reaction to conduct screening of 8$\mu$g of genomic DNA per transfection. Typical PCR results with maximum amount of pSB10 (2$\mu$g) are shown in the left panel. The right panel shows the results of the average number of positive PCR for each transfection and the RT-PCR analysis at the expression level. Actb: beta-actin.

(C) Southern blot analysis of methylation state in the transposon sequences of male mouse germ line cell and ES cell. EGFP was used as a probe. Genomic DNA derived from the germ line cell has not been digested by HpaII. This shows that the site has mostly been methylated. On the other hand, the genomic DNA from ES115 clone has been substantially completely digested. The presence of minor band (shown as asterisk) shows that a small fraction of HpaII site has been methylated in an ES cell. Solid circle: HpaII(H) or MspI (M) site; X: XhoI site; 0.5 kb bands derived from HpaII-MspI fragment in KM, KXM and KXH lanes are shown with arrow.

The results of the present example is shown in Figure 1D(b) . As is seen in the photograph, a cell in which methylated transposon has been introduced with a transposition enzyme, showed more often excision reactions than a cell with unmethylated transposons. Abbreviation: CAG: CAG promoter, EGFP: green fluorescent protein, pA: poly addition signal, L: left IR/DR, R: right IR/DR, M: methylation, N: non-methylation, NC: negative control (MEL cell, genomic DNA).

Accordingly, it was clarified that methylation allows significant effects in the transposon.

(EXAMPLE 2: establishment of a cell having methylated or unmethylated transposon in the same genetic locus of a murine genome)

Next, the present Example verifies whether methylation at the cellular level has an effect on transposition activity.

(Methods)

Two loxP sequences in reversed direction were constructed in a plasmid vector with a transposon located therebetween, and SssI CpG methylase was subj ected thereto for methylation of the sequence encoding a transposon. Next, the plasmid DNA and the Cre recombinant enzyme expression vector were introduced to a MEL cell (strains RL5, RL6, E.E. Bouhassira, J. Mol. Biol. 292:779-785,1999) with hygromycin resistant gene and herpes viral thymidine kinase (HSVTK) as foreign genes (transgene) between the loxP sequence in reversed direction. The Cre recombinant enzyme allows recombination between the plasmid and the loxP on the genome, resulting in the establishment of a cell having a transposon in a specific site on the genome. This allows efficient excision reaction due methylation in the same sites on the genome. SB transposase was introduced in to an established cell for detecting the excision reaction by PCR as described in Example 1 . Figure 2A shows the experimental flow chart. In Figure 2A, solid arrow shows the loxP site, and white and gray arrows show the PCR primers. CMV indicates CMV promoter.

(Excision assay of stably integrated transposon)

Excision assay of stably integrated transposon. For the excision assay of the stably integrated transposon, 2.0 0 X $10^5$ targeted ES cell clones or 1.0 X $10^6$ MEL cell clones were transfected with 2.0, 0.67, 0.22, 0.074, 0.025, and 0.0082 $\mu$g of pSB10, and genomic DNAs were extracted with a DNeasy kit 48 h post-transfection.

One microgram of genomic DNA was analyzed by PCR with the primers LCB2XL2 (5'-TTCCAAAAGAAGTAGAGTGGAGAACCAGTG-3') (SEQ ID NO: 49) and PGK2 (5'-AGGCCACTTGTGTAGCGCCAAGT-3') (SEQ ID NO: 50) for the targeted ES cell clone and with the primers TgTP-1U and TgTP-2L for MEL cell clones. Nested PCR with 1 $\mu$l of the first PCR product as a template was performed with the primers LCB2XL1 (5'-CCAACCAAATACATTTAACATATTCTAGGT-3') (SEQ ID NO: 51) and PGK4 (5'-GCTGCTAAAGCGCATGCTCCAGACTG-3') (SEQ ID NO: 52) for the targeted ES cell clone and with the primers TgTP-2U (5'-TCTATCGCCTTCTTGACGAGTTCTTCTGAG-3') (SEQ ID NO: 8) and TgTP-3L (5'-CAAGCGCGCAATTAACCCTCACTAAAGG-3') (SEQ ID NO: 9) for MEL cell clones. PCR conditions were 95°C for 15 min, followed by 30 cycles of 94°C for 1 min, 60°C for 1 min, and 72°C for 1 min, followed by a final extension at 72°C for 7 min. To quantitate the expression level of the SB transposase, total RNA was sing TRIzol (Invitrogen) in accordance with the manufacturer's protocol and treated with RQ1 RNase-Free DNase (Promega) . One microgram of RNA was reverse transcribed with SuperscriptII (Invitrogen) by using random hexamer primer in a total volume of 20 $\mu$l in accordance with the manufacturer's protocol, and 2 $\mu$l of the reaction product was analyzed by PCR. PCR primers were 5'-AATAGAACTGTTTGGCCATAATGACCATCG-3' (SEQ ID NO: 53) and 5'-ATCCACATAATTTTCCTTCCTCATG-3' (SEQ ID NO: 54) for the amplification of the SB transposase gene and 5'-CAGGGTGTGATGGTGGGAATGGGTCAGAAG-3' (SEQ ID NO: 55) and 5'-TACGTACATGGCTGGGGTGTTGAAGGTCTC-3' (SEQ ID NO: 56) for the amplification of the beta-actin gene that was used as an internal control. PCR conditions were the same as those used for the amplification of the excision product except that the number of cycles was 30 for the SB transposase gene and 18 for the beta-actin gene.

(Results)

Figure 2B shows a Southern blotting which shows a result of showing that the recombination is correctly performed. This figure shows restriction map of each strain on the right hand side. Clones 5M1, 5M2, and 5M3 are cell lines with methylated transposons from RL5 introduced thereinto, and clones 5N1, 5N2 and 5N3 are cell lines with unmethylated transposons from RL5 introduced thereinto. Further, 6M1 refers to a cell line with methylated transposons from RL6 introduced thereinto, and 6N1 refers to a cell line with unmethylated transposons from RL6 introduced thereinto. The bands having expected size have been detected. Accordingly, recombination via Cre-loxP are shown to properly arise. As used herein, EGFP was used as a probe.

Further, it was confirmed that the methylation introduced and non-methylation have been maintained. The results are shown in Figures **2C-D**. Experiments were conducted by means of Southern blotting using methylation sensitive restriction enzyme HpaII (New England Biolabs) .

The left hand and right hand panels show the results of restriction enzyme map and Southern blot analyses of directions A and B, respectively. In any event, it was observed that methylation introduced was maintained from the fact of band pattern, and no new methylation was introduced in the case of non-methylation.

Figure **2E** depicts production of a cell having methylated SB transposon or unmethylated SB transposon at the desired genetic locus of the genome. It shows a fluorescent activated cell sorting (FACS) profile for EGFP expression in a target clone. Gray region: wild-type cell. Light line: target clone. As shown in Figure **2E**, enhancement of efficiency of transposon methylation was demonstrated in the protein expression level.

Both in RL5 derived clone and RL6 derived clone transfected with the methylated transposons, HpaII and SalI sites were neither digested. This shows that the methylation state has been maintained. In contrast, both HpaII and SalI sites have been completely digested in all the clones transfected with the unmethylated transposons, which shows that the unmethylated state has been maintained. Concurrent with these results, the expression of EGFP reporter in the transposon vector was suppressed in a cell comprising the methylated transposon, but not in a cell comprising the unmethylated transposon (see Figure **2E**). Both methylated and unmethylated states in the transposon region were maintained for at least 10 weeks after the RMCE reaction.

As such, it was shown that the enhancement of efficiency of transposon by the methylation was also demonstrated in the protein expression level, and thus was demonstrated to be stably introduced therein.

(Example 3: Effects of DNA methylation in the same site in the murine genome on a transposon excision reaction)

The cell line established in Example 2, was introduced with SB transposase (PCMV-SB, available from P. Hackett, U. of Minnesota), and genomic DNA was recovered 48 hours thereafter. The collection of the genome was conducted using a genome extraction kit (DNeasy Tissue Kit, QIAGEN). Nested PCR was used for detecting the excision of a transposon. Ten rounds of PCR reactions were conducted per cell line. One $\mu$g of genomic DNA was used as a template for reaction in the 1st PCR. The primers used are shown as follows:

1-1) TgTP-1U (SEQ ID NO: 6)
1-2) TgTP-2L (SEQ ID NO: 7)
2-1) TgTP-2U (TCTATCGCCTTCTTGACGAGTTCTTCTGAG; SEQ ID NO: 8) (2nd PCR; nested PCR)
2-2) TgTP-3L(CAAGCGCGCAATTAACCCTCACTAAAGG; SEQ ID NO: 9) (2nd PCR; nested PCR)

The thus obtained sample (1 $\mu$g) was used as a template for the nested PCR for the following experiments. The genomic DNA of each cell line was used as a negative control (NC). In the transposons with methylation introduced therein, significant increase (at least 10 folds or greater) was found in the frequency of the excision response.

Figure **3A** is an example of showing effects of DNA methylation on the reaction of transposon excision in the murine genome.

Figure **3B-C** depicts the details of frequent excision of methylated SB transposon in the predetermined locus of the genome. A portion thereof overlaps with Figure **3A.** One micro gram or 10 nano gram of clones shown in Figure **2** have been used as a template for nested PCR. For each clone, 10 rounds of PCR have been performed. (B) Direction D; (C) Direction I. NC: genomic DNA which has not been transfected as negative control, M: 100 bp ladder. Right panel shows methylation state of the transposon region and the parent clone.

In order to determine the effects of CpG methylation on the excision of transposon, the present inventors have transfected a clone with a SB transposase expression vector, and conducted a PCR for detecting the excision forty-eight hours after the transfection (see Figures **4A** and **4B**). In the clones comprising unmethylated transposon, when 1 $\mu$g of genomic DNA is used as a template, the excision was detected one in ten rounds of PCR (Clones 5U1 or 5U2) or undetected (Clone 5U3 or 6U1). This frequency was similar to that observed in the ES cell having unmethylated transposon in the Sptlc2 locus (see Figure **1B).** On the other hand, in the clones comprising the methylated transposon, the excision was detected in all ten reactions (Clones 5M1, 5M2, 5M3 and 6M1). When the genomic DNA was diluted for the first PCR, 10 ng of the genomic DNA derived from the clone comprising the methylated transposon still presented positive signal (Figure 3B, Clones 5M3 and 6M1). When the amount of the SB transposase expression vector showed reduction by about 10 % for three consecutive rounds, the methylated transposon showed high excision in a consistent manner (data not shown) . This suggests that the expression level of the SB transposase is a cause of the change in excision frequency. This result shows that the excision efficiency of transposon is at least 100 times higher in the methylated transposon than the conventional. This result is consistent with the increase of about 100 times in excision efficiency in a methylated transposon, which was used for transient transfection (Figure **1B).**

As described above, it was demonstrated that the methylation of a portion encoding a transposon promotes excision reaction of a transposon.

(Example 4: Effects of methylation on the insertion of the transposon into the genome)

In the present Example, the inventors conducted detection of the integration of a plasmid DNA containing transposon into the genome. Promoter trap type transposon was constructed. The method for this construction is briefly described herein below. XhoI fragment from pROSA β geo was replaced with the EcoRI-BamHI fragment from pTransCX-GFP.

Trap-type transposon was inserted inside into the gene of the murine genome, and if the framework of the amino acids are consistent, then the β-geo (pROSAβgeo (Genes & Development 5:1513-1523,1991) is expressed and the cell will be resistant to the antibiotic G418 (Geneticin, Invitrogen). The structure of the plasmid herein used is shown in Figure **4A.** Abbreviations in Figure 4A are: SA, splice acceptor, β-geo: fusion gene with β-gal and neomycin resistant gene.

The promoter trap type transposon was methylated using SssI CpG methylase, and was integrated into a murine embryonic stem (ES) cell (RIES cell, A. Nagy) with an SB transposase enzyme. Thereafter selection was conducted using G418 for seven days. After fixation with methanol, Giemsa staining was conducted (Nakalai Tesque, Kumamoto, Japan).

(SB transposase- and Tc3-mediated integration of promoter trap transposon)

For SB transposase-mediated integration of the promoter trap transposon, 2.0 X $10^5$ ES cells were transfected with 1.6 $\mu$g of methylated or unmethylated pTransSAβ-geo together with 0.4 $\mu$g of pSB10 by using TransFast and plated into 1 well of a 24-well plate. The transfected cells were then transferred to a 10-cm dish 48 h posttransfection and selected for 7 days with 150 μg of G418/ml, after which the colonies were Giemsa stained and counted. Several G418-resistant clones were expanded, and their DNAs and RNAs were extracted. The flanking sequences of integration sites were determined by ligation-mediated PCR as described previously (Horie, K., A. Kuroiwa, M. Ikawa, M. Okabe, G. Kondoh, Y. Matsuda, and J. Takeda. 2001; Proc. Natl. Acad. Sci. USA 98 : 9191-9196) and were analyzed by using the Ensembl mouse genome database (version 16.30.1). Promoter trap events were verified by reverse transcription (RT)-PCR with the transposon-specific primer β-geo (5'-TGCCAGTTTGAGGGGACGACGACAGTATCG-3') (SEQ ID NO: 57) and the gene-specific primers 5'-TGGAGTGAGCTAGAATCAGAAAGATGACAC-3' (SEQ ID NO: 58) for M2S, 5'-GACTTTCAAGACCTTCGACGCACCGTTCAC-3' (SEQ ID NO: 59) for M2L, 5'-TCTTCAGCCACAGGCTCCCAGACATGACAG-3' (SEQ ID NO: 60) for M4, and 5'-GATATGAAGAGCTGTCAGTTTGTAGCAGTC-3' (SEQ ID NO: 61) for N1. PCR products were directly sequenced. For Tc3-mediated integration of the promoter trap transposon, 2.0 X $10^5$ ES cells were transfected with 1.0 $\mu$g of methylated or unmethylated pTc3/SAβ-geo together with 1. 0 $\mu$g of pRP2302 (Fischer, S.E., E.Wienholds, and R.H.Plasterk.2001; Proc.Natl.Acad.Sci.USA 98:6759-6764), an expression vector of Tc3 transposase, by using TransFast. Transfected cells were passaged and selected by G418 under the conditions described above.

Figure **4B** shows the results. As is seen from the number of blue spots on the disc, it was revealed that the methylated transposon significantly increased the number of G418 resistant colonies than the unmethylated transposon.

Figure **4B** depicts effects of CpG methylation on the SB transposition. Average values of G418 resistant colony per culture dish against three independent transfections are shown in the right panel. Error bar shows standard deviation. As shown, CpG methylation significantly enhances introduction of a gene.

Figure **4C** depicts the structure of a gene trapped and transposon insertion site. The insertion site is shown with black arrows. Clones M1-M4 are derived from transfection with methylated transposon, and Clones N1-N6 are derived from non-methylated transposon. The number of chromosomes, Ensemble gene designator, and the gene nomenclature are also shown. Except Clone M2, in which two insertion site have been characterized (called M2S and M2L) , one insertion site has been identified for each clone. Correct splicing in some clones (M2S, M2L, M4 and N1) have been confirmed with RT-PCR using primers to the upstream exon (white arrow) and transposon. Solid square: exon. Scale bar for 5kb is shown in the right hand.

Figure **4D** shows effect of methylation on Tc transposition. Construct of trapping vector is shown in the upper panel. TIR: terminal inverted repeat. Three independent transfections are shown in the lower panel with respect to the average number of G418 resistant colony per dish.

As shown in Figure **4,** the effects of methylation on the complete transposition was investigated. As described above, the excision of transposons from the genomic locus (the first step of the transposition reaction) was enhanced by the methylation of CpG. The excised transposon fragment needs to be reintegrated into the genome to complete the transposition reaction. We therefore compared methylated and unmethylated transposons in terms of the number of transposons integrated into the genome. Since CpG methylation inhibits promoter activity, the expression cassette of selection markers within a transposon could not be utilized to detect transposition into the genome. Instead, we employed a gene trap scheme (Friedrich, G., and P.Soriano.1991; Genes Dev.5:1513-1523) by constructing a gene trap-type transposon vector, pTransSAβ-geo, which contained a splice acceptor site upstream of β-geo (Fig. 4A). When the transposon is integrated into an active gene, a chimeric fusion transcript consisting of an endogenous gene and β-geo is thought to be generated. If β-geo is in frame with the trapped gene, cells become resistant to G418 due to the expression of β-geo (Fig. 4A). The methylated or unmethylated transposon vector was introduced into ES cells together with the SB transposase expression vector and selected with G418. The methylated transposon produced 1,600 colonies, while the unmethylated transposon yielded 140 colonies (Fig. 4B). This result indicates that the efficiency of overall transposition, which includes both excision and integration, is enhanced 11-fold in the methylated transposon. Transposon insertion sites were determined by ligation mediated PCR, and gene trap events were verified in randomly selected clones by RT-PCR analysis (Fig. 4C), and the results obtained indicated that vector methylation did not affect gene trap selection.

To test the effect of methylation in a different transposon system, we constructed another gene trap-type vector, pTc3/SAβ-geo, by using the Tc3 transposon derived from C. elegans (Fischer, S. E., H. G. van Luenen, and R. H. Plasterk. 1999. ; Mol. Gen. Genet. 262: 268-274.; and Fischer, S. E.,E. Wienholds, and R. H. Plasterk. 2001; Proc. Natl. Acad. Sci. USA 98:6759-6764) and introduced this vector with or without methylation into ES cells together with the Tc3 transposase expression vector. CpG methylation increased gene trap events two fold (Fig. 4D) . This result indicates that the enhancement of transposition by CpG methylation is seen in other transposon systems, although the extent of enhancement varies.

Accordingly, it was demonstrated that methylation significantly enhances the integration of transposon into the genome.

(Example 5: Expression of Protein)

(Protein Expression and EMSA)

The N123 peptide comprising IR/DR recognition domain was expressed and purified as described previously (Ivics, Z., P.B. Hackett, R. H. Plasterk, and Z. Izsvak. 1997; Cell 91: 501-510). The oligonucleotide sequence for the oligonucleotide probe corresponding to the outer binding sites of IR/DR-L were, for unmethylated probe: 5'-TACAGTTGAAGTCGGAAGTTTACATACACTTAAG-3' (Unmet-U) (SEQ ID NO: 62) and 5'-CTTAAGTGTATGTAAACTTCCGACTTCAACTGTA-3' (Unmet-L) (SEQ ID NO: 63), and for methylated probes: 5'-TACAGTTGAAGTOGGAAGTTTACATACACTTAAG-3' (Met-U) (SEQ ID NO: 64) and 5'-CTTAAGTGTATGTAAACTTCOGACTTCAACTGTA-3' (Met-L) (SEQ ID NO: 65) (as used herein the symbol "O" refers to 5-methyl cytosine). This oligonucleotide was first labeled with [$\gamma$-$^{32}$P] ATP using T4 polynucleotide kinase, and then annealed. DNA fragment of IR/DR-L was digested with HindIII and KpnI for the preparation, and thereafter, the fragment was methylated using SssI methylase or kept unmethylated and then the fragment was treated with an alkaline phosphatase. The digested fragment was extracted using phenol-chloroform, and precipitated with ethanol, and then finally, T4 polynucleotide was used to label with [$\gamma$-$^{32}$P]ATP. In the presence of poly(dI-dC) (1.0$\mu$g), 0.2 pmol of oligonucleotide probe or 100 pg of IR/DR-L fragment, and 1.0$\mu$l of N123, a nucleoprotein complex was formed in a buffer described previously in Ivics , Z., P. B. Hackett, R. H. Plasterk, and Z. Izsvak. 1997; Cell 91:501-510). After a thirty minute incubation at 25 degrees Celsius, samples were loaded on a 5%-native polyacrylamide gel, and electrophoresed at 150V constant voltage, and in 0.5xTris-borate-EDTA, 1.5 hours for oligonucleotide probe and 2.5 hours for IR/DR-L fragment. After the gel was dried, the nucleoprotein was visualized by autoradiography.

(ChIP Assay)

According to the manufacturer's protocol, ChIP assay kit (Upstate) was used for chromatin immunoprecipitation (ChIP). The antibodies used in the present study were anti acetyl H3 (Upstate) and anti-trimethylated H3K9 (Abcam). Precipitated DNA was used for analysis by PCR in a plurality of loci, for amylase 2.1: primers 5'-CCTTGTACGGGTTGGTGGAGGTCAC-3'(SEQ ID NO: 66) and 5'-CGCCACTCGAACAGGTGGACAATAG-3'(SEQ ID NO: 67), for β-small globin gene, 5'-TGCGAGGATAAGAACAGACACTAC-3'(SEQ ID NO: 68) and 5'-ACAGACTCAGAAGCAAACGTAAGA-3' (SEQ ID NO: 69); for EGFP gene, EGFP-1U and EGFP-1L, for IR/DR-L, 5'-GCACGGGTGTTGGGTCGTTTGTTC-3' (SEQ ID NO: 70) and 5'-CTTCTAAAGCCATGACATCATTTTCTG-3'(SEQ ID NO: 71), and for IR/DR-R, 5'-GAAGGCTACTCGAAATGTTTGACCCAAG-3'(SEQ ID NO: 72) and 5'-CAAGCGCGCAATTAACCCTCACTAAAGG-3'(SEQ ID NO: 9) are used. PCR conditions used are as follows: 95 degrees Celsius for 15 minutes using HotStarTaq, followed by 35 cycles of 94 degrees Celsius for thirty seconds, 60 degrees Celsius for thirty seconds, and 72 degrees Celsius for one minute, and finally the final extension at 72 degrees Celsius for seven minutes. In order to allow semi-quantitative analysis, 1 $\mu$l, 0.2 $\mu$l, 0.04 $\mu$l and 0. 008 $\mu$l of input DNA per reaction was used, and 1 $\mu$l and 0.2 $\mu$l of DNA were precipitated per reaction. For EGFP, IR/DR-L and IR/DR-R in the fractions of anti-trymethylated H3K9, 5 $\mu$l and 1 $\mu$l of DNA were used per reaction.

Figure **6** shows invariant affinity of SB transposon DNA binding domain against naked methylated IR/DR.

Figure **6A** shows an exemplary drawing of CpG sites in the IR/DR and transposase binding sites. Solid circle: CpG sites.

Figures **6B-E** shows electrophoretic mobility shift assay (EMSA) in the recombinant SB transposase peptide (N123).

Figures **6B** and **6C** shows results obtained in the external binding site of IR/DR-L 34 bp, and Figures 6D and **6E** show the results obtained by IR/DR-L fragment of 300 bp.

As shown in Figure **6B,** a concentration of N123 peptide sufficient for enhancing non-methylated or methylated external binding sites (1600 to 100 dilution of the purified peptide) mixed therewith to form a nucleoprotein complex.

As shown in Figure **6C,** non-methylated external binding site has been labeled for use as a probe, and non-methylated external binding site or methylated external binding site have been used as competitive substance in an increased concentration (10-50 folds molar excessive probes).

As shown in Figure **6D,** non-methylated IR/DR-L fragment or methylated IR/DR-L fragment have been mixed with an increased concentration of N123 peptides (5100-160 folds dilution of the purified peptides) for formation of nucleoprotein complex.

As shown in Figure **6E,** non-methylated IR/DR-L fragment was labeled for use as a probe, and non-methylated or methylated IR/DR-L fragments have been used as a competitive substance in an increased concentration (500-8000 folds molar excessive probes). Unmet: non-methylated, Met: methylated, F: free probe, C: complex. Complexes 1 (C1) and 2 (C2) indicate one and two molecule(s) of N123 peptides per IR/DR, respectively (Ivics, Z., P. B. Hackett, R. H. Plasterk, and Z. Izsvak. 1997; Cell 91: 501-510).

Figure **7** shows ChIP assay of methylated transposon or non-methylated transposon in a predetermined locus.

Figure **7A** shows SB transposon region, which was analyzed in the ChIP assay. PCR amplification region is shown as light line under each component. Solid triangle: lox 511 site.

Figures **7B** and **7C** show PCR analysis of precipitated DNA. As shown in Figures **3** and **4,** Clones 5M3 and 5U3 (B) and 6M11 and 6U1 (C) are derived from parent clone RL5 and RL6, respectively. Input and precipitated DNA have been analyzed in a five-fold stepwise dilution. AcH3: anti-acetylated H3, MeH3K9; anti-trymethylated H3K9, no Ab: control having no antibodies. Amylase 2.1 and beta-small globin have been used as a typical control against heterochromous region and euchromatin region. Intensity of bands of amylase 2.1 and beta-small globin are the same in the anti-acetyl H3 fraction, whereas the higher amplification efficiency than amylase 2.1 sequence in the input DNA, indicates that beta-small globin is enriched compared with the amylase 2.1 sequence in this fraction. Minor bands found in the control lanes without antibodies are derived from non-specific binding of the genomic DNA against the protein. Agarose beads are used for preparation. Determination has conducted twice. Representative results are shown.

As shown in Figures **6** and **7,** unchanged affinity of the SB transposase DNA binding domain to naked methylated IR/DR was demonstrated in the present invention.

In order to clarify the mechanism of transposition which has been enhanced by methylation of CpG, the present inventors have compared the affinity of recombinant SB transposase peptide against the methylated IR/DR and unmethylated IR/DR by EMSA. Both right and left IR/DR have two binding sites against the SB transposase (Figure **6A)** . Since only outer binding site comprises CpG sequence (Figure **6A)** , the binding site of the outer left was used as a probe and antagonist. The first 123 amino acids (N123) of SB transposase, which was previously reported to comprise IR/DR recognition domain (Ivics, Z., P. B. Hackett, R. H. Plasterk, and Z. Izsvak. 1997; Cell 91: 501-510), was used to express in Escherichia coli, to purify by means of histidine tag at the C-terminus, and to produce a nucleoprotein. If unmethylated or methylated binding sequence was used as a probe, the intensity of shifted bands are comparable (Figure **6B).** When unmethylated or methylated site was used as an antagonist against a labeled unmethylated probe, no difference was observed (Figure **6C).** The same experiment using the entire region of the left IR/DR fragment was used as a probe and an antagonist, no action of methylation was observed (Figures **6D** and **6E).** These results indicate that the CpG methylation of IR/DR does not change the direct binding of N123 peptide against a naked IR/DR, and indicated that it may be necessary that native chromatin structure in the transposon region enhances the transposition.

As shown in Figure 7, chromatin structure in the methylated and non-methylated transposons was confirmed.

DNA recombination reaction may be affected by the change in higher dimension of structure of the DNA substrate. For example, E.coli protein HU stimulate recombinant efficiency in bacteriophage Mu by binding to transposase binding site. Similar action of high mobile group B1 (HMGB1) protein relating to SB transposition was recently reported. The present inventors have therefore, investigated chromatin structure in the methylated and unmethylated transposon in the MEL cell clone using ChIP assay. It was shown that the modification of the histone tail played an important role in regulating chromatin structural machinery and gene expression in a higher stage. Recent studies shows that acetylated histone H3 is located in euchromatin where DNA is roughly packaged and methylated histone H3 was located onto heterochromatin by nine lysine residues (H3K9) , where DNA was concentrated (Grewal, S. I., and S. C. Elgin. 2002; Curr. Opin. Genet. Dev. 12: 178-187). The inventors therefore used an antibody recognizing acetyl H3 or trimethyl H3K9 in the ChIP assay to compare methylated transposon regions of two different loci and chromatin structures of unmethylated transposon using RL5 derived clone and RL6 derived clone (Figure 7). As an internal standard for the ChIP assay, pancreas amylase 2.1 gene was used as a heterochrome marker, and beta-small globin gene was used as a euchromatic marker (Dhar, V., A. I. Skoultchi, and C.L.Schildkraut.1989.; Mol. Cell. Biol. 9: 3524-3532; and Schubeler, D., C. Francastel, D. M. Cimbora, A. Reik, D. I. Martin, and M. Groudine. 2000; Genes Dev. 14: 940-950) . As shown in Figure 7, in proportion of those enriched for acetyl H3, the amount of amylase 2.1 gene in the fraction enriched for trimethylated H3K9, was significantly higher than that for beta-small globin, which concurs the expectation that amylase 2.1 gene and beta-small globine gene are located in heterochromatin and euchromatin, respectively. Enrichment of transposon sequence (IR/DR-R, EGFP and IR/DR-L) in the acetyl H3 fraction was observed in the clones comprising unmethylated transposons (5U3 and 6U1). In contrast, enrichment in the trimethylated H3K9 fraction, the clones comprising methylated transposon (5M3 and 6M1) have been observed. The enrichment was closely correlated to the expression pattern of the GFP reporter (Figure **3D)**. These results indicate that the methylated transposon region and unmethylated transposon region have involved in formation of heterochromatin and euchromatin, respectively.

As such, the transposition efficiency of a transposon was significantly enhanced in the protein expression level. Further, the mechanism therefore was also elucidated.

(Example 6: production of a transgenic mouse)

A system according to Example 4 was used to produce a transgenic mouse. In brief, ES cells obtained in Example 4 was injected into the blastcyst and the same was returned back to the uterus of a pseudo pregnant mouse to produce a mouse. This mouse has also been demonstrated that the methylation against the insertion of the transposon into the genome has significantly enhances the efficiency.

(Example 7: Examples using naturally occurring embodiments)

Next, it was demonstrated that the methylation is useful for production of a transgenic biological organism. In brief, a mouse was produced by means of simultaneously injecting a methylated transposon DNA and a transposase RNA into a fertilized egg. This mouse also demonstrated that the methylation significantly enhances the efficiency by the insertion of a transposon into the genome.

(Example 8: Examples of different transposons)

Next, as one of Tc1/mariner, Tc (Accession No. X01005) was used in lieu of SB system used in Example 1, to conduct the same experiments as in Examples 1-3. This mouse also demonstrated that the methylation significantly enhances the efficiency by the insertion of a transposon into the genome.

(Example 9: Example of a different transposon)

Next, as one of minos-2 (Accession No. Z29098) was used in lieu of SB system used in Example 1, to conduct the same experiments as in Examples 1-3. This mouse also demonstrated that the methylation significantly enhances the efficiency by the insertion of a transposon into the genome.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

INDUSTRIAL APPLICABILITY

The invention is used for allowing efficient production of a transgenic biological organism (in particular, vertebrates such as mammals). Such a biological organism is extremely useful as a model animal, screening, pharmacological tests and the like.

## Claims

**1.** An isolated nucleic acid molecule having a nucleic acid sequence encoding a transposon, wherein the nucleic acid sequence has a methylation at at least one nucleotide.

**2.** An isolated nucleic acid molecule according to Claim 1, further comprising a nucleic acid molecule encoding a desired gene.

**3.** An isolated nucleic acid molecule according to Claim 1, wherein said methylation is present at least C in a CG sequence.

**4.** An isolated nucleic acid molecule according to Claim 1, wherein said transposon is of a DNA-type.

**5.** An isolated nucleic acid molecule according to Claim 1, wherein said transposon belongs to Tc1/mariner types.

**6.** An isolated nucleic acid molecule according to Claim 1, wherein said transposon comprises *Sleeping Beauty.*

**7.** An isolated nucleic acid molecule according to Claim 2, wherein said desired gene is operably linked to said transposon, or is capable of being operably linked to said transposon when intracellularly introduced.

**8.** An isolated nucleic acid molecule according to Claim 1 for use in introducing a foreign gene into a host.

**9.** An isolated nucleic acid molecule according to Claim 8, wherein said host comprises a eukaryote.

**10.** An isolated nucleic acid molecule according to Claim 8, wherein said host comprises a mammal.

**11.** An isolated nucleic acid molecule according to Claim 8, wherein said host comprises a rodent.

**12.** An isolated nucleic acid molecule according to Claim 1, wherein a transposase functions at a location on a genome to which said nucleic acid molecule is inserted.

**13.** A gene cassette having a nucleic acid sequence encoding a transposon, wherein said nucleic acid sequence has a methylation at at least one nucleotide.

**14.** A vector having a nucleic acid sequence encoding a transposon and a nucleic acid sequence encoding a desired gene, wherein said nucleic acid sequence has a methylation at at least one nucleotide.

**15.** A vector according to Claim 14, wherein said methylation is present at least C in a CG sequence.

**16.** A vector according to Claim 14, wherein said transposon is of a DNA-type.

**17.** A vector according to Claim 14, wherein said transposon belongs to Tc1/mariner types.

**18.** A vector according to Claim 14, wherein said transposon comprises *Sleeping Beauty.*

**19.** A vector according to Claim 14, wherein said desired gene is operably linked to said transposon, or is capable of being operably linked to said transposon when intracellularly introduced.

**20.** A vector according to Claim 14 for use in introducing a foreign gene into a host.

**21.** A vector according to Claim 20, wherein said host comprises a eukaryote.

**22.** A vector according to Claim 20, wherein said host comprises a mammal.

**23.** A vector according to Claim 20, wherein said host comprises a rodent.

**24.** A vector according to Claim 14, wherein a transposase functions at a location on a genome to which said nucleic acid molecule is inserted.

**25.** A composition for rendering a transposase to act on a foreign nucleic acid molecule to be inserted on a genome, wherein said composition comprises a nucleic acid sequence encoding a transposon and a nucleic acid sequence encoding a desired gene, wherein said nucleic acid sequence has a methylation at at least one nucleotide.

**26.** A cell comprising a nucleic acid sequence encoding a transposon and a nucleic acid sequence encoding a desired gene, wherein said nucleic acid sequence has a methylation at at least one nucleotide.

**27.** A cell according to Claim 26, wherein said methylation is present at least C in a CG sequence.

**28.** A cell according to Claim 26, wherein said transposon is of a DNA-type.

**29.** A cell according to Claim 26, wherein said transposon belongs to Tc1/mariner types.

**30.** A cell according to Claim 26, wherein said transposon comprises *Sleeping Beauty.*

**31.** A cell according to Claim 26, wherein said desired gene is operably linked to said transposon.

**32.** A cell according to Claim 26 for use in introducing a foreign gene into a host.

**33.** A cell according to Claim 26, wherein said host comprises a eukaryote.

**34.** A cell according to Claim 26, wherein said host comprises a mammal.

**35.** A cell according to Claim 26, wherein said host comprises a rodent.

**36.** A tissue comprising a nucleic acid sequence encoding a transposon and a nucleic acid sequence encoding a desired gene, wherein said nucleic acid sequence has a methylation at at least one nucleotide.

**37.** A tissue according to Claim 36, wherein said methylation is present at least C in a CG sequence.

**38.** A tissue according to Claim 36, wherein said transposon is of a DNA-type.

**39.** A tissue according to Claim 36, wherein said transposon belongs to Tc1/mariner types.

**40.** A tissue according to Claim 36, wherein said transposon comprises *Sleeping Beauty.*

**41.** A tissue according to Claim 36, wherein said desired gene is operably linked to said transposon.

**42.** A tissue according to Claim 36 for use in introducing a foreign gene into a host.

**43.** A tissue according to Claim 42, wherein said tissue comprises a eukaryotic tissue.

**44.** A tissue according to Claim 42, wherein said tissue comprises a mammalian tissue.

**45.** A tissue according to Claim 42, wherein said tissue comprises a rodent tissue.

**46.** A biological organism comprising a nucleic acid sequence encoding a transposon and a nucleic acid sequence encoding a desired gene, wherein said nucleic acid sequence has a methylation at at least one nucleotide.

**47.** A biological organism according to Claim 46, wherein said methylation is present at least C in a CG sequence.

**48.** A biological organism according to Claim 46, wherein said transposon is of a DNA-type.

**49.** A biological organism according to Claim 46, wherein said transposon belongs to Tc1/marine types.

**50.** A biological organism according to Claim 46, wherein said transposon comprises *Sleeping Beauty.*

**51.** A biological organism according to Claim 46, wherein said desired gene is operably linked to said transposon.

**52.** A biological organism according to Claim 46, which comprises a eukaryote.

**53.** A biological organism according to Claim 46, which comprises a mammal.

**54.** A biological organism according to Claim 46, which comprises a rodent.

**55.** A biological organism according to Claim 46, wherein said desired gene is not derived from said biological organism.

**56.** A method for producing a transgenic biological organism, comprising the steps of:

A) providing an isolated nucleic acid molecule having a nucleic acid sequence encoding a transposon;
B) transforming a germ cell of a desired biological organism with said nucleic acid molecule;
C) selecting an individual in which the germ cell has a methylation in a nucleic acid sequence encoding said transposon; and
D) regenerating a biological organism using the transformed germ cell.

**57.** A method according to Claim 56, wherein said biological organism comprises a eukaryote.

**58.** A method according to Claim 56, wherein said biological organism comprises a mammal.

**59.** A method for producing a transgenic biological organism comprising the steps of:

A) providing an isolated nucleic acid molecule having a nucleic acid sequence encoding a transposon, wherein the nucleic acid sequence has a methylation at at least one nucleotide;
B) transforming a germ cell of a desired biological organism with said nucleic acid molecule; and
C) regenerating a biological organism using the transformed germ cell.

**60.** A method according to Claim 59, wherein said biological organism comprises a eukaryote.

**61.** A method according to Claim 59, wherein said biological organism comprises a mammal.

**62.** A method according to Claim 59, wherein said biological organism comprises a rodent.

**63.** A kit for producing a transgenic biological organism, comprising:

A) an isolated nucleic acid molecule having a nucleic acid sequence encoding a transposon, wherein the nucleic acid sequence has a methylation at at least one nucleotide; and
B) a transposase.

**64.** A kit according to Claim 63, further comprising instructions indicating a method for use of said nucleic acid molecule and transposase.

**65.** Use of an isolated nucleic acid molecule having a nucleic acid sequence encoding a transposon, wherein the nucleic acid sequence has a methylation at at least one nucleotide for producing a transgenic biological organism.

**66.** A nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated.

**67.** A nucleic acid fragment according to Claim 66, wherein the nucleic acid sequence comprises at least a portion of a foreign gene.

**68.** A nucleic acid fragment according to Claim 66, wherein said nucleic acid sequence comprises at least one expression controlling region.

**69.** A nucleic acid fragment according to Claim 68, wherein the expression controlling region is selected from the group consisting of a promoter, enhancer and silencer.

**70.** A nucleic acid fragment according to Claim 66, further comprising at least a portion of a foreign gene, wherein the nucleic acid sequence is operably linked to a sequence encoding at least a portion of the foreign gene.

**71.** A nucleic acid fragment according to Claim 66 wherein said cell is derived from an animal.

**72.** A nucleic acid fragment according to Claim 71, wherein said cell is obtained from a vertebrate.

**73.** A nucleic acid fragment according to Claim 72, wherein the vertebrate is a mammal.

**74.** A nucleic acid fragment according to Claim 73, wherein the mammal is a primate or a rodent.

**75.** A nucleic acid fragment according to Claim 66, the DNA of the cell is selected from the group consisting of a cellular genome, episome and plasmid.

**76.** A nucleic acid fragment according to Claim 66, wherein said at least one inverted repeat sequence comprising the sequence set forth in SEQ ID NO: 20 or 21, or a portion thereof.

**77.** A nucleic acid fragment according to Claim 66, wherein said transposase is SB protein.

**78.** A nucleic acid fragment according to Claim 77, wherein the transposase has at least 80 % amino acid homology to the sequence set forth in SEQ ID NO: 3.

**79.** A nucleic acid fragment according to Claim 66, wherein said at least one inverted repeat sequence comprises at least one tandem repeat sequence, and the tandem repeat sequence comprises a nucleotide sequence set forth in SEQ ID NO: 26 or that having at least 80% homology to the sequence set forth in SEQ ID NO: 26.

**80.** A nucleic acid fragment according to Claim 66, wherein said at least one inverted repeat sequence comprises at least one tandem repeat sequence, wherein the tandem repeat sequence is selected from the group consisting of nucleic acid sequences set forth in SEQ ID NOs: 22-25.

**81.** A nucleic acid introduction system for introducing into a DNA of a cell another DNA, the system comprising:

A) a nucleic acid fragment comprising a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; and
B) a transposase or a nucleic acid encoding a transposase.

**82.** A nucleic acid introduction system according to Claim 81, therein the transposase is SB protein.

**83.** A nucleic acid introduction system according to Claim 81, wherein said transposase has the amino acid sequence set forth in SEQ ID NO:3 or a variant thereof, or the nucleic acid sequence encoding the transposase has the nucleic acid sequence set forth in SEQ ID NO: 2 or a variant thereof.

**84.** A nucleic acid introduction system according to Claim 81, wherein said nucleic acid encoding the transposase is incorporated into the cellular genome.

**85.** A nucleic acid introduction system according to Claim 81, further comprising a plasmid or a virus vector, wherein said plasmid or virus vector comprises the nucleic acid fragment as a part thereof.

**86.** A nucleic acid introduction system according to Claim 81, wherein nucleic acid fragment comprises at least a portion of a sequence encoding a foreign gene.

**87.** A nucleic acid introduction system according to Claim 81, wherein the nucleic acid fragment is introduced into the cell by means of a method selected from the group consisting of particle bombardment; electroporation; microinjection; use of a gene introduction reagent; and use of a virus vector.

**88.** A method for producing a transgenic biological organism, comprising the steps of:

introducing a nucleic acid fragment and transposase into a pluripotent cell, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated; and
growing the cell into a living body.

**89.** A method according to Claim 88, wherein said pluripotent cell is selected from the group consisting of an oocyte, an embryo, an egg and a stem cell.

**90.** A method according to Claim 88, wherein said biological organism is a rodent or a primate.

**91.** A method according to Claim 89, wherein said biological organism is a mouse or a rat.

**92.** A method for introducing a nucleic acid into a DNA of a cell, comprising the step of:

introducing a nucleic acid fragment into a cell, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated.

**93.** A method according to Claim 92 further comprising the step of introducing a transposase into the cell.

**94.** A method according to Claim 92, wherein the transposase has at least 80 % homology to the sequence set forth in SEQ ID NO: 3.

**95.** A method according to Claim 92, wherein said cell comprises a nucleic acid encoding the transposase.

**96.** A method according to Claim 95, wherein the nucleic acid encoding the transposase is incorporated into the cellular genome.

**97.** A method according to Claim 95, wherein the transposase is stably expressed in the cell.

**98.** A method according to Claim 95, wherein the transposase is operably linked such that it is under the control of an inducible promoter.

**99.** A method according to Claim 92, wherein the nucleic acid sequence encodes a protein.

**100.** A method according to Claim 92, wherein the nucleic acid sequence encodes a marker protein.

**101.** A method for tranposing a nucleic acid sequence in a cell, comprising the steps of:

introducing a transposase into a cell comprising a nucleic acid fragment, wherein the nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated;
wherein the transposase transposes the nucleic acid sequence from a first location in a DNA of the cell to a second location of the DNA.

**102.** A method according to Claim 101, wherein the DNA of the cell is a genomic DNA.

**103.** A method according to Claim 101, wherein the first location is an extrachromosomal DNA.

**104.** A method according to Claim 101, wherein the second location is an extrachromosomal DNA.

**105.** A method according to Claim 101, wherein the transposase introduces a nucleic acid into the cell.

**106.** A method for identifying a gene in a cell, comprising the steps of:

introducing into a cell, a nucleic acid fragment comprises a nucleic acid sequence located between at least two inverted repeat sequences, wherein the inverted repeat sequences have the capability of binding to a transposase, wherein the nucleic acid fragment is capable of being incorporated into a DNA in a cell, wherein at least one nucleotide thereof is methylated, and a transposase;
digesting the DNA in the cell with a restriction endonuclease capable of digesting the nucleic acid sequence;
identifying the inverted repeat sequence;
determining a sequence of a nucleic acid having a similar sequence to the inverted repeat sequence; and
comparing the sequence with sequence information in a sequence information database.

Fig. 1A

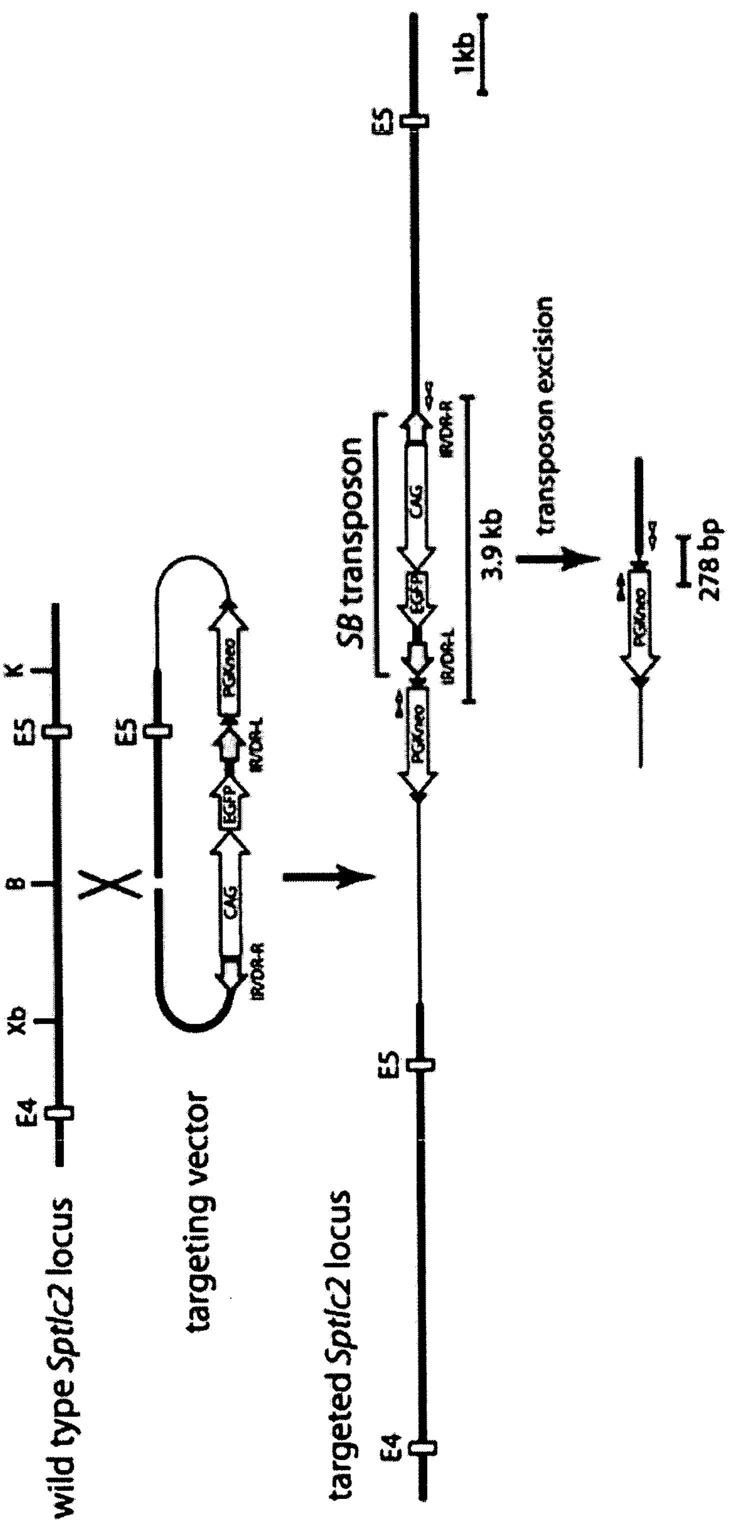
wild type Sptlc2 locus
E4
Xb
B
E5
K
targeting vector
E5
CAG
EGFP
IR/DR-R
IR/DR-L
PGKneo
targeted Sptlc2 locus
E4
E5
SB transposon
3.9 kb
PGKneo
EGFP
CAG
IR/DR-L
IR/DR-R
E5
1kb
transposon excision
PGKneo
278 bp

Fig. 1B

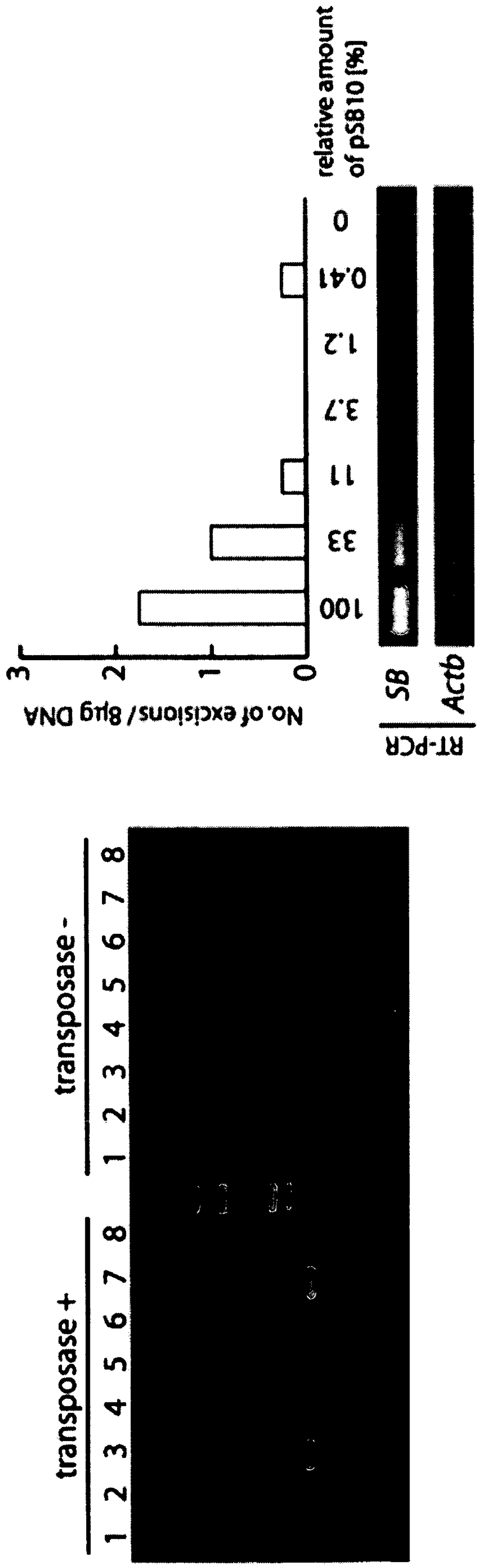
transposase +
1 2 3 4 5 6 7 8
transposase -
1 2 3 4 5 6 7 8
No. of excisions/ 8µg DNA
0
1
2
3
100
33
11
3.7
1.2
0.41
0
relative amount of pSB10 [%]
RT-PCR
SB
Actb

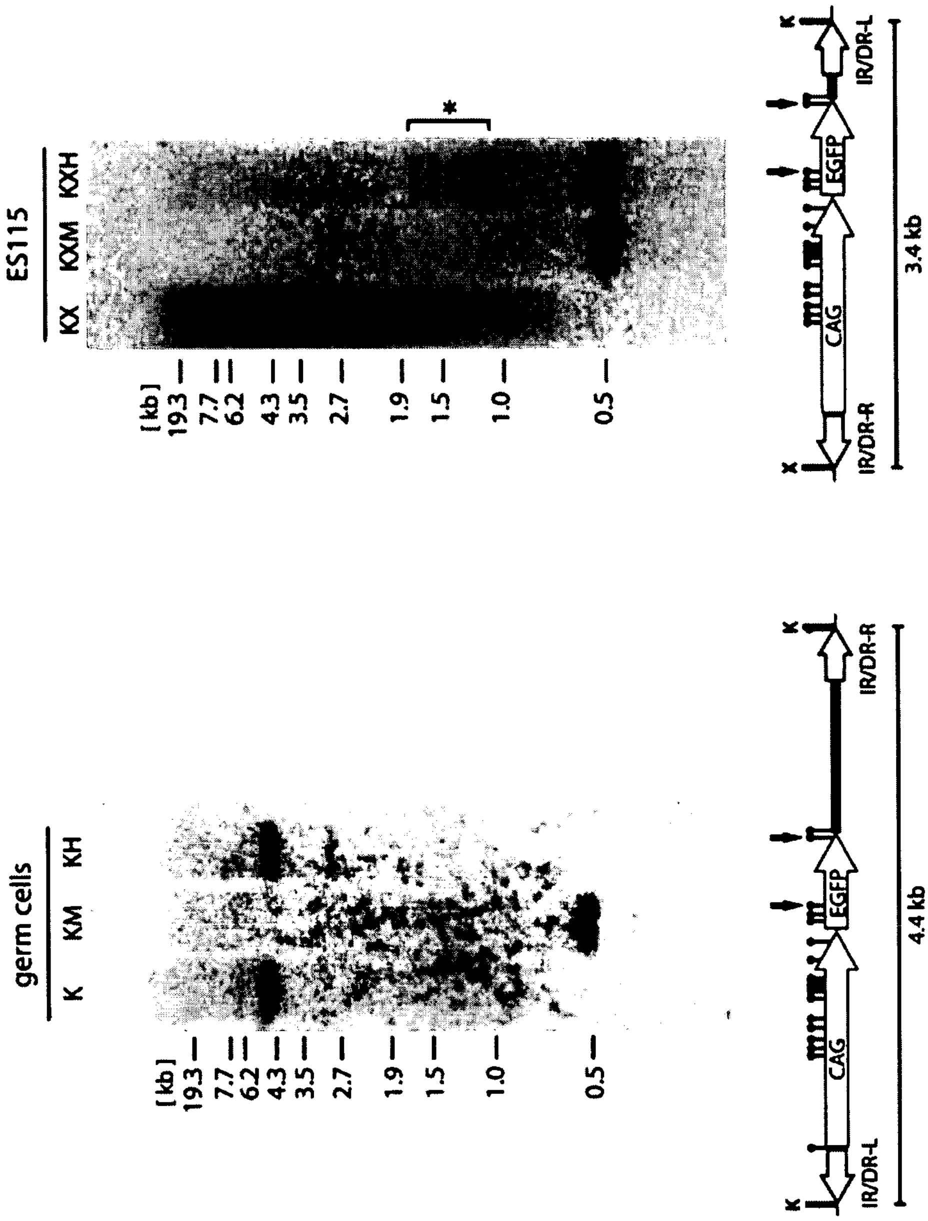
germ cells
K KM KH
[kb]
19.3
7.7
6.2
4.3
3.5
2.7
1.9
1.5
1.0
0.5
K
IR/DR-L
CAG
EGFP
IR/DR-R
K
4.4 kb
ES115
KX KXM KXH
[kb]
19.3
7.7
6.2
4.3
3.5
2.7
1.9
1.5
1.0
0.5
*
X
IR/DR-R
CAG
EGFP
IR/DR-L
K
3.4 kb

Fig. 1C

Fig. 1D

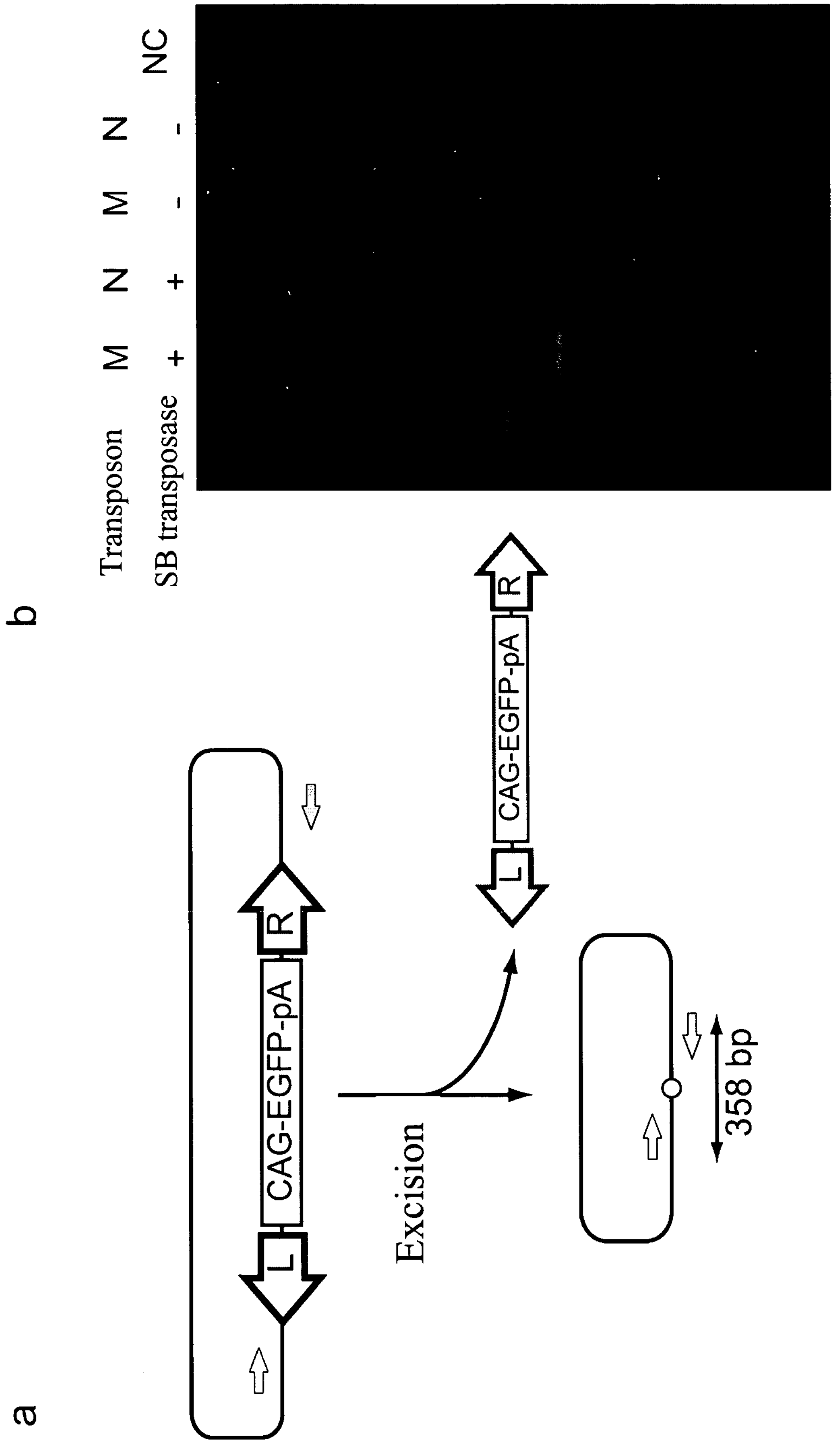
a
CAG-EGFP-pA
L
R
Excision
358 bp
b
Transposon
SB transposase
M
N
M
N
NC
+
+
-
-
CAG-EGFP-pA
L
R

Fig. 2A

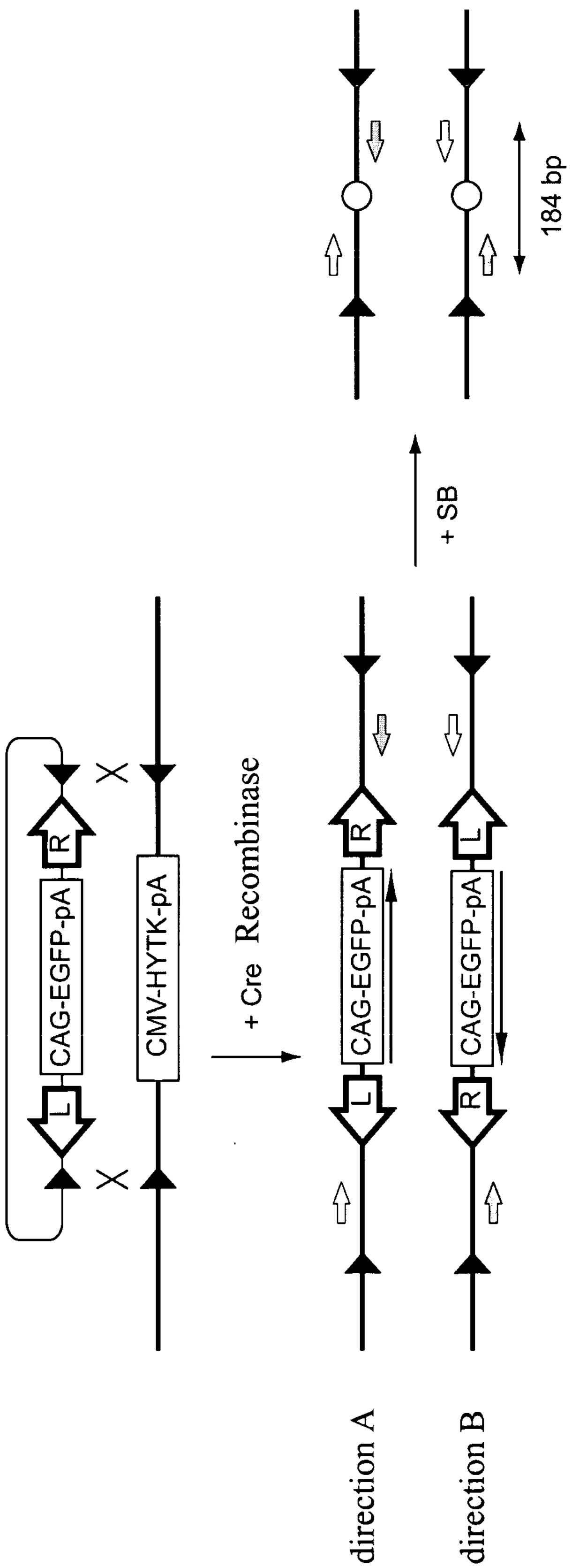
CAG-EGFP-pA
L
R
CMV-HYTK-pA
+ Cre Recombinase
direction A
direction B
+ SB
184 bp

Fig. 2B

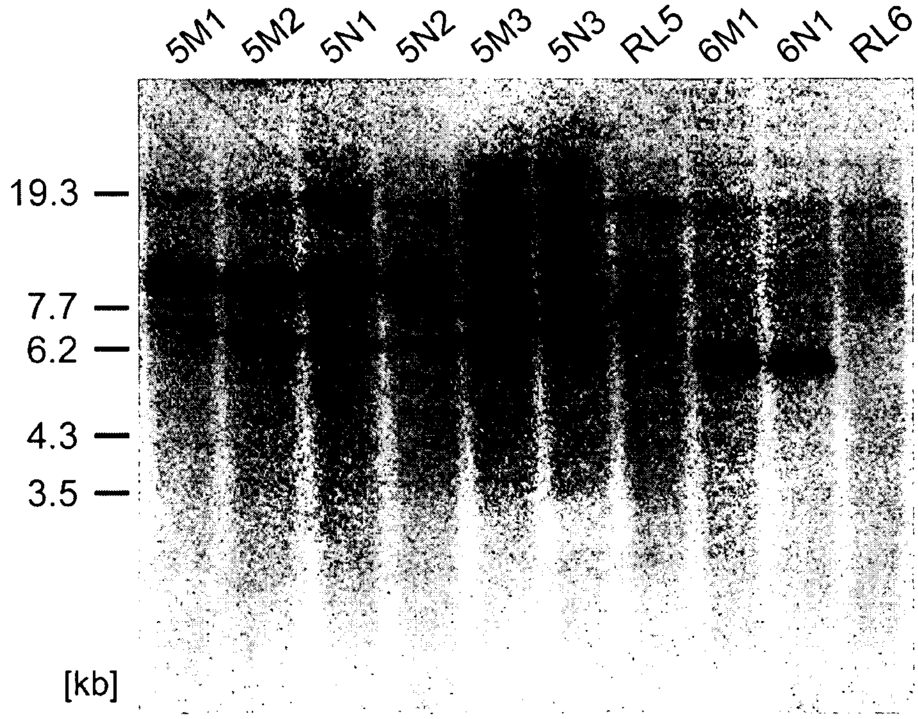

Fig. 2C

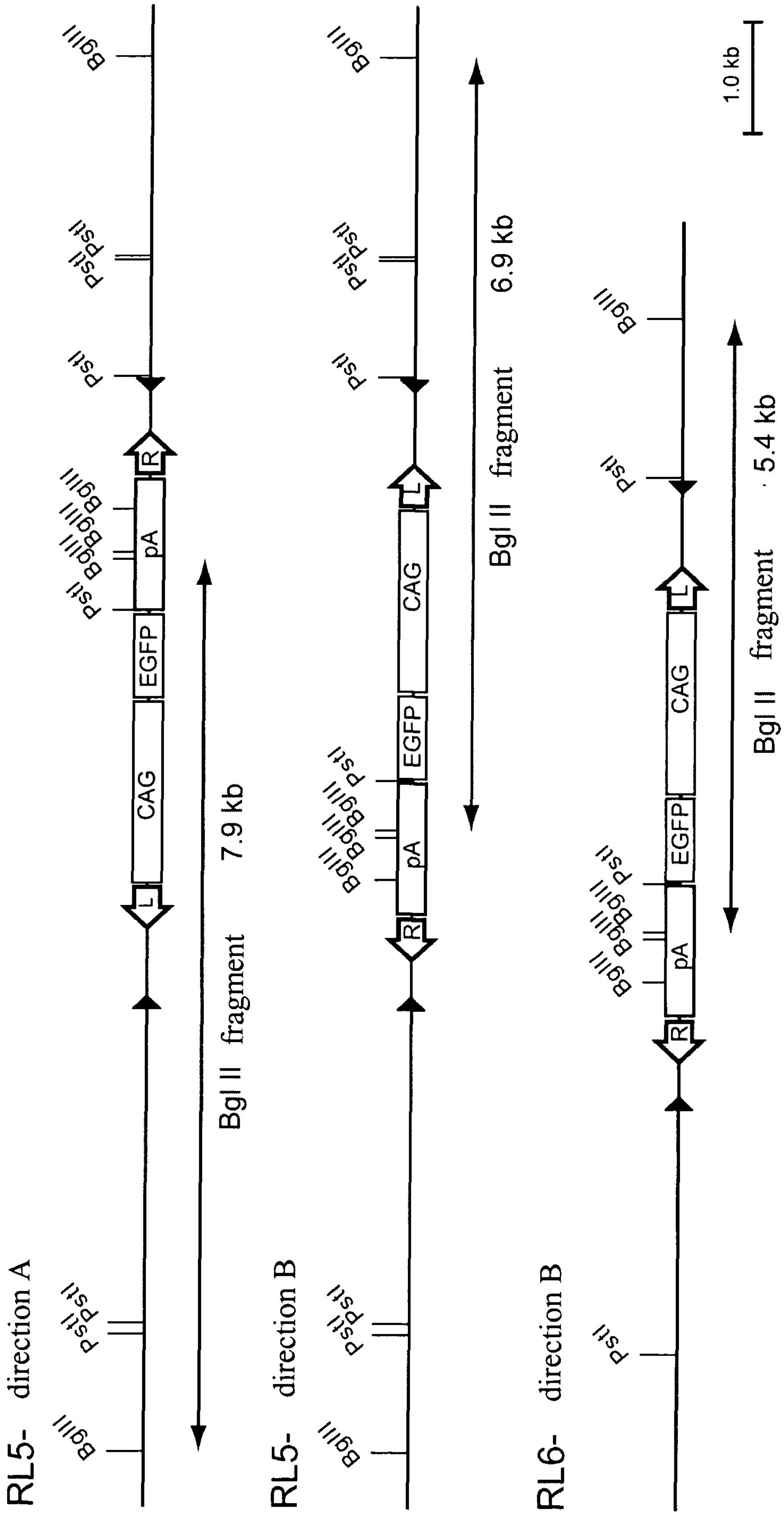
RL5- direction A
BglII
PstI
PstI
CAG
EGFP
pA
L
R
Bgl II fragment 7.9 kb
RL5- direction B
Bgl II fragment 6.9 kb
RL6- direction B
Bgl II fragment 5.4 kb
1.0 kb

Fig. 2D

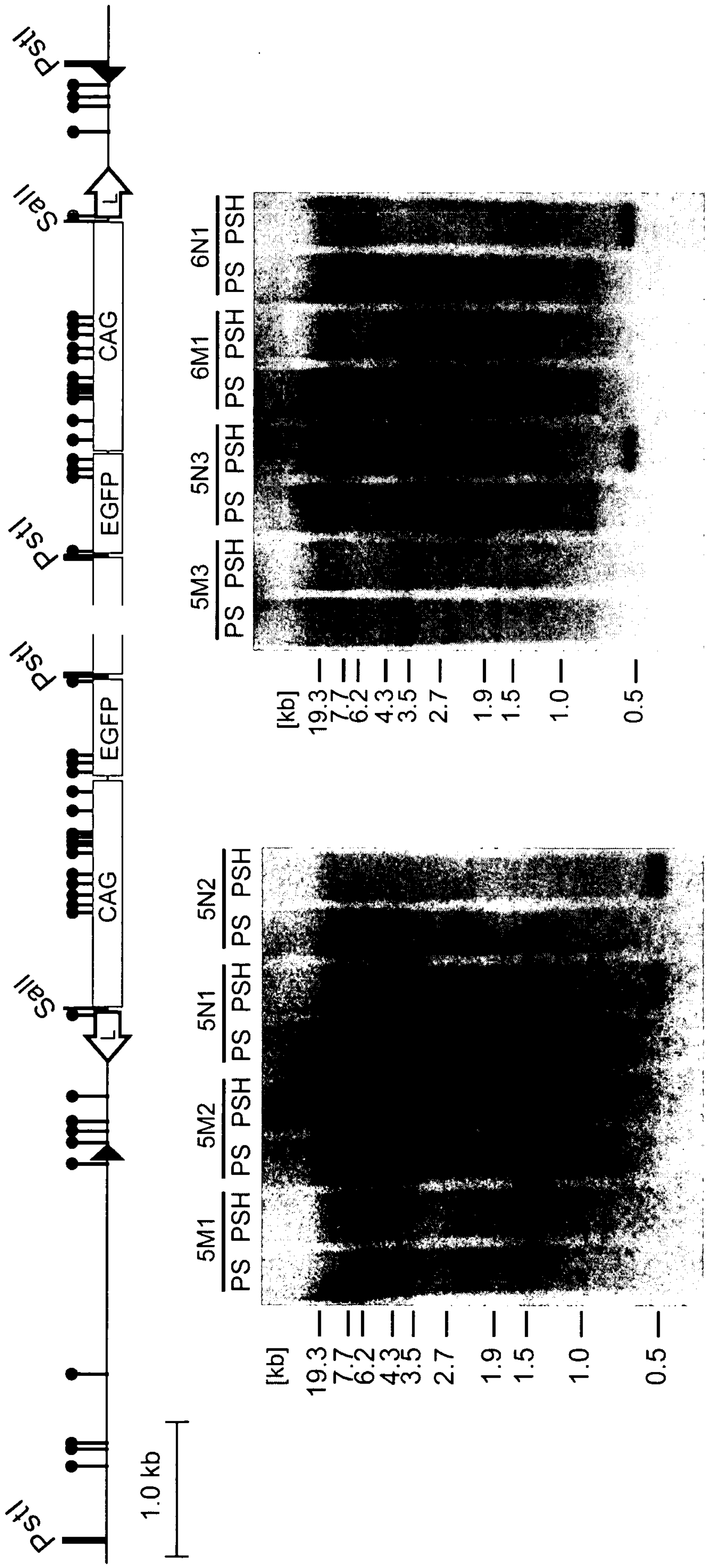
PstI
SalI
PstI
CAG
EGFP
L
1.0 kb
[kb]
19.3
7.7
6.2
4.3
3.5
2.7
1.9
1.5
1.0
0.5
5M1
5M2
5N1
5N2
5M3
5N3
6M1
6N1
PS
PSH

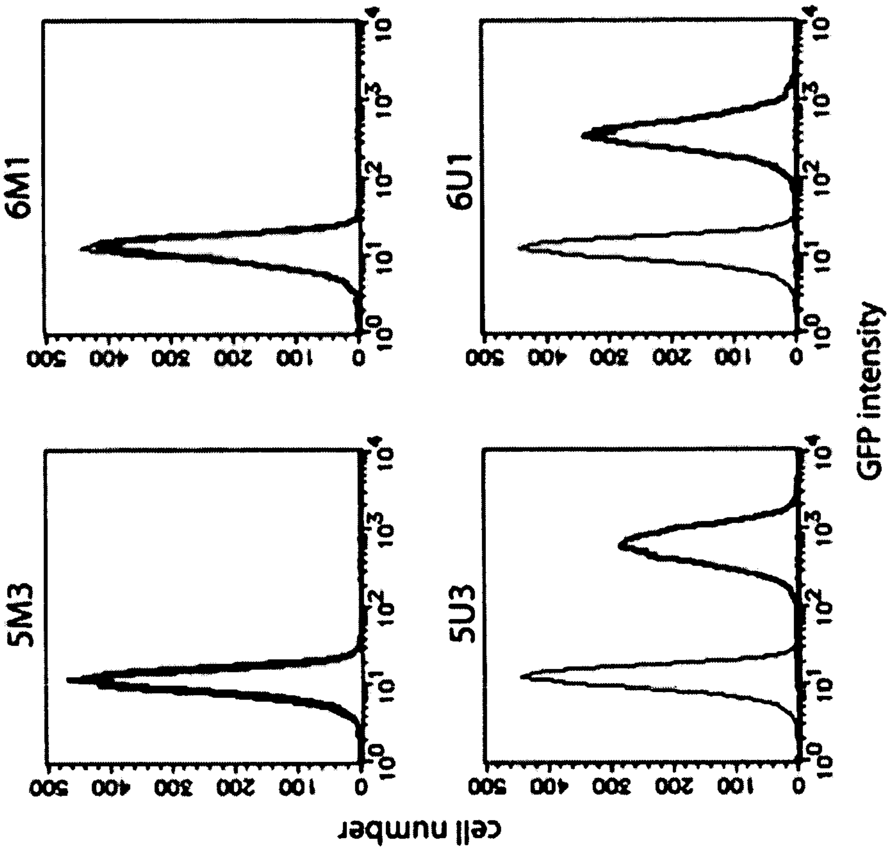


Fig. 2E

Fig. 3A

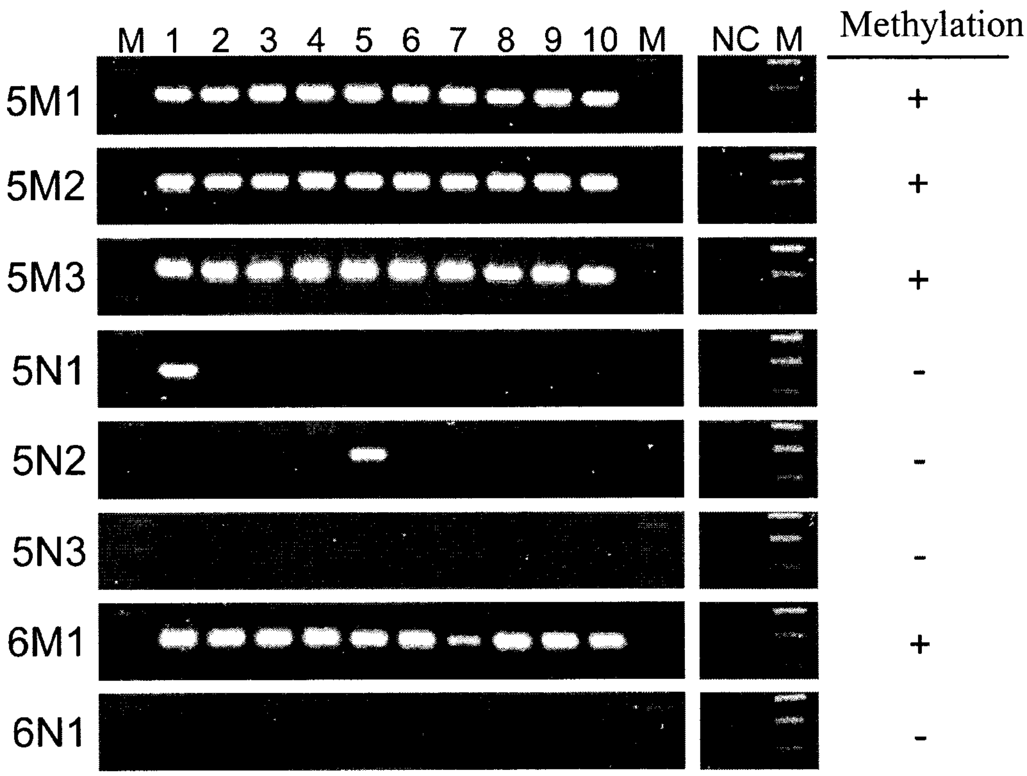
M 1 2 3 4 5 6 7 8 9 10 M
NC M
Methylation
5M1
5M2
5M3
5N1
5N2
5N3
6M1
6N1
+
+
+
-
-
-
+
-

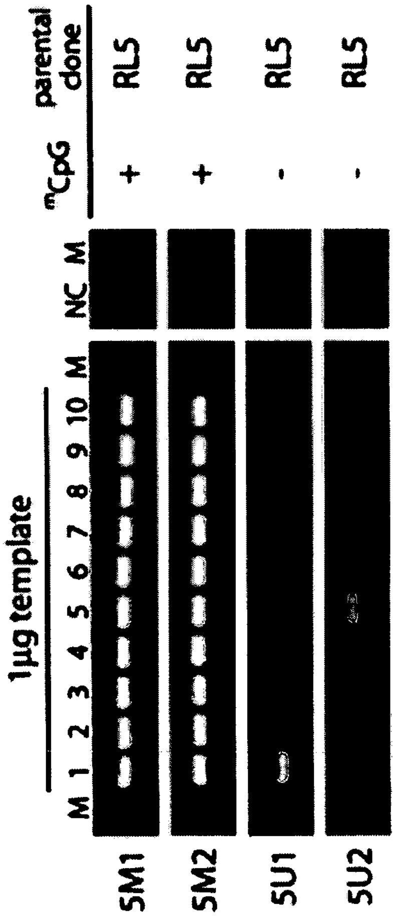
1µg template
M 1 2 3 4 5 6 7 8 9 10 M
NC M
mCpG
parental clone
5M1
5M2
5U1
5U2
+
+
-
-
RL5
RL5
RL5
RL5

Fig. 3B

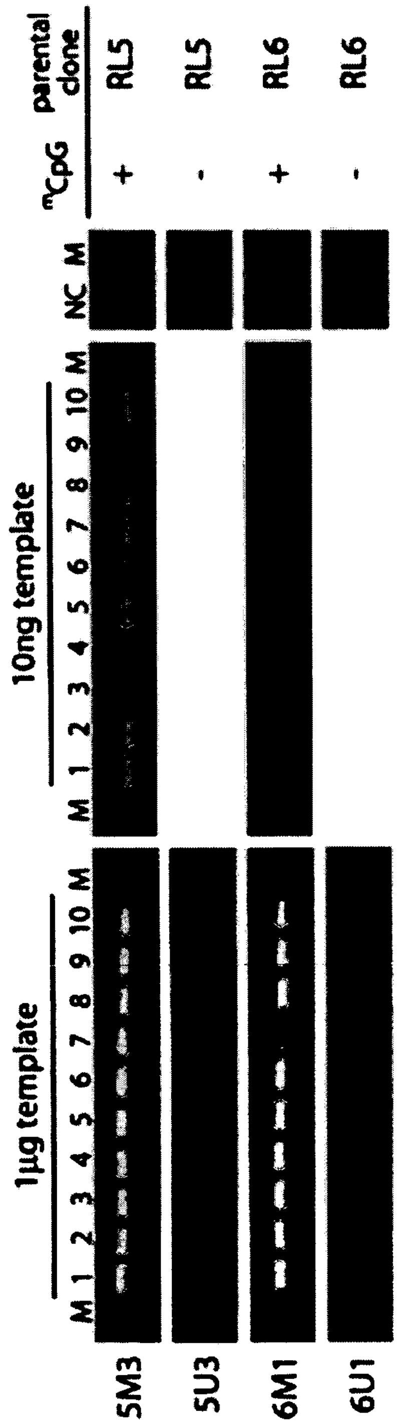
1µg template
10ng template
M 1 2 3 4 5 6 7 8 9 10 M
NC M
mCpG
parental clone
5M3
5U3
6M1
6U1
+
-
RL5
RL6

Fig. 3C

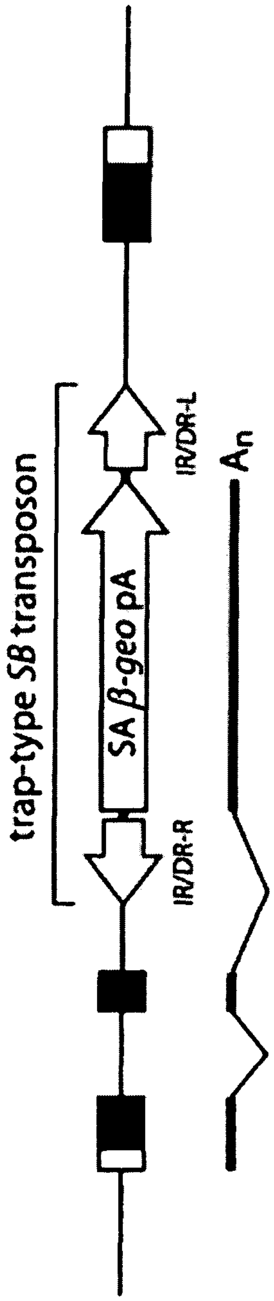


Fig. 4A

Fig. 4B

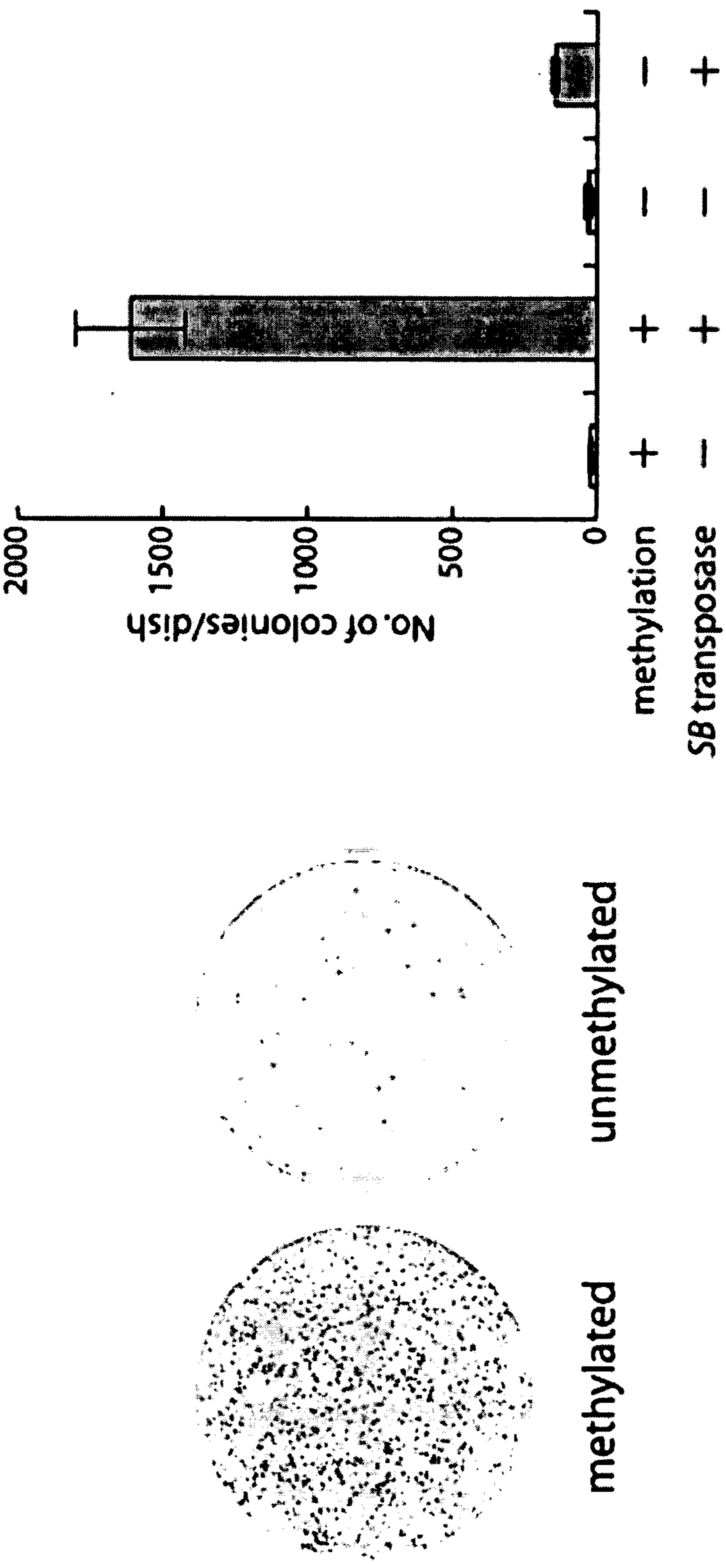
methylated
unmethylated
No. of colonies/dish
2000
1500
1000
500
0
methylation
SB transposase
+ + − −
− + − +

Fig. 4C

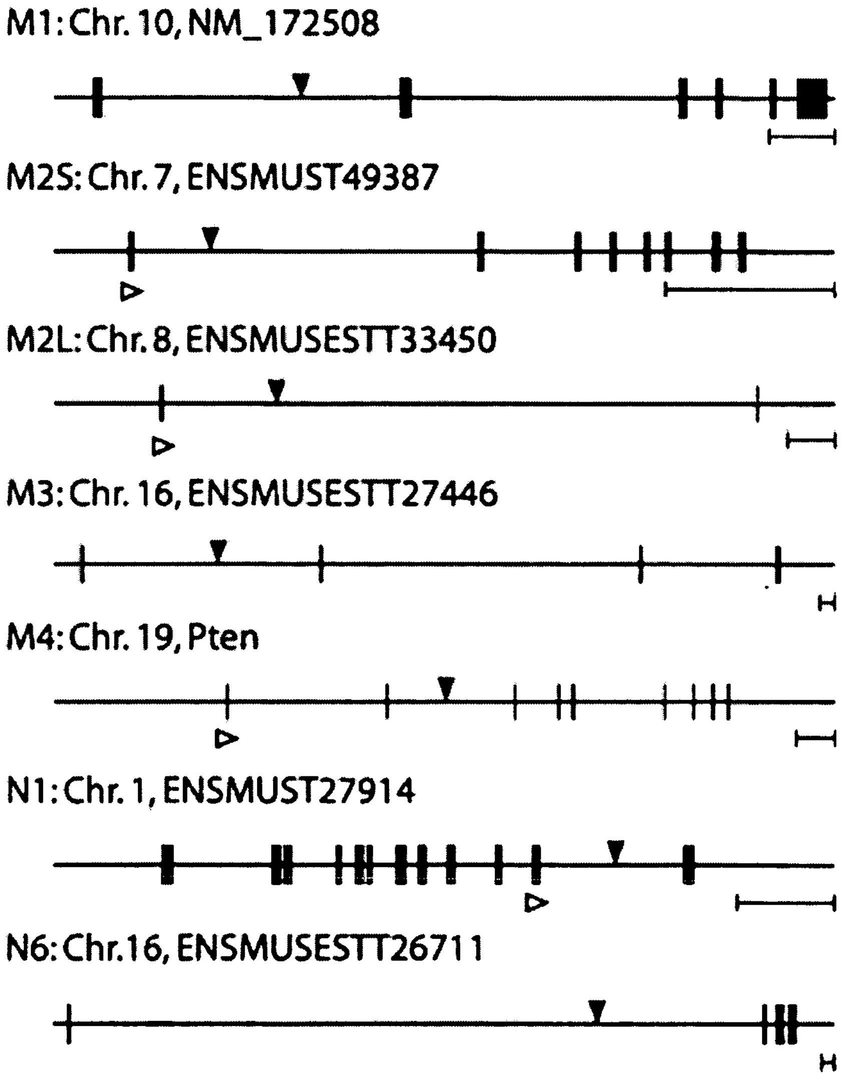
M1: Chr. 10, NM_172508
M2S: Chr. 7, ENSMUST49387
M2L: Chr. 8, ENSMUSESTT33450
M3: Chr. 16, ENSMUSESTT27446
M4: Chr. 19, Pten
N1: Chr. 1, ENSMUST27914
N6: Chr.16, ENSMUSESTT26711

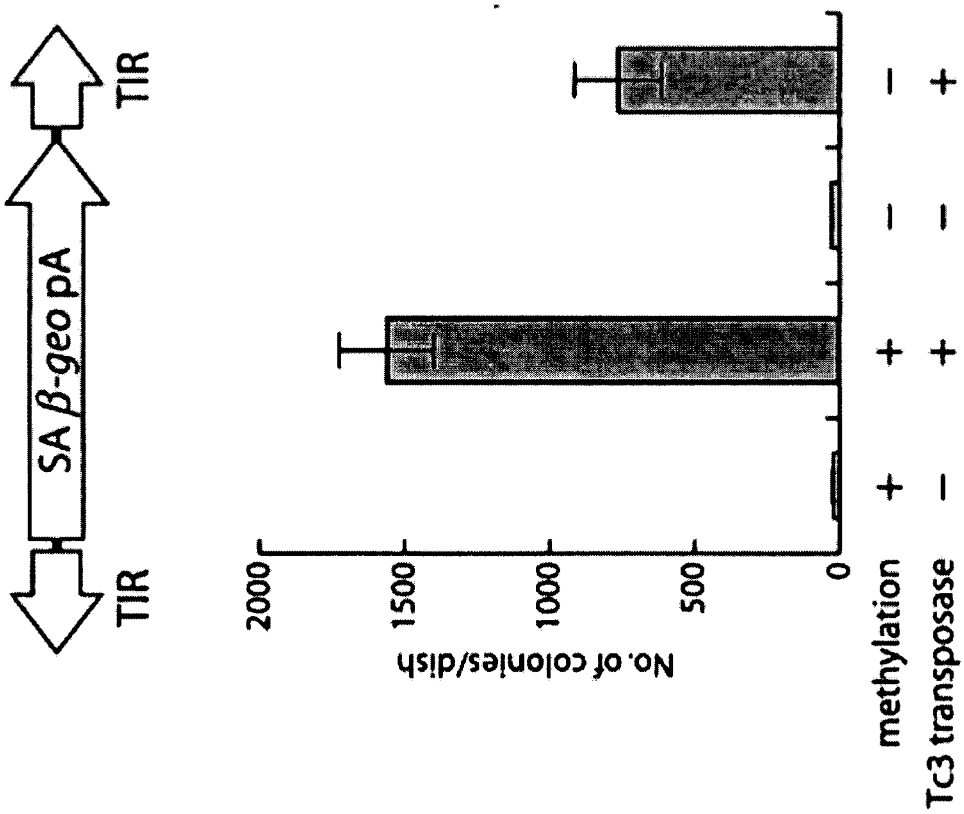
TIR
SA β-geo pA
TIR
No. of colonies/dish
2000
1500
1000
500
0
methylation
Tc3 transposase
+ − + + − − − +

Fig. 4D

Fig. 5A

```
CLUSTAL W (1.81) Multiple Sequence Alignments


Sequence type explicitly set to DNA
Sequence format is Pearson
Sequence 1: X01005          1610 bp
Sequence 2: Z29098          1773 bp
Sequence 3: Z29102          1717 bp
Sequence 4: U11641          1263 bp
Sequence 5: U11652          1296 bp
Sequence 6: L48685          1455 bp
Start of Pairwise alignments
Aligning...
Sequences (5:6) Aligned. Score:  3
Sequences (3:4) Aligned. Score:  3
Sequences (1:2) Aligned. Score:  9
Sequences (3:5) Aligned. Score:  3
Sequences (4:5) Aligned. Score:  95
Sequences (1:3) Aligned. Score:  9
Sequences (3:6) Aligned. Score:  10
Sequences (4:6) Aligned. Score:  3
Sequences (1:4) Aligned. Score:  1
Sequences (2:3) Aligned. Score:  99
Sequences (1:5) Aligned. Score:  3
Sequences (2:4) Aligned. Score:  3
Sequences (1:6) Aligned. Score:  2
Sequences (2:5) Aligned. Score:  3
Sequences (2:6) Aligned. Score:  10
Guide tree        file created:   [clustalw.dnd]
Start of Multiple Alignment
There are 5 groups
Aligning...
Group 1: Sequences:   2        Score:32613
Group 2:                       Delayed
Group 3:                       Delayed
Group 4: Sequences:   2        Score:22721
Group 5: Sequences:   4        Score:12095
Sequence:6      Score:13071
Sequence:1      Score:12960
Alignment Score 47622
CLUSTAL-Alignment file created  [clustalw.aln]
CLUSTAL W (1.81) multiple sequence alignment


Z29098          CGAGCCCCAACCACTATTAATTCGAACAGCATGTTTTTTTTGCAGTGCGCAATGTTTAAC
Z29102          --------------------------------------------------------TAAC
U11641          ------------------------------------------------------------
U11652          ------------------------------------------------------------
L48685          ------------------------------------------------------------
X01005          ------------------------------------------------------------

Z29098          ACACTATATTATCAATACTACTAAAGATAACACATACCAATGCATTTCGTCTCAAAGAGA
Z29102          ACACTATATTATCAATACTACTAAAGATAACACATACCAATGCATTTCGTCTCAAAGAGA
U11641          ------------------------------------------------------------
U11652          ------------------------------------------------------------
```

Fig. 5B

```
L48685    ——————————————————————————————————CAGTTGAAGTC—GGAAG
X01005    ——————————————————————————————————CAGTGCTGGCCAAAAAGA

Z29098    ATTTTATTCTCTTCACGACGAAAAAAAAAGTTTTGCTCTATTTCCAACAACAACAAAAAT
Z29102    ATTTTATTCTCTTCACGACGAAAAAAAAAGTTTTGCTCTATTTCCAACAACAACAAAAAT
U11641    ————————————————————————————————————————————————————————————
U11652    ————————————————————————————————————————————————————————————
L48685    TTTACATACACTTAAGTTGGAGTCATTAAA———ACTCGTTTTTCAACTACACCACAAAT
X01005    TATCCA—CTTTTGGTTTTTTGTGTGTAA————CTTTTTTCTCAAGCATCCATTTGAC

Z29098    ATGAGTAATTTATTCAAACGGTTTGCTTAAGAGATAAGAAAAAAGTGACCACTATTAATT
Z29102    ATGAGTAATTTATTCAAACGGTTTGCTTAAGAGATAAGAAAAAAGTGACCACTATTAATT
U11641    ———————————————————————————————————————————————————AACATGT
U11652    ———————————————————————————————————————————————————ATTAGGT
L48685    TTC—TTGTTAA—CAAACAAT————AGTTTTGGCAAGTCAGTTAGGACATCTACTT
X01005    TTG——AATTTTTCCGTGTGCATAAAGCGAAATGTTACGCAAATTTGCGGACCAA-ACAT
                                                                *  *

Z29098    CGAACGCGGCGTAAGCTTACCTTAATCTCAAGAAGAGCAAAACAAAAGCAACTAATGTAA
Z29102    CGAACGCGGCGTAAGCTTACCTTAATCTCAAGAAGAGCAAAACAAAAGCAACTAATGTAA
U11641    TGGCTG———ATAAGTCC-CCGGTTTGACAC————————TAGTATTAAATGCA-
U11652    TGGCTG———ATAAGTCC-CCGGTCTGACACATAGATGGCGTCGCTAGTATTAAATGCA-
L48685    TGTGCATGACACAAGT———CATTTTTCCAACAATTGT——TTACAGACAGATTATTTCA
X01005    TACATGATTATCGATTTTTTCTGAATTTTATTTCAATTTT-TTGATTTTTTCGTTTTTCC
                      *     *    *   *                       *

Z29098    CGGAATCATTATCTAGTTATGATCTGCAAATAAT———GTCACAATACAGCATGCAAAAA
Z29102    CGGAATCATTATCTAGTTATGATCTGCAAATAAT———GTCACAATACAGCATGCAAAAA
U11641    TATTATTTTTATATAGGACCAACCTTCAAATGATTCGTGTCAAAATTTGACGTC———
U11652    TATTATTTTTATATAGTACCAACCTTCAAATGATTCGTGTCAAAATTTGACGTCTGTAAG
L48685    CTTATAATTCACTGTATCACAATT—CCAGTGG———GTCAGAAGTTTACATACA———
X01005    AATTTTCATTATTTTTTTTGAATTATCAATAAAACGCACTCTGTTTGTTGCACTGG——A
                * *        *   * *        **      *

Z29098    AATTTTAGATTGCTGCA—GATCAGTAGAAGTTTAGCAACGATGGTTCGTGGTAAACCTA
Z29102    AATTTTAGATTGCTGCA—GATCAGTAGAAGTTTAGCAACGATGGTTCGTGGTAAACCTA
U11641    —AATTAGTTTGTGAGA——————————GCAACTTTTGTTATTGTGAAGAAAA
U11652    TCAATTAGTTTGTGAGATAGAGCGTCTTTTGTGAAGCAACTTTTGTTATTGTGAAAAAAA
L48685    ——CTAAGTTGACTGTG———CCTTTAAA———CAGCTTGGAAAATTCCAG—AAAATGA
X01005    TTTGTTTGGTTGATAAAT—TATTTTTAAGGTATGGTAAAATCTGTTGGGTGTAAAAATC
            *  * *                    *        **     **

Z29098    TTTCTAAAG——————AAATCAGAGTATTGATTAGGGATTATTTTAAATCTGGAAAG
Z29102    TTTCTAAAG——————AAATCAGAGTATTGATTAGGGATTATTTTAAATCTGGAAAG
U11641    TGGAAAAAATTTCATTTCGAATTTCGTGTTTTGATAAAATACTGTTTTCTGAAGGGAAAA
U11652    TGGAAAAAA————AGGAATTTCGTGTTTTGATAAAATACTGTTTTCTGAAGGGAAAA
L48685    TGTCATGGC———————TTTAGAAGCTTC-TGATAGACTAATTGACATCATTTGAG
X01005    TTTCCTTGG——————ACGTCAAGAAAGCCATTGTAG-CTGGCTTCGAACAAGGAAT
          *               *        *      *  *        *

Z29098    ACACTTACGGAGATAAGCAAGCAATTAAATTTGCCTAAGTCGTCTGTGCATGGGGTGAT-
Z29102    ACACTTACGGAGATAAGCAAGCAATTAAATTTGCCTAAGTCGTCTGTGCATGGGGTGAT-
U11641    AA——TGCGGTGG-AAGCAAAAAGTTGGCTTGATAATGAGTTTCCGGACTCTGCCCCAA-
U11652    AA——TACAGTGG-AAGCAAAAACTTGGCTTGATAATGAGTTTCCGGACTCTGCCCCAG-
L48685    TCAATT—GGAGGTGTACCTGTGGATGTATTT——CAAGGCCTACCTTCA-AACGCAGT-
X01005    ACCCACGAAAAGCTCGCGCTGCAAATTCAACGTTCTCCGTCGACTATTTGGAAAGTAATC
```

Fig. 5C

```
                     *             *
Z29098    ACAAATTTTCAAAAAA-AATGGGAATATTGAAAATAA-CA--TTGCGAATAGAGGCCGAA
Z29102    ACAAATTTTCAAAAAA-AATGGGAATATTGAAAATAA-CA--TTGCGAATAGAGGCCGAA
U11641    GGAAATCAATAATAATTGATTGGTATGCAAAATTCAAGCG--AGGTGAAATGAGCACGGA
U11652    GGAAATCAACAATAATTGATTGGTATGCAAAATTCAAGCG--TGGTGAAATGAGCACGGA
L48685    GCCTCTTTGCTTGACATAATGGGAAAATCAAAAGAAATCAGCCAACACCATGGGACCACG
X01005    AAGAAGTACCAAACTGAGGTGAGTTCGAAAAATATTATTTTTTAATAATAAATGTTTAGA
                             *  *       **    *                *

Z29098    CATCAGCAA---TAACACCCCGCGACAAAAGACAA-CTGGCCAAAATTGTTAAGGCTGAT
Z29102    CATCAGCAA---TAACACCCCGCGACAAAAGACAA-CTGGCCAAAATTGTTAAGGCTGAT
U11641    GGACGGTGA---ACGCAGTGGACGCCCGAAAG-AG-GTGGTTACCGACGAAAA-------
U11652    GGACGGTGA---ACGCAGTGGACGCCCGAAAG-AG-GTGGTTACCGACGAAAA-------
L48685    CAGCCGTCA---TACCGCTCAGGAATGAGACGCATTCTGTCTCCTAGAGATAAA------
X01005    AATCCGTCGCTTTGAGAATCTCGCCCGGCAGGCCT-CGAGTGACAACCCATAGGATGGAT
             * *                       *                     *

Z29098    CGTCGCCAATCTTTGAGAAATTTGGCTTCTAAGTGGTCGCA----GCAATTGGCAAAACT
Z29102    CGTCGCCAATCTTTGAGAAATTTGGCTTCTAAGTGGTCGCA----GCAATTGGCAAAACT
U11641    CATCAAAAAAATCCACAAAAT---GATTTTGAATGACCGTAAAATGAAGTTGATCGAGAT
U11652    CATCAAAAAAATCCACAAAAT---GATTTTGAATGACCGTAAAATGAAGTTGATCGAGAT
L48685    CAT-ACTGTGGTGCGAAAAGT------GCAAATCAATCCCAGAACGACAGCAAAGGACCT
X01005    CGC-AACATCCTCCGATCAGCA-----AGAGAAGATCCGCATAG-GACCGCCACGGATAT
          *          *      *            *     *  *    *          *  *

Z29098    GTCAAGCGAGAGTGGACGCGACAAATTAAAAAGTAT---TGGATATGGTTTTTATAAAGT
Z29102    GTCAAGCGAGAGTGGACGCGACAAATTAAAAAGTAT---TGGATATGGTTTTTATAAAGT
U11641    AACAAA-GGCCTTAAACATATCAAA--GGAACGTGT---TGGTCATATCATTCATCAA--
U11652    AGCAGA-GGCCTTAAAGATATCAAA--GGAACGTGT---TGGTCATATCATTCATCAA--
L48685    TGTGAA-GATGCTGGAGAAAACAGGTATGAATGTTTCTATATCCACAGTAAAAACGAGTC
X01005    -TCAAATGATTATAAGTTCTCCAAATGAACCTGTAC---CAAGTAAACGAACTGTTCGTC
                 *    *        **        **           *

Z29098    ATGTTTTGTTATTACCTGTGCATCGTACCCAATAACTTACTCGTAATCTTACTCGTAGGC
Z29102    ATGTTTTGTTATTACCTGTGCATCGTACCCAATAACTTACTCGTAATCTTACTCGTAGGC
U11641    -TATTTGGATAT-GCGGAAGCTCTGTGCAAAATGGGTGCCGCGCGAACTCACAT-TTGAC
U11652    -TATTTGGATAT-GCGGAAGCTCTGTGCAAAATGGGTGCCGCGCGAGCTCACAT-TTGAC
L48685    CTATATCGACATAACCTGAAAGGC-CGCTCAGCAAGGAAGAAGCCA--CTGCTCCAAAAC
X01005    GACGTTTACAGCAAGCAGGACTACACGGACGA-AAGCCAGTCAAGAAACCGTTCATCAGT
               *                                       *

Z29098    CAAGG-AAAAACCCTTGCTTACGCTTCGTCAAAAAAAGAAGCGTTTGCAATGGG--CTCG
Z29102    CAAGG-AAAAACCCTTGCTTACGCTTCGTCAAAAAAAGAAGCGTTTGCAATGGG--CTCG
U11641    CAAAA-ACAACAACGTGTTGATGATTCT-------GAGCGGTGTTTGCAGCTGT--TAAC
U11652    CAAAA-ACAACAACGTGTTGATGATTCT-------GAGCGGTGTTTGCAGCTGT--TAAC
L48685    CGCCATAAAAAAGCCAGACTACGGTTTGCAACTGCACATGGGGACAAATATGGTACTTTT
X01005    AAGAA-AAATCGCATGGCTCGAGTTGCGTGGGCAAAAGC-GCATCTTCGTTGGGGACGTC
                * *       *     * *               *           *

Z29098    GGAAAGGATGTCTTGGACTCAAAGGCAATGGGATACCATCATATTCAGCGATGAAGCTAA
Z29102    GGAAAGGATGTCTTGGACTCAAAGGCAATGGGATACCATCATATTCAGCGATGAAGCTAA
U11641    TCGTAATACACCCGAGTTTTTCCGTCGATATG-TAACAATGGATGAAACATGGCTCCATC
U11652    TCGTAATACACCCGAGTTTTTGCGTCGATATG-TGACAATGGATGAAACATGGCTCCATC
L48685    TGGAGAAATGTCCTCTCTTCTGGTCTGATGAA----AAAAAAATAGAACTATTTGGCCAT
X01005    AGGAATGGGCTAAACACATCTGGTCTGACGAA----AGCAAGTTCAATTTGTTCGGGAGT
                            *        *               *  *
```

Fig. 5D

```
Z29098    ATTTGATGTTAGTGTCGGCGATACGAGAAAACGCGTCATCCGTAAGAGGTCAGAAACATA
Z29102    ATTTGATGTTAGTGTCGGCGATACGAGAAAACGCGTCATCCGTAAGAGGTCAGAAACATA
U11641    ACTACACTCCTGAGTTCGATCAACAGTCGGCTGAGTGGACAGCGACCGGT--GAACCGTC
U11652    ACTACACTCCTGAGTCCAAACGACAGTCGGCTGAGTGGACAGCGACCGGT--GAACCGTC
L48685    AATGACCATCGTTAT--GTTTGGAGGAAAAAGGGGGAGCTTGCAAGCCG----AAGATCA
X01005    GATGGAAATTCCTG-------GGTACGTCGTCCTGTTGGCTCTAGGTACTCTCCAAAGTA
            *                         *                *

Z29098    CCATAAAGACTGCCTTAAAAGAACAACAAAGTTTCCTGCGAGCACTATGGTATGGGGATG
Z29102    CCATAAAGACTGCCTTAAAAGAACAACAAAGTTTCCTGCGAGCACTATGGTATGGGGATG
U11641    TCCGAAG------CGTGGAAAGACTCAAAAGTCCGCTGGCAAAGTAATGGCCTCTGTTTT
U11652    TCCGAAG------CGTGGAAAGACTCAAAAGTCCGCTGGCAAAGTAATGGCCTCTGTTTT
L48685    CCATC-------CCAAGCGTGAAGCACGGGGG----TGGCAGCATCATGTTGTGGGGGTG
X01005    TCAATGC-----CCAACCGTTAAGCATGGAGG----TGGGAGCGTCATGGTGTGGGGGTG
           *        *      *     *     ** *    ***  *  *  *

Z29098    TATGTCTGCCAAAGGATTAGGAAAACTTCATTTCATTGAAGGGACAGTTAATGCTGAAAA
Z29102    TATGTCTGCCAAAGGATTAGGAAAACTTCATTTCATTGAAGGGACAGTTAATGCTGAAAA
U11641    TTGGAATGCGCATGGAATAATTTTTATCGATTATCTTGAGAAGGAAAAAACCATCAACAG
U11652    TTTCGATGCGCATGGAATAATTTTTATCGATTATCTTGAGAAGGGAAAAACCATCAACAG
L48685    CTTTGCTGCAGGAGGGACTGGTGCACTTCACAAAATAGATGGCATCATGACAAAGGAAAA
X01005    CTTCACCAGCACTTCCATGGGCCCACTAAGGAGAATCCAAAGCATTATGGATCGTTTTCA
                                   *      * *

Z29098    ATATATTAATATTTTACAAGATAGTTTGTTGCCATCAATACCAAAACTATTAGATTGCGG
Z29102    ATATATTAATATTTTACAAGATAGTTTGTTGCCATCAATACCAAAACTATCAGATTGCGG
U11641    -----TGACTATTATATGGCGTTATTGTAGCGTTTGAAGGTCGAAATCGCGGCAAAATGG
U11652    -----TGACTATTATATGGCGTTATTGGAGCGTTTGAAGGTCGAAATCGCGGCAAAACGG
L48685    TTATGTGGCTATATTGAAGCAACATCTCAAGACATCAGTCAGGAAGTTCAAGCTTGGTCA
X01005    ATACGAAAACATCTTTGAAACTACAATGCGACCCTGGGCACTTCAAAATGTGGGCCGTGG
                **  *                 *         *

Z29098    TGAATTCACTTTTCAGCAGGACGGAGCATCATCGCAC----ACAGCCAAGCGAACCAAAA
Z29102    TGAATTCACTTTTCAGCAGGACGGAGCATCATCGCAC----ACAGCCAAGCGAACCAAAA
U11641    ----CCCCATATGAAGAAGAAAAAAGTGTTGTTCGACCAAGACAATGCACCGTGCCACAA
U11652    ----CCCCATATGAAGAAGAAAAAAGTGTTGTTCCACCAAGACAACGCACCGTGCCACAA
L48685    CAAATGGGTCTTCCAAATGGACAATGACCTCAAGCAT----ACTTCCAAAGTTGTGGCAA
X01005    C---TTCGTGTTTCAGCAGGATAACGATCCTAAGCAT----ACTTCTCTTCATGTGCGTT
                  *  *   * *    *        *   **

Z29098    ATTGGCTGCAATATAATCAAATGGAGGTTTTAGATTGGCCATCAAATAGTCCAGATCTAA
Z29102    ATTGGCTGCAATATAATCAAATGGAGGTTTTAGATTGGCCATCAAATAGTCCAGATCTAA
U11641    GTCAGTAAGAACGATGGCAAA---AATTCATGAATTGGGCTTCGAATTGCTTCCCCACCC
U11652    GTCATTGAGAACGATGGCAAA---AATTCATGAATTGGGCTTCGAATTGCTTCCCCACCC
L48685    AATGGCTTAAGGTCAACAAAGTCAAGGTATTGGAGTGGCCATCACAAAGCTCTGACCTCA
X01005    CATGGTTTCAACGTCGTCATGTGCATTTGCTCGATTGGCCAAGTCAGTCTCCGGACTTGA
                *         *     *  *  *  * *** *      *

Z29098    GCCCAATTGAAAATATTTGGTGGCTAATGAAAAACCAGCTT--CGAAAT--GAGCC-ACA
Z29102    GCCCAATTGAAAATATTTGGTGGCTAATGAAAAACCAGCTT--CGAAAT--GAGCC-ACA
U11641    ACTATATTCTCCAGATCTGGCCCCCAGCGAATTTTTCTTGT--TCTCA---GACCT-CAA
U11652    ACCGTATTCTCCAGATCTGGCCCCCAGCGACTTTTTCTTGT--TCTCA---GACCT-CAA
L48685    ATCCTATAGAAAGGAGGAATGAGCCAAAATTCACCCAACTTATTGTGGG--AAGCTTGTG
X01005    ATCCAATAGAGCATTTGTGGGAAGAGTTGGAAAGACGTCTTGGAGGTATTCGGGCT-TCA
             **                                    *              *

Z29098    AAGGAATATTTCTGACTTGAAAATCAAGTTGCAAGAGATGTGGGACTCAATTTCTCAAGA
Z29102    AAGGAATATTTCTGACTTGAAAATCAAGTTGCAAGAGATGTGGGACTCAATTTCTCAAGA
```

Fig. 5E

```
U11641   AAGGGATGCTCGCAGGGAAAAAATTTGGCTGCAATGAA——————————————GAGG
U11652   AAGG-ATGCTCGCAGGGAAAAAATTTGGCTGCAATGAA——————————————GAGG
L48685   GAAGGCTACTCGAAATGTTTGACCCAAGTTAAACAATTT——————————————AAAG
X01005   AATGCAGATGCCAAATTC——AACCAGTTGGAAAACGCTTGGAAAGCTATCCCCATGTCA
          * *                    *          *

Z29098   GCATTGCAAAAAATTTGTTAAGCTCAATGCCAAAACGAGTTAAATGCGTAATGCAGGCCAA
Z29102   GCATTGCAAAAAATTTGTTAAGCTCAATGCCAAAACGAGTTAAATGCGTAATGCAGGCCAA
U11641   TAATCGCCGAAAC——TAAGGCCTATTTTGAGGCAAAACCGTAAGAGTACTA————CCA
U11652   TGATCGCCGAAAC——TGAGGCCTATTTTGAGGCAAAACCGAAGGAGTACTA————CCA
L48685   GCAATGCTA————CCAAATACTAATTGAGTGTATGTTAACTTC-TGACCCA—CTGG
X01005   GTTATTCACAAGCTGATCGA-CTCGATGCCACGTCGTTGTCAAGCTGTTATTGATGCAAA
             *                      *                *

Z29098   GGGCGACGTTACACAATTCTAATATTAATTAAATTATTGTTTTAAGTATGATAGTAAATC
Z29102   GGGCGACGTTACACAATTCTAATATTAATTAAATTATTGTTTTAAGTATGATAGTAAATC
U11641   AAATGGTATCAAAAAATTGGAAGGTCGTTATAATCGTGGTATCGCTCTTGA-AGGGGACT
U11652   AAATGGTATCAAAAAATTGGAAGGTCGTTATAATCGTTGTATCGCTCTTGA-AGGGAACT
L48685   GAATGTGATGAAAGAAATAAAAGCTGAAATGAATCATTCTCTCTACTATTATTCTG———
X01005   CGGATACGCGACAAAGTATTAAGCATAATTATGTTGT—TTTTAAATCCAATTGC——TC
                  * * *    **        *  *  * *       *

Z29098   ACATTACGCCGCGTTCGAATTAATAGTGGTCACTTTTTTCTTATCTCTTAAGCAAACCGT
Z29102   ACATTACGCCGCGTTCGAATTAATAGTGGTCACTTTTTTCTTATCTCTTAAGCAAACCGT
U11641   ATGTTGAATAATAA—AAACGAATTTTGACAAAAAA-TGTGTTTTTCTTTGTTAGACCGG
U11652   ATGTTGAATAATAA—AAACGAATTTTCACAAAAAAATGTGTTTTTCTTTGTTAGACCGG
L48685   ATATTTCACATTCTTAAAATAAA—GTGGTGA—TCCTAACTGACCTTAAGACAGGGAAT
X01005   ATATTCCGGTACTT————TAATTGTCATTTCCTTGCAACCTCGGTTTTTTCAATATTT
         *  **                **   *                   *    *

Z29098   TTGAATAAATTACTCATATTTTTGTTGTTGTTGGAAATAGAGCAAAACTTTTTTTTTCGT
Z29102   TTGAATAAATTACTCATATTTTTGTTGTTGTTGGAAATAGAGCAAAACTTTTTTTTTCGT
U11641   —GGACTTATCACCCAACCTGTTA————————————————————————————
U11652   —GGACTTATCAGCCAACCTGTTA————————————————————————————
L48685   C—TTTACTCGGATTAAATGTCAGGAATTGTGAAAAAGTGAGTTTAAATGTATTTG—GC
X01005   C———TAGTTTTTCGATTTTTTTGAATTTTTCTGAAGTTTTTTCAAAATCTGTTGAACAT
                        *

Z29098   CGTGAAGAGAATAAAATTCTCTTTGAGACGAAATGCATTGGTATGTGTTATCTTTAGTAG
Z29102   CGTGAAGAGAATAAAATTCTCTTTGAGACGAAATGCATTGGTATGTGTTATCTTTAGTAG
U11641   ————————————————————————————————————————————
U11652   ————————————————————————————————————————————
L48685   TAAGGTGTATGTAAACTTCCGACTTCAACTG—————————————————————
X01005   TTTTG——ATGAATATTGTGTTTTTAGATTTTGTGAACACTGTGGTGAAGTTTCAAAACA

Z29098   TATTGATAATATAGTGTGTTAAACATTGCGCACTGCAAAAAAAAACATGCTGTTCGAATTA
Z29102   TATTGATAATATAGTGTGTTAAACATTGCGCACTGCAAAAAAAAACATGCTGTTCGAATTA
U11641   ————————————————————————————————————————————
U11652   ————————————————————————————————————————————
L48685   ————————————————————————————————————————————
X01005   AAATAACCACTTAGAAAAAAGTTACACACAAAAAACCAAAAGTGGATATCTTTTTGGCCA

Z29098   ATAGTGGTTGGGGCTCG
Z29102   ATAGTGGTTGGGGCTCG
U11641   —————————————
U11652   —————————————
```

Fig. 5F

```
L48685          ------------------
X01005          GCACTG------------

(
(
X01005:0.47463,
(
U11641:0.02397,
U11652:0.01879)
:0.47911)
:0.01531,
(
Z29098:0.00029,
Z29102:0.00029)
:0.42978,
L48685:0.46683);
```

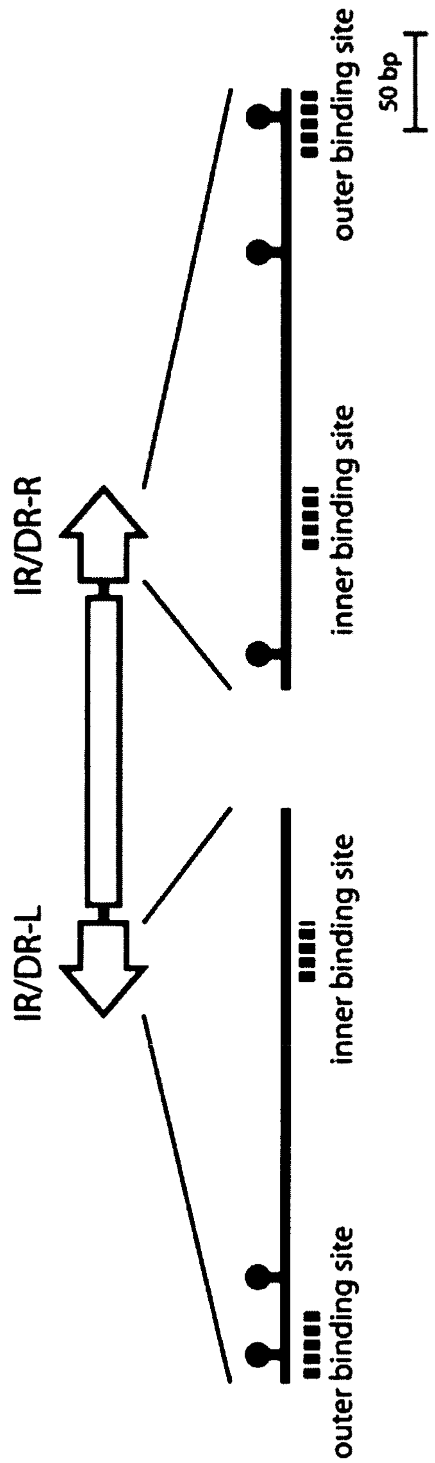
IR/DR-L
IR/DR-R
outer binding site
inner binding site
inner binding site
outer binding site
50 bp

Fig. 6A

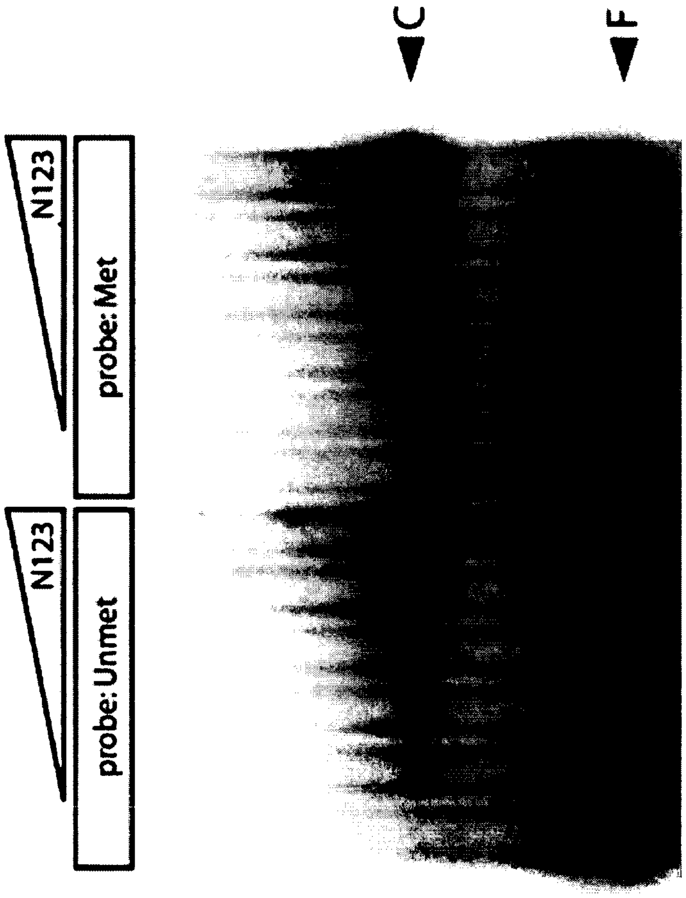


Fig. 6B

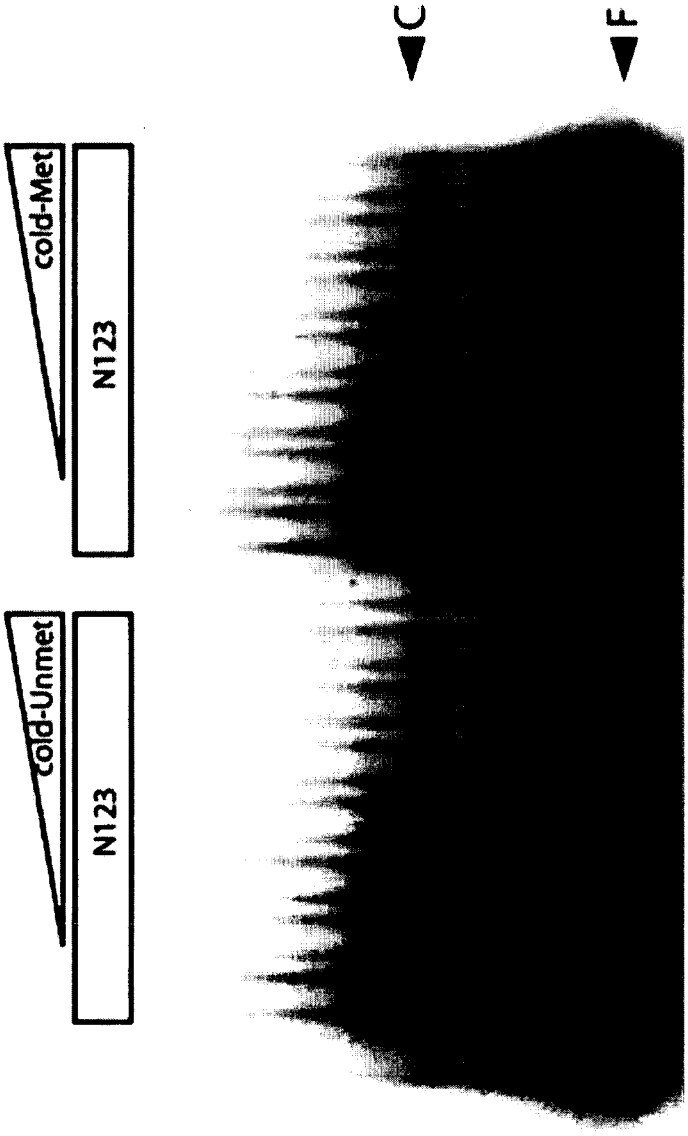


Fig. 6C

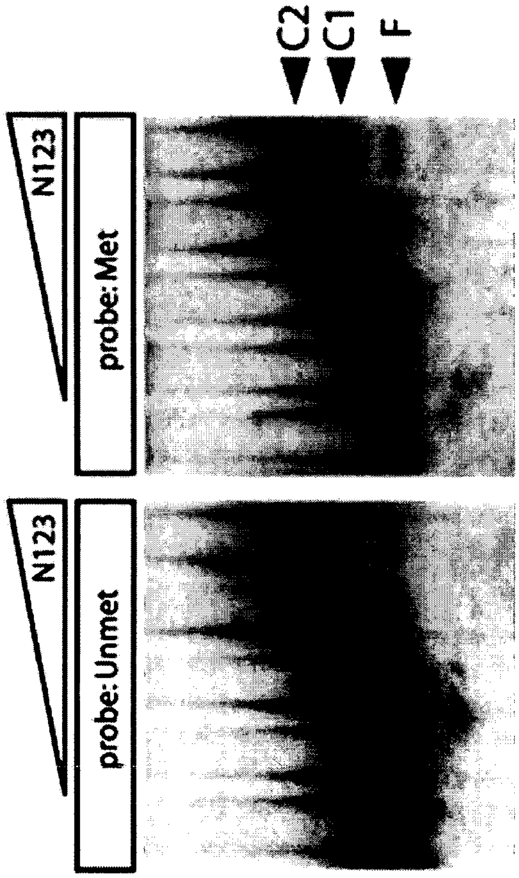


Fig. 6D

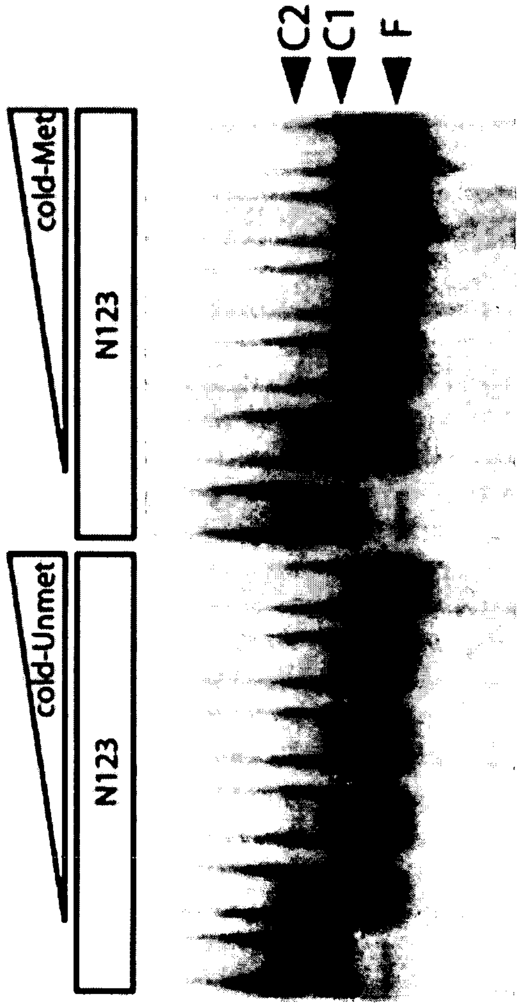


Fig. 6E

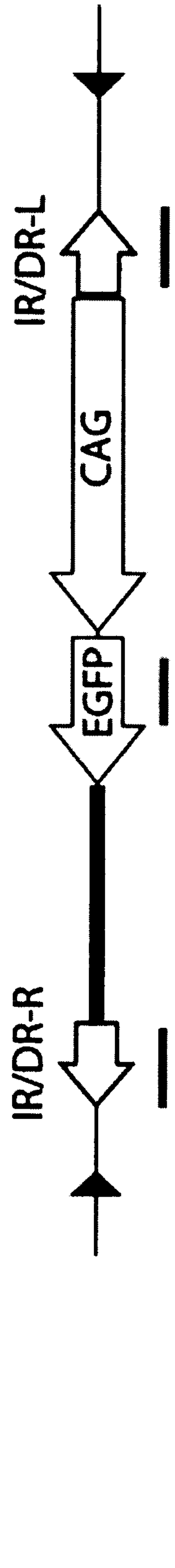


Fig. 7A

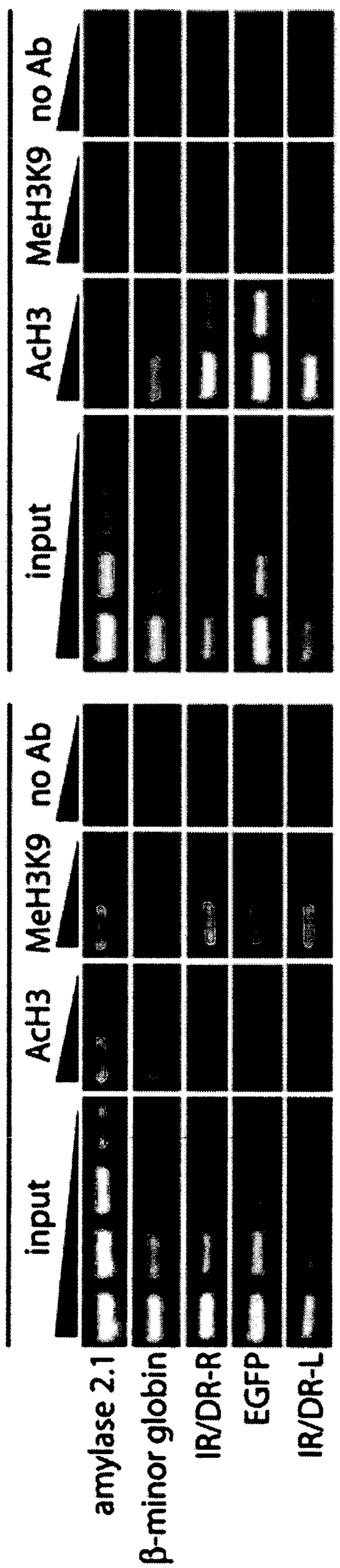
5M3
5U3
input
AcH3
MeH3K9
no Ab
amylase 2.1
β-minor globin
IR/DR-R
EGFP
IR/DR-L

Fig. 7B

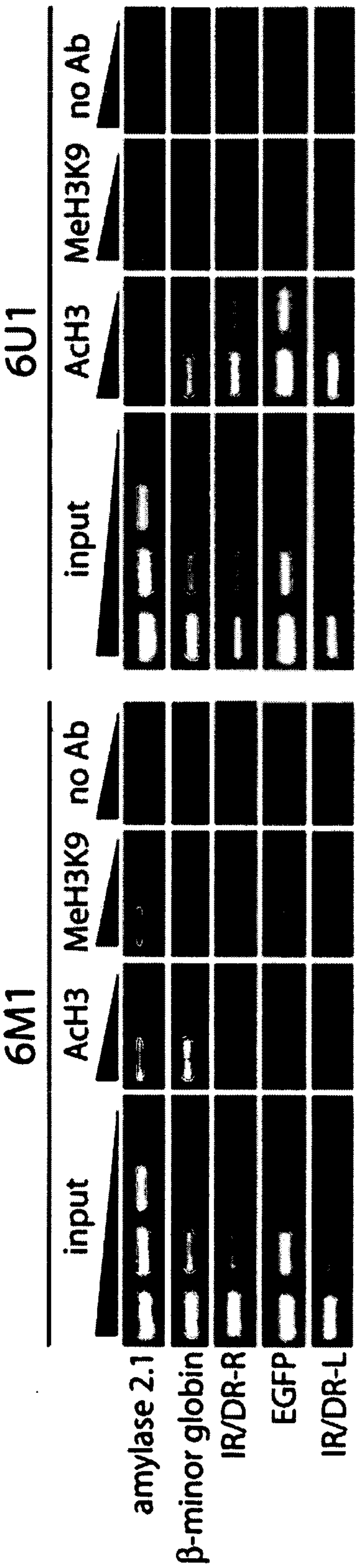
6M1
6U1
input
AcH3
MeH3K9
no Ab
amylase 2.1
β-minor globin
IR/DR-R
EGFP
IR/DR-L

Fig. 7C

**INTERNATIONAL SEARCH REPORT**

International application No. PCT/JP2004/010090

A. CLASSIFICATION OF SUBJECT MATTER
Int.Cl[7] C12N15/09, C12N5/10, A01K67/027

According to International Patent Classification (IPC) or to both national classification and IPC

B. FIELDS SEARCHED

Minimum documentation searched (classification system followed by classification symbols)
Int.Cl[7] C12N15/09, C12N5/10, A01K67/027

Documentation searched other than minimum documentation to the extent that such documents are included in the fields searched

Electronic data base consulted during the international search (name of data base and, where practicable, search terms used)
BIOSIS/WPI(DIALOG), MEDLINE(STN), JSTPlus/JST7580(JOIS)

C. DOCUMENTS CONSIDERED TO BE RELEVANT

| Category* | Citation of document, with indication, where appropriate, of the relevant passages | Relevant to claim No. |
|---|---|---|
| X/A | Fischer S.E. et al., Regulated transposition of a fish transposon in the mouse germ line, Proc.Natl.Acad.Sci.USA, 2001, Vol.98, No.12, pages 6759 to 6764 | 26-55 /1-25,57-106 |
| X/A | Horie K. et al., Efficient chromosomal transposition of a Tc1/mariner- like transposon Sleeping Beauty in mice, Proc.Natl.Acad.Sci.USA, 2001, Vol.98, No.16, pages 9191 to 9196 | 26-55 /1-25,57-106 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 August, 2004 (25.08.04) | 14 September, 2004 (14.09.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/010090

C (Continuation). DOCUMENTS CONSIDERED TO BE RELEVANT

| Category* | Citation of document, with indication, where appropriate, of the relevant passages | Relevant to claim No. |
|---|---|---|
| A | Luo G. et al., Chromosomal transposition of a Tc1/mariner-like element in mouse embryonic stem cells, Proc.Natl.Acad.Sci.USA, 1998, Vol.95, No.18, pages 10769 to 10773 | 1-106 |
| A | Lee S.Y. et al., Efficient Tn10 transposition into a DNA insertion hot spot in vivo requires the 5-methyl groups of symmetrically disposed thymines within the hot-spot consensus sequence, Proc.Natl.Acad.Sci.USA, 1987, Vol.84, No.22, pages 7876 to 7880 | 1-106 |
| A | Woodcock D.M. et al., RglB facilitated cloning of highly methylated eukaryotic DNA: the human L1 transposon, plant DNA, and DNA methylated in vitro with human DNA methyltransferase, Nucleic Acids Res, 1988, Vol.16, No.10, pages 4465 to 4482 | 1-106 |
| P,X | Yusa K. et al., Enhancement of Sleeping Beauty transposition by CpG methylation: possible role of heterochromatin formation, Mol.Cell Biol., 2004 May, Vol.24, No.9, pages 4004 to 4018 | 1-106 |

Form PCT/ISA/210 (continuation of second sheet) (January 2004)

SEQUENCE LISTING

<110> JAPAN SCIENCE AND TECHNOLOGY AGENCY
TAKEDA, Junji
HORIE, Kyoji

<120> Method and sysytem for producing transgenic organisms using methylation

<130> KJ007PCT

<150> PCT/JP03/08681
<151> 2003-07-08

<160> 76

<170> PatentIn version 3.2

<210> 1
<211> 1455
<212> DNA
<213> Tanichthys albonubes

<220>
<221> misc_feature
<222> (1)..(1455)
<223> /note="Tc1-like transposon"

<300>
<308> L48685
<309> 1996-05-31
<313> (1)..(1455)

<400> 1
cagttgaagt cggaagttta catacactta agttggagtc attaaaactc gtttttcaac 60

tacaccacaa atttcttgtt aacaaacaat agttttggca agtcagttag gacatctact 120

ttgtgcatga cacaagtcat ttttccaaca attgtttaca gacagattat ttcacttata 180

attcactgta tcacaattcc agtgggtcag aagtttacat acactaagtt gactgtgcct 240

ttaaacagct tggaaaattc cagaaaatga tgtcatggct ttagaagctt ctgatagact 300

aattgacatc atttgagtca attggaggtg tacctgtgga tgtatttcaa ggcctacctt 360

caaacgcagt gcctctttgc ttgacataat gggaaaatca aaagaaatca gccaacacca 420

tgggaccacg cagccgtcat accgctcagg aatgagacgc attctgtctc ctagagataa 480

acatactgtg gtgcgaaaag tgcaaatcaa tcccagaacg acagcaaagg accttgtgaa 540

gatgctggag aaaacaggta tgaatgtttc tatatccaca gtaaaaacga gtcctatatc 600

gacataacct gaaaggccgc tcagcaagga agaagccact gctccaaaac cgccataaaa 660

aagccagact acggtttgca actgcacatg gggacaaata tggtactttt tggagaaatg 720

tcctctcttc tggtctgatg aaaaaaaaat agaactattt ggccataatg accatcgtta 780

```
tgtttggagg aaaaaggggg agcttgcaag ccgaagatca ccatcccaag cgtgaagcac     840
gggggtggca gcatcatgtt gtgggggtgc tttgctgcag gagggactgg tgcacttcac     900
aaaatagatg gcatcatgac aaaggaaaat tatgtggcta tattgaagca acatctcaag     960
acatcagtca ggaagttcaa gcttggtcac aaatgggtct tccaaatgga caatgacctc    1020
aagcatactt ccaaagttgt ggcaaaatgg cttaaggtca acaaagtcaa ggtattggag    1080
tggccatcac aaagctctga cctcaatcct atagaaagga ggaatgagcc aaaattcacc    1140
caacttattg tgggaagctt gtggaaggct actcgaaatg tttgacccaa gttaaacaat    1200
ttaaaggcaa tgctaccaaa tactaattga gtgtatgtta acttctgacc cactgggaat    1260
gtgatgaaag aaataaaagc tgaaatgaat cattctctct actattattc tgatatttca    1320
cattcttaaa ataaagtggt gatcctaact gaccttaaga cagggaatct ttactcggat    1380
taaatgtcag gaattgtgaa aaagtgagtt taaatgtatt tggctaaggt gtatgtaaac    1440
ttccgacttc aactg                                                     1455


<210>  2
<211>  1023
<212>  DNA
<213>  Artificial Sequence

<220>
<223>  Sleeping Beauty transposase


<220>
<221>  CDS
<222>  (1)..(1023)

<220>
<221>  misc_feature
<222>  (1)..(1023)
<223>  Sleeping Beauty transposase

<400>  2
atg gga aaa tca aaa gaa atc agc caa gac ctc aga aaa aaa att gta       48
Met Gly Lys Ser Lys Glu Ile Ser Gln Asp Leu Arg Lys Lys Ile Val
1               5                   10                  15

gac ctc cac aag tct ggt tca tcc ttg gga gca att tcc aaa cgc ctg       96
Asp Leu His Lys Ser Gly Ser Ser Leu Gly Ala Ile Ser Lys Arg Leu
            20                  25                  30

aaa gta cca cgt tca tct gta caa aca ata gta cgc aag tat aaa cac      144
Lys Val Pro Arg Ser Ser Val Gln Thr Ile Val Arg Lys Tyr Lys His
        35                  40                  45

cat ggg acc acg cag ccg tca tac cgc tca gga agg aga cgc gtt ctg      192
His Gly Thr Thr Gln Pro Ser Tyr Arg Ser Gly Arg Arg Arg Val Leu
    50                  55                  60

tct cct aga gat gaa cgt act ttg gtg cga aaa gtg caa atc aat ccc      240
```

```
Ser Pro Arg Asp Glu Arg Thr Leu Val Arg Lys Val Gln Ile Asn Pro
65                  70                  75                  80

aga aca aca gca aag gac ctt gtg aag atg ctg gag gaa aca ggt aca      288
Arg Thr Thr Ala Lys Asp Leu Val Lys Met Leu Glu Glu Thr Gly Thr
                85                  90                  95

aaa gta tct ata tcc aca gta aaa cga gtc cta tat cga cat aac ctg      336
Lys Val Ser Ile Ser Thr Val Lys Arg Val Leu Tyr Arg His Asn Leu
            100                 105                 110

aaa ggc cgc tca gca agg aag aag cca ctg ctc caa aac cga cat aag      384
Lys Gly Arg Ser Ala Arg Lys Lys Pro Leu Leu Gln Asn Arg His Lys
        115                 120                 125

aaa gcc aga cta cgg ttt gca act gca cat ggg gac aaa gat cgt act      432
Lys Ala Arg Leu Arg Phe Ala Thr Ala His Gly Asp Lys Asp Arg Thr
    130                 135                 140

ttt tgg aga aat gtc ctc tgg tct gat gaa aca aaa ata gaa ctg ttt      480
Phe Trp Arg Asn Val Leu Trp Ser Asp Glu Thr Lys Ile Glu Leu Phe
145                 150                 155                 160

ggc cat aat gac cat cgt tat gtt tgg agg aag aag ggg gag gct tgc      528
Gly His Asn Asp His Arg Tyr Val Trp Arg Lys Lys Gly Glu Ala Cys
                165                 170                 175

aag ccg aag aac acc atc cca acc gtg aag cac ggg ggt ggc agc atc      576
Lys Pro Lys Asn Thr Ile Pro Thr Val Lys His Gly Gly Gly Ser Ile
            180                 185                 190

atg ttg tgg ggg tgc ttt gct gca gga ggg act ggt gca ctt cac aaa      624
Met Leu Trp Gly Cys Phe Ala Ala Gly Gly Thr Gly Ala Leu His Lys
        195                 200                 205

ata gat ggc atc atg agg aag gaa aat tat gtg gat ata ttg aag caa      672
Ile Asp Gly Ile Met Arg Lys Glu Asn Tyr Val Asp Ile Leu Lys Gln
    210                 215                 220

cat ctc aag aca tca gtc agg aag tta aag ctt ggt cgc aaa tgg gtc      720
His Leu Lys Thr Ser Val Arg Lys Leu Lys Leu Gly Arg Lys Trp Val
225                 230                 235                 240

ttc caa atg gac aat gac ccc aag cat act tcc aaa gtt gtg gca aaa      768
Phe Gln Met Asp Asn Asp Pro Lys His Thr Ser Lys Val Val Ala Lys
                245                 250                 255

tgg ctt aag gac aac aaa gtc aag gta ttg gag tgg cca tca caa agc      816
Trp Leu Lys Asp Asn Lys Val Lys Val Leu Glu Trp Pro Ser Gln Ser
            260                 265                 270

cct gac ctc aat cct ata gaa aat ttg tgg gca gaa ctg aaa aag cgt      864
Pro Asp Leu Asn Pro Ile Glu Asn Leu Trp Ala Glu Leu Lys Lys Arg
        275                 280                 285

gtg cga gca agg agg cct aca aac ctg act cag tta cac cag ctc tgt      912
Val Arg Ala Arg Arg Pro Thr Asn Leu Thr Gln Leu His Gln Leu Cys
    290                 295                 300

cag gag gaa tgg gcc aaa att cac cca act tat tgt ggg aag ctt gtg      960
Gln Glu Glu Trp Ala Lys Ile His Pro Thr Tyr Cys Gly Lys Leu Val
```

```
305                 310                 315                 320

gaa ggc tac ccg aaa cgt ttg acc caa gtt aaa caa ttt aaa ggc aat      1008
Glu Gly Tyr Pro Lys Arg Leu Thr Gln Val Lys Gln Phe Lys Gly Asn
                325                 330                 335

gct acc aaa tac tag                                                  1023
Ala Thr Lys Tyr
            340


<210>  3
<211>  340
<212>  PRT
<213>  Artificial Sequence

<220>
<223>  Synthetic Construct

<400>  3

Met Gly Lys Ser Lys Glu Ile Ser Gln Asp Leu Arg Lys Lys Ile Val
1               5                   10                  15

Asp Leu His Lys Ser Gly Ser Ser Leu Gly Ala Ile Ser Lys Arg Leu
            20                  25                  30

Lys Val Pro Arg Ser Ser Val Gln Thr Ile Val Arg Lys Tyr Lys His
        35                  40                  45

His Gly Thr Thr Gln Pro Ser Tyr Arg Ser Gly Arg Arg Arg Val Leu
    50                  55                  60

Ser Pro Arg Asp Glu Arg Thr Leu Val Arg Lys Val Gln Ile Asn Pro
65                  70                  75                  80

Arg Thr Thr Ala Lys Asp Leu Val Lys Met Leu Glu Glu Thr Gly Thr
                85                  90                  95

Lys Val Ser Ile Ser Thr Val Lys Arg Val Leu Tyr Arg His Asn Leu
            100                 105                 110

Lys Gly Arg Ser Ala Arg Lys Lys Pro Leu Leu Gln Asn Arg His Lys
        115                 120                 125

Lys Ala Arg Leu Arg Phe Ala Thr Ala His Gly Asp Lys Asp Arg Thr
    130                 135                 140

Phe Trp Arg Asn Val Leu Trp Ser Asp Glu Thr Lys Ile Glu Leu Phe
145                 150                 155                 160
```

```
Gly His Asn Asp His Arg Tyr Val Trp Arg Lys Lys Gly Glu Ala Cys
                165                 170                 175

Lys Pro Lys Asn Thr Ile Pro Thr Val Lys His Gly Gly Gly Ser Ile
            180                 185                 190

Met Leu Trp Gly Cys Phe Ala Ala Gly Gly Thr Gly Ala Leu His Lys
        195                 200                 205

Ile Asp Gly Ile Met Arg Lys Glu Asn Tyr Val Asp Ile Leu Lys Gln
    210                 215                 220

His Leu Lys Thr Ser Val Arg Lys Leu Lys Leu Gly Arg Lys Trp Val
225                 230                 235                 240

Phe Gln Met Asp Asn Asp Pro Lys His Thr Ser Lys Val Val Ala Lys
                245                 250                 255

Trp Leu Lys Asp Asn Lys Val Lys Val Leu Glu Trp Pro Ser Gln Ser
            260                 265                 270

Pro Asp Leu Asn Pro Ile Glu Asn Leu Trp Ala Glu Leu Lys Lys Arg
        275                 280                 285

Val Arg Ala Arg Arg Pro Thr Asn Leu Thr Gln Leu His Gln Leu Cys
    290                 295                 300

Gln Glu Glu Trp Ala Lys Ile His Pro Thr Tyr Cys Gly Lys Leu Val
305                 310                 315                 320

Glu Gly Tyr Pro Lys Arg Leu Thr Gln Val Lys Gln Phe Lys Gly Asn
                325                 330                 335

Ala Thr Lys Tyr
            340


<210>  4
<211>  26
<212>  DNA
<213>  Artificial Sequence

<220>
<223>  Left outside sequence

<400>  4
gttgaagtcg gaagtttaca cttagg                                          26


<210>  5
```

<211> 30
<212> DNA
<213> Artificial Sequence

<220>
<223> Left inside sequence

<400> 5
ccagtgggtc agaagtttac atacactaag 30

<210> 6
<211> 27
<212> DNA
<213> Artificial Sequence;

<220>
<223> TgTP-1U

<400> 6
gaccgcttcc tcgtgcttta cggtatc 27

<210> 7
<211> 30
<212> DNA
<213> Artificial Sequence;

<220>
<223> TgTP-2L

<400> 7
acacaggaaa cagctatgac catgattacg 30

<210> 8
<211> 30
<212> DNA
<213> Artificial Sequence;

<220>
<223> TgTP-2U

<400> 8
tctatcgcct tcttgacgag ttcttctgag 30

<210> 9
<211> 28
<212> DNA
<213> Artificial Sequence;

<220>
<223> TgTP-3L

<400> 9
caagcgcgca attaaccctc actaaagg 28

<210> 10
<211> 1610

```
<212>  DNA
<213>  Caenorhabditis elegans


<220>
<221>  misc_feature
<222>  (1)..(1610)
<223>  Transposon="Tc1"

<220>
<221>  CDS
<222>  (523)..(1344)

<300>
<308>  X01005
<309>  2002-07-07
<313>  (1)..(1610)

<400>  10
cagtgctggc caaaaagata tccacttttg gttttttgtg tgtaactttt ttctcaagca      60

tccatttgac ttgaattttt ccgtgtgcat aaagcgaaat gttacgcaaa tttgcggacc     120

aaacattaca tgattatcga ttttttctga attttatttc aatttttga ttttttcgtt      180

tttccaattt tcattatttt ttttgaatta tcaataaaac gcactctgtt tgttgcactg     240

gatttgtttg gttgataaat tatttttaag gtatggtaaa atctgttggg tgtaaaaatc     300

tttccttgga cgtcaagaaa gccattgtag ctggcttcga acaaggaata cccacgaaaa     360

gctcgcgctg caaattcaac gttctccgtc gactatttgg aaagtaatca agaagtacca     420

aactgaggtg agttcgaaaa atattatttt ttaataataa atgtttagaa atccgtcgct     480

ttgagaatct cgcccggcag gcctcgagtg acaacccata gg atg gat cgc aac        534
                                               Met Asp Arg Asn
                                               1

atc ctc cga tca gca aga gaa gat ccg cat agg acc gcc acg gat att       582
Ile Leu Arg Ser Ala Arg Glu Asp Pro His Arg Thr Ala Thr Asp Ile
5                   10                  15                  20

caa atg att ata agt tct cca aat gaa cct gta cca agt aaa cga act       630
Gln Met Ile Ile Ser Ser Pro Asn Glu Pro Val Pro Ser Lys Arg Thr
                25                  30                  35

gtt cgt cga cgt tta cag caa gca gga cta cac gga cga aag cca gtc       678
Val Arg Arg Arg Leu Gln Gln Ala Gly Leu His Gly Arg Lys Pro Val
            40                  45                  50

aag aaa ccg ttc atc agt aag aaa aat cgc atg gct cga gtt gcg tgg       726
Lys Lys Pro Phe Ile Ser Lys Lys Asn Arg Met Ala Arg Val Ala Trp
        55                  60                  65

gca aaa gcg cat ctt cgt tgg gga cgt cag gaa tgg gct aaa cac atc       774
Ala Lys Ala His Leu Arg Trp Gly Arg Gln Glu Trp Ala Lys His Ile
    70                  75                  80

tgg tct gac gaa agc aag ttc aat ttg ttc ggg agt gat gga aat tcc       822
Trp Ser Asp Glu Ser Lys Phe Asn Leu Phe Gly Ser Asp Gly Asn Ser
```

```
85                  90                  95                  100

tgg gta cgt cgt cct gtt ggc tct agg tac tct cca aag tat caa tgc      870
Trp Val Arg Arg Pro Val Gly Ser Arg Tyr Ser Pro Lys Tyr Gln Cys
                105                 110                 115

cca acc gtt aag cat gga ggt ggg agc gtc atg gtg tgg ggg tgc ttc      918
Pro Thr Val Lys His Gly Gly Gly Ser Val Met Val Trp Gly Cys Phe
            120                 125                 130

acc agc act tcc atg ggc cca cta agg aga atc caa agc att atg gat      966
Thr Ser Thr Ser Met Gly Pro Leu Arg Arg Ile Gln Ser Ile Met Asp
        135                 140                 145

cgt ttt caa tac gaa aac atc ttt gaa act aca atg cga ccc tgg gca     1014
Arg Phe Gln Tyr Glu Asn Ile Phe Glu Thr Thr Met Arg Pro Trp Ala
    150                 155                 160

ctt caa aat gtg ggc cgt ggc ttc gtg ttt cag cag gat aac gat cct     1062
Leu Gln Asn Val Gly Arg Gly Phe Val Phe Gln Gln Asp Asn Asp Pro
165                 170                 175                 180

aag cat act tct ctt cat gtg cgt tca tgg ttt caa cgt cgt cat gtg     1110
Lys His Thr Ser Leu His Val Arg Ser Trp Phe Gln Arg Arg His Val
                185                 190                 195

cat ttg ctc gat tgg cca agt cag tct ccg gac ttg aat cca ata gag     1158
His Leu Leu Asp Trp Pro Ser Gln Ser Pro Asp Leu Asn Pro Ile Glu
            200                 205                 210

cat ttg tgg gaa gag ttg gaa aga cgt ctt gga ggt att cgg gct tca     1206
His Leu Trp Glu Glu Leu Glu Arg Arg Leu Gly Gly Ile Arg Ala Ser
        215                 220                 225

aat gca gat gcc aaa ttc aac cag ttg gaa aac gct tgg aaa gct atc     1254
Asn Ala Asp Ala Lys Phe Asn Gln Leu Glu Asn Ala Trp Lys Ala Ile
    230                 235                 240

ccc atg tca gtt att cac aag ctg atc gac tcg atg cca cgt cgt tgt     1302
Pro Met Ser Val Ile His Lys Leu Ile Asp Ser Met Pro Arg Arg Cys
245                 250                 255                 260

caa gct gtt att gat gca aac gga tac gcg aca aag tat taa             1344
Gln Ala Val Ile Asp Ala Asn Gly Tyr Ala Thr Lys Tyr
                265                 270

gcataattat gttgttttta aatccaattg ctcatattcc ggtactttaa ttgtcatttc   1404

cttgcaacct cggtttttttc aatatttcta gtttttcgat ttttttgaat ttttctgaag   1464

ttttttcaaa atctgttgaa catttttgat gaatattgtg tttttagatt ttgtgaacac   1524

tgtggtgaag tttcaaaaca aaataaccac ttagaaaaaa gttacacaca aaaaaccaaa   1584

agtggatatc tttttggcca gcactg                                         1610


<210>  11
<211>  273
<212>  PRT
<213>  Caenorhabditis elegans
```

<400> 11

```
Met Asp Arg Asn Ile Leu Arg Ser Ala Arg Glu Asp Pro His Arg Thr
1               5                   10                  15

Ala Thr Asp Ile Gln Met Ile Ile Ser Ser Pro Asn Glu Pro Val Pro
            20                  25                  30

Ser Lys Arg Thr Val Arg Arg Arg Leu Gln Gln Ala Gly Leu His Gly
        35                  40                  45

Arg Lys Pro Val Lys Lys Pro Phe Ile Ser Lys Lys Asn Arg Met Ala
    50                  55                  60

Arg Val Ala Trp Ala Lys Ala His Leu Arg Trp Gly Arg Gln Glu Trp
65                  70                  75                  80

Ala Lys His Ile Trp Ser Asp Glu Ser Lys Phe Asn Leu Phe Gly Ser
                85                  90                  95

Asp Gly Asn Ser Trp Val Arg Arg Pro Val Gly Ser Arg Tyr Ser Pro
            100                 105                 110

Lys Tyr Gln Cys Pro Thr Val Lys His Gly Gly Gly Ser Val Met Val
        115                 120                 125

Trp Gly Cys Phe Thr Ser Thr Ser Met Gly Pro Leu Arg Arg Ile Gln
    130                 135                 140

Ser Ile Met Asp Arg Phe Gln Tyr Glu Asn Ile Phe Glu Thr Thr Met
145                 150                 155                 160

Arg Pro Trp Ala Leu Gln Asn Val Gly Arg Gly Phe Val Phe Gln Gln
                165                 170                 175

Asp Asn Asp Pro Lys His Thr Ser Leu His Val Arg Ser Trp Phe Gln
            180                 185                 190

Arg Arg His Val His Leu Leu Asp Trp Pro Ser Gln Ser Pro Asp Leu
        195                 200                 205

Asn Pro Ile Glu His Leu Trp Glu Glu Leu Glu Arg Arg Leu Gly Gly
    210                 215                 220

Ile Arg Ala Ser Asn Ala Asp Ala Lys Phe Asn Gln Leu Glu Asn Ala
225                 230                 235                 240
```

```
Trp Lys Ala Ile Pro Met Ser Val Ile His Lys Leu Ile Asp Ser Met
                245                 250                 255

Pro Arg Arg Cys Gln Ala Val Ile Asp Ala Asn Gly Tyr Ala Thr Lys
            260                 265                 270

Tyr
```

<210> 12
<211> 1801
<212> DNA
<213> Drosophila hydei

<220>
<221> misc_feature
<222> (15)..(1787)
<223> /note="transposon"

<220>
<221> CDS
<222> (347)..(760)

<220>
<221> CDS
<222> (821)..(1489)

<300>
<308> Z29098
<309> 1993-12-22
<313> (1)..(1801)

<400> 12

```
taatatatat tatacgagcc ccaaccacta ttaattcgaa cagcatgttt tttttgcagt      60

gcgcaatgtt taacacacta tattatcaat actactaaag ataacacata ccaatgcatt     120

tcgtctcaaa gagaatttta ttctcttcac gacgaaaaaa aaagttttgc tctatttcca     180

acaacaacaa aaatatgagt aatttattca aacggtttgc ttaagagata agaaaaaagt     240

gaccactatt aattcgaacg cggcgtaagc ttaccttaat ctcaagaaga gcaaaacaaa     300

agcaactaat gtaacggaat cattatctag ttatgatctg caaata atg tca caa        355
                                                   Met Ser Gln
                                                   1

tac agc atg caa aaa aat ttt aga ttg ctg cag atc agt aga agt tta       403
Tyr Ser Met Gln Lys Asn Phe Arg Leu Leu Gln Ile Ser Arg Ser Leu
    5                   10                  15

gca acg atg gtt cgt ggt aaa cct att tct aaa gaa atc aga gta ttg       451
Ala Thr Met Val Arg Gly Lys Pro Ile Ser Lys Glu Ile Arg Val Leu
20                  25                  30                  35
```

```
att agg gat tat ttt aaa tct gga aag aca ctt acg gag ata agc aag      499
Ile Arg Asp Tyr Phe Lys Ser Gly Lys Thr Leu Thr Glu Ile Ser Lys
                40                  45                  50

caa tta aat ttg cct aag tcg tct gtg cat ggg gtg ata caa att ttc      547
Gln Leu Asn Leu Pro Lys Ser Ser Val His Gly Val Ile Gln Ile Phe
            55                  60                  65

aaa aaa aat ggg aat att gaa aat aac att gcg aat aga ggc cga aca      595
Lys Lys Asn Gly Asn Ile Glu Asn Asn Ile Ala Asn Arg Gly Arg Thr
        70                  75                  80

tca gca ata aca ccc cgc gac aaa aga caa ctg gcc aaa att gtt aag      643
Ser Ala Ile Thr Pro Arg Asp Lys Arg Gln Leu Ala Lys Ile Val Lys
    85                  90                  95

gct gat cgt cgc caa tct ttg aga aat ttg gct tct aag tgg tcg cag      691
Ala Asp Arg Arg Gln Ser Leu Arg Asn Leu Ala Ser Lys Trp Ser Gln
100                 105                 110                 115

caa ttg gca aaa ctg tca agc gag agt gga cgc gac aaa tta aaa agt      739
Gln Leu Ala Lys Leu Ser Ser Glu Ser Gly Arg Asp Lys Leu Lys Ser
                120                 125                 130

att gga tat ggt ttt tat aaa gtatgttttg ttattacctg tgcatcgtac      790
Ile Gly Tyr Gly Phe Tyr Lys
            135

ccaataactt actcgtaatc ttactcgtag gcc aag gaa aaa ccc ttg ctt acg      844
                                 Ala Lys Glu Lys Pro Leu Leu Thr
                                     140                 145

ctt cgt caa aaa aag aag cgt ttg caa tgg gct cgg gaa agg atg tct      892
Leu Arg Gln Lys Lys Lys Arg Leu Gln Trp Ala Arg Glu Arg Met Ser
            150                 155                 160

tgg act caa agg caa tgg gat acc atc ata ttc agc gat gaa gct aaa      940
Trp Thr Gln Arg Gln Trp Asp Thr Ile Ile Phe Ser Asp Glu Ala Lys
        165                 170                 175

ttt gat gtt agt gtc ggc gat acg aga aaa cgc gtc atc cgt aag agg      988
Phe Asp Val Ser Val Gly Asp Thr Arg Lys Arg Val Ile Arg Lys Arg
    180                 185                 190

tca gaa aca tac cat aaa gac tgc ctt aaa aga aca aca aag ttt cct     1036
Ser Glu Thr Tyr His Lys Asp Cys Leu Lys Arg Thr Thr Lys Phe Pro
195                 200                 205                 210

gcg agc act atg gta tgg gga tgt atg tct gcc aaa gga tta gga aaa     1084
Ala Ser Thr Met Val Trp Gly Cys Met Ser Ala Lys Gly Leu Gly Lys
                215                 220                 225

ctt cat ttc att gaa ggg aca gtt aat gct gaa aaa tat att aat att     1132
Leu His Phe Ile Glu Gly Thr Val Asn Ala Glu Lys Tyr Ile Asn Ile
            230                 235                 240

tta caa gat agt ttg ttg cca tca ata cca aaa cta tta gat tgc ggt     1180
Leu Gln Asp Ser Leu Leu Pro Ser Ile Pro Lys Leu Leu Asp Cys Gly
        245                 250                 255

gaa ttc act ttt cag cag gac gga gca tca tcg cac aca gcc aag cga     1228
```

```
Glu Phe Thr Phe Gln Gln Asp Gly Ala Ser Ser His Thr Ala Lys Arg
    260                 265                 270

acc aaa aat tgg ctg caa tat aat caa atg gag gtt tta gat tgg cca      1276
Thr Lys Asn Trp Leu Gln Tyr Asn Gln Met Glu Val Leu Asp Trp Pro
275                 280                 285                 290

tca aat agt cca gat cta agc cca att gaa aat att tgg tgg cta atg      1324
Ser Asn Ser Pro Asp Leu Ser Pro Ile Glu Asn Ile Trp Trp Leu Met
                295                 300                 305

aaa aac cag ctt cga aat gag cca caa agg aat att tct gac ttg aaa      1372
Lys Asn Gln Leu Arg Asn Glu Pro Gln Arg Asn Ile Ser Asp Leu Lys
            310                 315                 320

atc aag ttg caa gag atg tgg gac tca att tct caa gag cat tgc aaa      1420
Ile Lys Leu Gln Glu Met Trp Asp Ser Ile Ser Gln Glu His Cys Lys
        325                 330                 335

aat ttg tta agc tca atg cca aaa cga gtt aaa tgc gta atg cag gcc      1468
Asn Leu Leu Ser Ser Met Pro Lys Arg Val Lys Cys Val Met Gln Ala
    340                 345                 350

aag ggc gac gtt aca caa ttc taatattaat taaattattg ttttaagtat         1519
Lys Gly Asp Val Thr Gln Phe
355                 360

gatagtaaat cacattacgc cgcgttcgaa ttaatagtgg tcactttttt cttatctctt   1579

aagcaaaccg tttgaataaa ttactcatat ttttgttgtt gttggaaata gagcaaaact   1639

tttttttttcg tcgtgaagag aataaaattc tctttgagac gaaatgcatt ggtatgtgtt  1699

atctttagta gtattgataa tatagtgtgt taaacattgc gcactgcaaa aaaaacatgc   1759

tgttcgaatt aatagtggtt ggggctcgta tattatatat ta                      1801


<210>  13
<211>  361
<212>  PRT
<213>  Drosophila hydei

<400>  13

Met Ser Gln Tyr Ser Met Gln Lys Asn Phe Arg Leu Leu Gln Ile Ser
1               5                   10                  15


Arg Ser Leu Ala Thr Met Val Arg Gly Lys Pro Ile Ser Lys Glu Ile
            20                  25                  30


Arg Val Leu Ile Arg Asp Tyr Phe Lys Ser Gly Lys Thr Leu Thr Glu
        35                  40                  45


Ile Ser Lys Gln Leu Asn Leu Pro Lys Ser Ser Val His Gly Val Ile
    50                  55                  60
```

Gln Ile Phe Lys Lys Asn Gly Asn Ile Glu Asn Asn Ile Ala Asn Arg
65 70 75 80

Gly Arg Thr Ser Ala Ile Thr Pro Arg Asp Lys Arg Gln Leu Ala Lys
85 90 95

Ile Val Lys Ala Asp Arg Arg Gln Ser Leu Arg Asn Leu Ala Ser Lys
100 105 110

Trp Ser Gln Gln Leu Ala Lys Leu Ser Ser Glu Ser Gly Arg Asp Lys
115 120 125

Leu Lys Ser Ile Gly Tyr Gly Phe Tyr Lys Ala Lys Glu Lys Pro Leu
130 135 140

Leu Thr Leu Arg Gln Lys Lys Lys Arg Leu Gln Trp Ala Arg Glu Arg
145 150 155 160

Met Ser Trp Thr Gln Arg Gln Trp Asp Thr Ile Ile Phe Ser Asp Glu
165 170 175

Ala Lys Phe Asp Val Ser Val Gly Asp Thr Arg Lys Arg Val Ile Arg
180 185 190

Lys Arg Ser Glu Thr Tyr His Lys Asp Cys Leu Lys Arg Thr Thr Lys
195 200 205

Phe Pro Ala Ser Thr Met Val Trp Gly Cys Met Ser Ala Lys Gly Leu
210 215 220

Gly Lys Leu His Phe Ile Glu Gly Thr Val Asn Ala Glu Lys Tyr Ile
225 230 235 240

Asn Ile Leu Gln Asp Ser Leu Leu Pro Ser Ile Pro Lys Leu Leu Asp
245 250 255

Cys Gly Glu Phe Thr Phe Gln Gln Asp Gly Ala Ser Ser His Thr Ala
260 265 270

Lys Arg Thr Lys Asn Trp Leu Gln Tyr Asn Gln Met Glu Val Leu Asp
275 280 285

Trp Pro Ser Asn Ser Pro Asp Leu Ser Pro Ile Glu Asn Ile Trp Trp
290 295 300

Leu Met Lys Asn Gln Leu Arg Asn Glu Pro Gln Arg Asn Ile Ser Asp

```
305                 310                 315                 320

Leu Lys Ile Lys Leu Gln Glu Met Trp Asp Ser Ile Ser Gln Glu His
                325                 330                 335

Cys Lys Asn Leu Leu Ser Ser Met Pro Lys Arg Val Lys Cys Val Met
            340                 345                 350

Gln Ala Lys Gly Asp Val Thr Gln Phe
        355                 360
```

<210> 14
<211> 1801
<212> DNA
<213> Drosophila hydei

<220>
<221> misc_feature
<222> (15)..(1787)
<223> /note="transposon"

<220>
<221> CDS
<222> (347)..(760)

<220>
<221> CDS
<222> (821)..(1489)

<300>
<308> Z29102
<309> 1994-07-01
<313> (1)..(1801)

<400> 14

```
gttagcagct tctacgagcc ccaaccacta ttaattcgaa cagcatgttt tttttgcagt      60

gcgcaatgtt taacacacta tattatcaat actactaaag ataacacata ccaatgcatt     120

tcgtctcaaa gagaatttta ttctcttcac gacgaaaaaa aaagttttgc tctatttcca     180

acaacaacaa aaatatgagt aatttattca aacggtttgc ttaagagata agaaaaaagt     240

gaccactatt aattcgaacg cggcgtaagc ttaccttaat ctcaagaaga gcaaaacaaa     300

agcaactaat gtaacggaat cattatctag ttatgatctg caaata atg tca caa        355
                                                   Met Ser Gln
                                                   1

tac agc atg caa aaa aat ttt aga ttg ctg cag atc agt aga agt tta       403
Tyr Ser Met Gln Lys Asn Phe Arg Leu Leu Gln Ile Ser Arg Ser Leu
    5                   10                  15

gca acg atg gtt cgt ggt aaa cct att tct aaa gaa atc aga gta ttg       451
Ala Thr Met Val Arg Gly Lys Pro Ile Ser Lys Glu Ile Arg Val Leu
20                  25                  30                  35
```

```
att agg gat tat ttt aaa tct gga aag aca ctt acg gag ata agc aag      499
Ile Arg Asp Tyr Phe Lys Ser Gly Lys Thr Leu Thr Glu Ile Ser Lys
                40                  45                  50

caa tta aat ttg cct aag tcg tct gtg cat ggg gtg ata caa att ttc      547
Gln Leu Asn Leu Pro Lys Ser Ser Val His Gly Val Ile Gln Ile Phe
            55                  60                  65

aaa aaa aat ggg aat att gaa aat aac att gcg aat aga ggc cga aca      595
Lys Lys Asn Gly Asn Ile Glu Asn Asn Ile Ala Asn Arg Gly Arg Thr
        70                  75                  80

tca gca ata aca ccc cgc gac aaa aga caa ctg gcc aaa att gtt aag      643
Ser Ala Ile Thr Pro Arg Asp Lys Arg Gln Leu Ala Lys Ile Val Lys
    85                  90                  95

gct gat cgt cgc caa tct ttg aga aat ttg gct tct aag tgg tcg cag      691
Ala Asp Arg Arg Gln Ser Leu Arg Asn Leu Ala Ser Lys Trp Ser Gln
100                 105                 110                 115

caa ttg gca aaa ctg tca agc gag agt gga cgc gac aaa tta aaa agt      739
Gln Leu Ala Lys Leu Ser Ser Glu Ser Gly Arg Asp Lys Leu Lys Ser
                120                 125                 130

att gga tat ggt ttt tat aaa gtatgttttg ttattacctg tgcatcgtac         790
Ile Gly Tyr Gly Phe Tyr Lys
            135

ccaataactt actcgtaatc ttactcgtag gcc aag gaa aaa ccc ttg ctt acg     844
                                 Ala Lys Glu Lys Pro Leu Leu Thr
                                     140                 145

ctt cgt caa aaa aag aag cgt ttg caa tgg gct cgg gaa agg atg tct      892
Leu Arg Gln Lys Lys Lys Arg Leu Gln Trp Ala Arg Glu Arg Met Ser
            150                 155                 160

tgg act caa agg caa tgg gat acc atc ata ttc agc gat gaa gct aaa      940
Trp Thr Gln Arg Gln Trp Asp Thr Ile Ile Phe Ser Asp Glu Ala Lys
        165                 170                 175

ttt gat gtt agt gtc ggc gat acg aga aaa cgc gtc atc cgt aag agg      988
Phe Asp Val Ser Val Gly Asp Thr Arg Lys Arg Val Ile Arg Lys Arg
    180                 185                 190

tca gaa aca tac cat aaa gac tgc ctt aaa aga aca aca aag ttt cct     1036
Ser Glu Thr Tyr His Lys Asp Cys Leu Lys Arg Thr Thr Lys Phe Pro
195                 200                 205                 210

gcg agc act atg gta tgg gga tgt atg tct gcc aaa gga tta gga aaa     1084
Ala Ser Thr Met Val Trp Gly Cys Met Ser Ala Lys Gly Leu Gly Lys
                215                 220                 225

ctt cat ttc att gaa ggg aca gtt aat gct gaa aaa tat att aat att     1132
Leu His Phe Ile Glu Gly Thr Val Asn Ala Glu Lys Tyr Ile Asn Ile
            230                 235                 240

tta caa gat agt ttg ttg cca tca ata cca aaa cta tca gat tgc ggt     1180
Leu Gln Asp Ser Leu Leu Pro Ser Ile Pro Lys Leu Ser Asp Cys Gly
        245                 250                 255
```

```
gaa ttc act ttt cag cag gac gga gca tca tcg cac aca gcc aag cga      1228
Glu Phe Thr Phe Gln Gln Asp Gly Ala Ser Ser His Thr Ala Lys Arg
    260                 265                 270

acc aaa aat tgg ctg caa tat aat caa atg gag gtt tta gat tgg cca      1276
Thr Lys Asn Trp Leu Gln Tyr Asn Gln Met Glu Val Leu Asp Trp Pro
275                 280                 285                 290

tca aat agt cca gat cta agc cca att gaa aat att tgg tgg cta atg      1324
Ser Asn Ser Pro Asp Leu Ser Pro Ile Glu Asn Ile Trp Trp Leu Met
                295                 300                 305

aaa aac cag ctt cga aat gag cca caa agg aat att tct gac ttg aaa      1372
Lys Asn Gln Leu Arg Asn Glu Pro Gln Arg Asn Ile Ser Asp Leu Lys
            310                 315                 320

atc aag ttg caa gag atg tgg gac tca att tct caa gag cat tgc aaa      1420
Ile Lys Leu Gln Glu Met Trp Asp Ser Ile Ser Gln Glu His Cys Lys
        325                 330                 335

aat ttg tta agc tca atg cca aaa cga gtt aaa tgc gta atg cag gcc      1468
Asn Leu Leu Ser Ser Met Pro Lys Arg Val Lys Cys Val Met Gln Ala
    340                 345                 350

aag ggc gac gtt aca caa ttc taatattaat taaattattg ttttaagtat        1519
Lys Gly Asp Val Thr Gln Phe
355                 360

gatagtaaat cacattacgc cgcgttcgaa ttaatagtgg tcactttttt cttatctctt   1579

aagcaaaccg tttgaataaa ttactcatat ttttgttgtt gttggaaata gagcaaaact   1639

tttttttttcg tcgtgaagag aataaaattc tctttgagac gaaatgcatt ggtatgtgtt  1699

atctttagta gtattgataa tatagtgtgt taaacattgc gcactgcaaa aaaaacatgc   1759

tgttcgaatt aatagtggtt ggggctcgta aagctaacta ta                      1801
```

<210> 15
<211> 361
<212> PRT
<213> Drosophila hydei

<400> 15

```
Met Ser Gln Tyr Ser Met Gln Lys Asn Phe Arg Leu Leu Gln Ile Ser
1               5                   10                  15

Arg Ser Leu Ala Thr Met Val Arg Gly Lys Pro Ile Ser Lys Glu Ile
            20                  25                  30

Arg Val Leu Ile Arg Asp Tyr Phe Lys Ser Gly Lys Thr Leu Thr Glu
        35                  40                  45

Ile Ser Lys Gln Leu Asn Leu Pro Lys Ser Ser Val His Gly Val Ile
    50                  55                  60
```

```
Gln Ile Phe Lys Lys Asn Gly Asn Ile Glu Asn Asn Ile Ala Asn Arg
65                  70                  75                  80

Gly Arg Thr Ser Ala Ile Thr Pro Arg Asp Lys Arg Gln Leu Ala Lys
                85                  90                  95

Ile Val Lys Ala Asp Arg Arg Gln Ser Leu Arg Asn Leu Ala Ser Lys
            100                 105                 110

Trp Ser Gln Gln Leu Ala Lys Leu Ser Ser Glu Ser Gly Arg Asp Lys
        115                 120                 125

Leu Lys Ser Ile Gly Tyr Gly Phe Tyr Lys Ala Lys Glu Lys Pro Leu
    130                 135                 140

Leu Thr Leu Arg Gln Lys Lys Lys Arg Leu Gln Trp Ala Arg Glu Arg
145                 150                 155                 160

Met Ser Trp Thr Gln Arg Gln Trp Asp Thr Ile Ile Phe Ser Asp Glu
                165                 170                 175

Ala Lys Phe Asp Val Ser Val Gly Asp Thr Arg Lys Arg Val Ile Arg
            180                 185                 190

Lys Arg Ser Glu Thr Tyr His Lys Asp Cys Leu Lys Arg Thr Thr Lys
        195                 200                 205

Phe Pro Ala Ser Thr Met Val Trp Gly Cys Met Ser Ala Lys Gly Leu
    210                 215                 220

Gly Lys Leu His Phe Ile Glu Gly Thr Val Asn Ala Glu Lys Tyr Ile
225                 230                 235                 240

Asn Ile Leu Gln Asp Ser Leu Leu Pro Ser Ile Pro Lys Leu Ser Asp
                245                 250                 255

Cys Gly Glu Phe Thr Phe Gln Gln Asp Gly Ala Ser Ser His Thr Ala
            260                 265                 270

Lys Arg Thr Lys Asn Trp Leu Gln Tyr Asn Gln Met Glu Val Leu Asp
        275                 280                 285

Trp Pro Ser Asn Ser Pro Asp Leu Ser Pro Ile Glu Asn Ile Trp Trp
    290                 295                 300
```

```
Leu Met Lys Asn Gln Leu Arg Asn Glu Pro Gln Arg Asn Ile Ser Asp
305                 310                 315                 320

Leu Lys Ile Lys Leu Gln Glu Met Trp Asp Ser Ile Ser Gln Glu His
                325                 330                 335

Cys Lys Asn Leu Leu Ser Ser Met Pro Lys Arg Val Lys Cys Val Met
            340                 345                 350

Gln Ala Lys Gly Asp Val Thr Gln Phe
        355                 360


<210>  16
<211>  1635
<212>  DNA
<213>  Haematobia irritans


<220>
<221>  CDS
<222>  (329)..(1366)

<400>  16
aaaatatgtg attaccgtta tagcggaaaa tatattcaga gagattagtt tactttaata      60

gcgtacataa agtttttga cctgatttt actctttctt cactattttg taaacactga      120

attaggattt gcgaatttat atggaaggaa atatctagaa caaacataaa caaagagata    180

ttgagagtaa catgttggct gataagtccc cggtttgaca ctagtattaa atgcatatta    240

tttttatata ggaccaacct tcaaatgatt cgtgtcaaaa tttgacgtca attagtttgt    300

gagagcaact tttgttattg tgaagaaa atg gaa aaa gaa ttt cgt gtt ttg      352
                               Met Glu Lys Glu Phe Arg Val Leu
                               1               5

ata aaa tac tgt ttt ctg aag gga aaa aat gcg gtg gaa gca aaa agt      400
Ile Lys Tyr Cys Phe Leu Lys Gly Lys Asn Ala Val Glu Ala Lys Ser
    10                  15                  20

tgg ctt gat aat gag ttt ccg gac tct gcc cca agg aaa tca ata ata      448
Trp Leu Asp Asn Glu Phe Pro Asp Ser Ala Pro Arg Lys Ser Ile Ile
25                  30                  35                  40

att gat tgg tat gca aaa ttc aag cga ggt gaa atg agc acg gag gac      496
Ile Asp Trp Tyr Ala Lys Phe Lys Arg Gly Glu Met Ser Thr Glu Asp
                45                  50                  55

ggt gaa cgc agt gga cgc ccg aaa gag gtg gtt acc gac gaa aac atc      544
Gly Glu Arg Ser Gly Arg Pro Lys Glu Val Val Thr Asp Glu Asn Ile
            60                  65                  70

aaa aaa atc cac aaa atg att ttg aat gac cgt aaa atg aag ttg atc      592
Lys Lys Ile His Lys Met Ile Leu Asn Asp Arg Lys Met Lys Leu Ile
        75                  80                  85
```

```
gag ata aca aag gcc tta aac ata tca aag gaa cgt gtt ggt cat atc      640
Glu Ile Thr Lys Ala Leu Asn Ile Ser Lys Glu Arg Val Gly His Ile
    90                  95                  100

att cat caa tat ttg gat atg cgg aag ctc tgt gca aaa tgg gtg ccg      688
Ile His Gln Tyr Leu Asp Met Arg Lys Leu Cys Ala Lys Trp Val Pro
105                 110                 115                 120

cgc gaa ctc aca ttt gac caa aaa caa caa cgt gtt gat gat tct gag      736
Arg Glu Leu Thr Phe Asp Gln Lys Gln Gln Arg Val Asp Asp Ser Glu
                125                 130                 135

cgg tgt ttg cag ctg tta act cgt aat aca ccc gag ttt ttc cgt cga      784
Arg Cys Leu Gln Leu Leu Thr Arg Asn Thr Pro Glu Phe Phe Arg Arg
            140                 145                 150

tat gta aca atg gat gaa aca tgg ctc cat cac tac act cct gag ttc      832
Tyr Val Thr Met Asp Glu Thr Trp Leu His His Tyr Thr Pro Glu Phe
        155                 160                 165

gat caa cag tcg gct gag tgg aca gcg acc ggt gaa ccg tct ccg aag      880
Asp Gln Gln Ser Ala Glu Trp Thr Ala Thr Gly Glu Pro Ser Pro Lys
    170                 175                 180

cgt gga aag act caa aag tcc gct ggc aaa gta atg gcc tct gtt ttt      928
Arg Gly Lys Thr Gln Lys Ser Ala Gly Lys Val Met Ala Ser Val Phe
185                 190                 195                 200

tgg aat gcg cat gga ata att ttt atc gat tat ctt gag aag gaa aaa      976
Trp Asn Ala His Gly Ile Ile Phe Ile Asp Tyr Leu Glu Lys Glu Lys
                205                 210                 215

acc atc aac agt gac tat tat atg gcg tta ttg gag cgt ttg aag gtc     1024
Thr Ile Asn Ser Asp Tyr Tyr Met Ala Leu Leu Glu Arg Leu Lys Val
            220                 225                 230

gaa atc gcg gca aaa tgg ccc cat atg aag aag aaa aaa gtg ttg ttc     1072
Glu Ile Ala Ala Lys Trp Pro His Met Lys Lys Lys Lys Val Leu Phe
        235                 240                 245

gac caa gac aat gca ccg tgc cac aag tca gta aga acg atg gca aaa     1120
Asp Gln Asp Asn Ala Pro Cys His Lys Ser Val Arg Thr Met Ala Lys
    250                 255                 260

att cat gaa ttg ggc ttc gaa ttg ctt ccc cac cca cta tat tct cca     1168
Ile His Glu Leu Gly Phe Glu Leu Leu Pro His Pro Leu Tyr Ser Pro
265                 270                 275                 280

gat ctg gcc ccc agc gaa ttt ttc ttg ttc tca gac ctc aaa agg ctc     1216
Asp Leu Ala Pro Ser Glu Phe Phe Leu Phe Ser Asp Leu Lys Arg Leu
                285                 290                 295

gca ggg aaa aaa ttt ggc tgc aat gaa gag gta atc gcc gaa act aag     1264
Ala Gly Lys Lys Phe Gly Cys Asn Glu Glu Val Ile Ala Glu Thr Lys
            300                 305                 310

gcc tat ttt gag gca aaa ccg aaa gag tac tac caa aat ggt atc aaa     1312
Ala Tyr Phe Glu Ala Lys Pro Lys Glu Tyr Tyr Gln Asn Gly Ile Lys
        315                 320                 325

aaa ttg gaa ggt cgt tat aat cgt ggt atc gct ctt gaa ggg gac tat     1360
```

```
Lys Leu Glu Gly Arg Tyr Asn Arg Gly Ile Ala Leu Glu Gly Asp Tyr
    330                 335                 340

gtt gaa taataaaaac gaattttgac aaaaaatgtg tttttctttg ttagaccggg      1416
Val Glu
345

gacttatcac ccaacctgtt aaaaactgtt actttttgtt aaagtaagtc agaataaaac   1476

aaatatttga atttttggag gtgtacgtaa acttctttga ttcactgtat atatttttaa   1536

gcttcacaat aaagtacaca cttgtagagt taaaatcgtc tcgtcttctc ttttactaaa   1596

tacaacatgg tgtcagaagg tgtgtgaagt ctaattaaa                          1635


<210>  17
<211>  346
<212>  PRT
<213>  Haematobia irritans

<400>  17

Met Glu Lys Glu Phe Arg Val Leu Ile Lys Tyr Cys Phe Leu Lys Gly
1               5                   10                  15

Lys Asn Ala Val Glu Ala Lys Ser Trp Leu Asp Asn Glu Phe Pro Asp
            20                  25                  30

Ser Ala Pro Arg Lys Ser Ile Ile Ile Asp Trp Tyr Ala Lys Phe Lys
        35                  40                  45

Arg Gly Glu Met Ser Thr Glu Asp Gly Glu Arg Ser Gly Arg Pro Lys
    50                  55                  60

Glu Val Val Thr Asp Glu Asn Ile Lys Lys Ile His Lys Met Ile Leu
65                  70                  75                  80

Asn Asp Arg Lys Met Lys Leu Ile Glu Ile Thr Lys Ala Leu Asn Ile
                85                  90                  95

Ser Lys Glu Arg Val Gly His Ile Ile His Gln Tyr Leu Asp Met Arg
            100                 105                 110

Lys Leu Cys Ala Lys Trp Val Pro Arg Glu Leu Thr Phe Asp Gln Lys
        115                 120                 125

Gln Gln Arg Val Asp Asp Ser Glu Arg Cys Leu Gln Leu Leu Thr Arg
    130                 135                 140

Asn Thr Pro Glu Phe Phe Arg Arg Tyr Val Thr Met Asp Glu Thr Trp
145                 150                 155                 160
```

```
Leu His His Tyr Thr Pro Glu Phe Asp Gln Gln Ser Ala Glu Trp Thr
                165                 170                 175

Ala Thr Gly Glu Pro Ser Pro Lys Arg Gly Lys Thr Gln Lys Ser Ala
            180                 185                 190

Gly Lys Val Met Ala Ser Val Phe Trp Asn Ala His Gly Ile Ile Phe
        195                 200                 205

Ile Asp Tyr Leu Glu Lys Glu Lys Thr Ile Asn Ser Asp Tyr Tyr Met
    210                 215                 220

Ala Leu Leu Glu Arg Leu Lys Val Glu Ile Ala Ala Lys Trp Pro His
225                 230                 235                 240

Met Lys Lys Lys Lys Val Leu Phe Asp Gln Asp Asn Ala Pro Cys His
                245                 250                 255

Lys Ser Val Arg Thr Met Ala Lys Ile His Glu Leu Gly Phe Glu Leu
            260                 265                 270

Leu Pro His Pro Leu Tyr Ser Pro Asp Leu Ala Pro Ser Glu Phe Phe
        275                 280                 285

Leu Phe Ser Asp Leu Lys Arg Leu Ala Gly Lys Lys Phe Gly Cys Asn
    290                 295                 300

Glu Glu Val Ile Ala Glu Thr Lys Ala Tyr Phe Glu Ala Lys Pro Lys
305                 310                 315                 320

Glu Tyr Tyr Gln Asn Gly Ile Lys Lys Leu Glu Gly Arg Tyr Asn Arg
                325                 330                 335

Gly Ile Ala Leu Glu Gly Asp Tyr Val Glu
            340                 345
```

<210> 18
<211> 1543
<212> DNA
<213> Chrysoperla plorabunda

<220>
<221> source
<222> (146)..(1442)
<223> /transposon="mariner transposon"

```
<220>
<221>  CDS
<222>  (327)..(1373)

<300>
<308>  U11652
<309>  1995-12-16
<313>  (1)..(1543)

<400>  18
ccttaaattt atttgtattg atttgaagct taaaaaatgt atacaatttt aaagaaagct      60

gaaagtattc tcgaactaac gtggagaatt tattacgaat ttttggtggt gattgtaatt     120

gcttgcttaa tatcggctct ctgtatatta ggttggctga taagtccccg gtctgacaca     180

tagatggcgt cgctagtatt aaatgcatat tatttttata tagtaccaac cttcaaatga     240

ttcgtgtcaa aatttgacgt ctgtaagtca attagtttgt gagatagagc gtcttttgtg     300

aagcaacttt tgttattgtg aaaaaa atg gaa aaa aag gaa ttt cgt gtt ttg     353
                             Met Glu Lys Lys Glu Phe Arg Val Leu
                             1               5

ata aaa tac tgt ttt ctg aag gga aaa aat aca gtg gaa gca aaa act      401
Ile Lys Tyr Cys Phe Leu Lys Gly Lys Asn Thr Val Glu Ala Lys Thr
10                  15                  20                  25

tgg ctt gat aat gag ttt ccg gac tct gcc cca ggg aaa tca aca ata      449
Trp Leu Asp Asn Glu Phe Pro Asp Ser Ala Pro Gly Lys Ser Thr Ile
                30                  35                  40

att gat tgg tat gca aaa ttc aag cgt ggt gaa atg agc acg gag gac      497
Ile Asp Trp Tyr Ala Lys Phe Lys Arg Gly Glu Met Ser Thr Glu Asp
            45                  50                  55

ggt gaa cgc agt gga cgc ccg aaa gag gtg gtt acc gac gaa aac atc      545
Gly Glu Arg Ser Gly Arg Pro Lys Glu Val Val Thr Asp Glu Asn Ile
        60                  65                  70

aaa aaa atc cac aaa atg att ttg aat gac cgt aaa atg aag ttg atc      593
Lys Lys Ile His Lys Met Ile Leu Asn Asp Arg Lys Met Lys Leu Ile
    75                  80                  85

gag ata gca gag gcc tta aag ata tca aag gaa cgt gtt ggt cat atc      641
Glu Ile Ala Glu Ala Leu Lys Ile Ser Lys Glu Arg Val Gly His Ile
90                  95                  100                 105

att cat caa tat ttg gat atg cgg aag ctc tgt gca aaa tgg gtg ccg      689
Ile His Gln Tyr Leu Asp Met Arg Lys Leu Cys Ala Lys Trp Val Pro
                110                 115                 120

cgc gag ctc aca ttt gac caa aaa caa caa cgt gtt gat gat tct gag      737
Arg Glu Leu Thr Phe Asp Gln Lys Gln Gln Arg Val Asp Asp Ser Glu
            125                 130                 135

cgg tgt ttg cag ctg tta act cgt aat aca ccc gag ttt ttg cgt cga      785
Arg Cys Leu Gln Leu Leu Thr Arg Asn Thr Pro Glu Phe Leu Arg Arg
        140                 145                 150

tat gtg aca atg gat gaa aca tgg ctc cat cac tac act cct gag tcc      833
```

```
Tyr Val Thr Met Asp Glu Thr Trp Leu His His Tyr Thr Pro Glu Ser
    155                 160                 165

aaa cga cag tcg gct gag tgg aca gcg acc ggt gaa ccg tct ccg aag      881
Lys Arg Gln Ser Ala Glu Trp Thr Ala Thr Gly Glu Pro Ser Pro Lys
170                 175                 180                 185

cgt gga aag act caa aag tcc gct ggc aaa gta atg gcc tct gtt ttt      929
Arg Gly Lys Thr Gln Lys Ser Ala Gly Lys Val Met Ala Ser Val Phe
                190                 195                 200

ttc gat gcg cat gga ata att ttt atc gat tat ctt gag aag gga aaa      977
Phe Asp Ala His Gly Ile Ile Phe Ile Asp Tyr Leu Glu Lys Gly Lys
            205                 210                 215

acc atc aac agt gac tat tat atg gcg tta ttg gag cgt ttg aag gtc     1025
Thr Ile Asn Ser Asp Tyr Tyr Met Ala Leu Leu Glu Arg Leu Lys Val
        220                 225                 230

gaa atc gcg gca aaa cgg ccc cat atg aag aag aaa aaa gtg ttg ttc     1073
Glu Ile Ala Ala Lys Arg Pro His Met Lys Lys Lys Lys Val Leu Phe
    235                 240                 245

cac caa gac aac gca ccg tgc cac aag tca ttg aga acg atg gca aaa     1121
His Gln Asp Asn Ala Pro Cys His Lys Ser Leu Arg Thr Met Ala Lys
250                 255                 260                 265

att cat gaa ttg ggc ttc gaa ttg ctt ccc cac cca ccg tat tct cca     1169
Ile His Glu Leu Gly Phe Glu Leu Leu Pro His Pro Pro Tyr Ser Pro
                270                 275                 280

gat ctg gcc ccc agc gac ttt ttc ttg ttc tca gac ctc aaa agg atg     1217
Asp Leu Ala Pro Ser Asp Phe Phe Leu Phe Ser Asp Leu Lys Arg Met
            285                 290                 295

ctc gca ggg aaa aaa ttt ggc tgc aat gaa gag gtg atc gcc gaa act     1265
Leu Ala Gly Lys Lys Phe Gly Cys Asn Glu Glu Val Ile Ala Glu Thr
        300                 305                 310

gag gcc tat ttt gag gca aaa ccg aag gag tac tac caa aat ggt atc     1313
Glu Ala Tyr Phe Glu Ala Lys Pro Lys Glu Tyr Tyr Gln Asn Gly Ile
    315                 320                 325

aaa aaa ttg gaa ggt cgt tat aat cgt tgt atc gct ctt gaa ggg aac     1361
Lys Lys Leu Glu Gly Arg Tyr Asn Arg Cys Ile Ala Leu Glu Gly Asn
330                 335                 340                 345

tat gtt gaa taa taaaaacgaa ttttcacaaa aaaatgtgtt tttctttgtt         1413
Tyr Val Glu

agaccgggga cttatcagcc aacctgttat cttgacgaaa aaatgaatgg tcgataaata   1473

atgtgatgtg atccttactg tgttcacttg actggacgaa accgttatga tcaatttgga   1533

tgcctaaaac                                                          1543


<210>  19
<211>  348
<212>  PRT
```

<213> Chrysoperla plorabunda

<400> 19

Met Glu Lys Lys Glu Phe Arg Val Leu Ile Lys Tyr Cys Phe Leu Lys
1 5 10 15

Gly Lys Asn Thr Val Glu Ala Lys Thr Trp Leu Asp Asn Glu Phe Pro
20 25 30

Asp Ser Ala Pro Gly Lys Ser Thr Ile Ile Asp Trp Tyr Ala Lys Phe
35 40 45

Lys Arg Gly Glu Met Ser Thr Glu Asp Gly Glu Arg Ser Gly Arg Pro
50 55 60

Lys Glu Val Val Thr Asp Glu Asn Ile Lys Lys Ile His Lys Met Ile
65 70 75 80

Leu Asn Asp Arg Lys Met Lys Leu Ile Glu Ile Ala Glu Ala Leu Lys
85 90 95

Ile Ser Lys Glu Arg Val Gly His Ile Ile His Gln Tyr Leu Asp Met
100 105 110

Arg Lys Leu Cys Ala Lys Trp Val Pro Arg Glu Leu Thr Phe Asp Gln
115 120 125

Lys Gln Gln Arg Val Asp Asp Ser Glu Arg Cys Leu Gln Leu Leu Thr
130 135 140

Arg Asn Thr Pro Glu Phe Leu Arg Arg Tyr Val Thr Met Asp Glu Thr
145 150 155 160

Trp Leu His His Tyr Thr Pro Glu Ser Lys Arg Gln Ser Ala Glu Trp
165 170 175

Thr Ala Thr Gly Glu Pro Ser Pro Lys Arg Gly Lys Thr Gln Lys Ser
180 185 190

Ala Gly Lys Val Met Ala Ser Val Phe Phe Asp Ala His Gly Ile Ile
195 200 205

Phe Ile Asp Tyr Leu Glu Lys Gly Lys Thr Ile Asn Ser Asp Tyr Tyr
210 215 220

Met Ala Leu Leu Glu Arg Leu Lys Val Glu Ile Ala Ala Lys Arg Pro

```
225                 230                 235                 240

His Met Lys Lys Lys Lys Val Leu Phe His Gln Asp Asn Ala Pro Cys
                245                 250                 255

His Lys Ser Leu Arg Thr Met Ala Lys Ile His Glu Leu Gly Phe Glu
            260                 265                 270

Leu Leu Pro His Pro Pro Tyr Ser Pro Asp Leu Ala Pro Ser Asp Phe
        275                 280                 285

Phe Leu Phe Ser Asp Leu Lys Arg Met Leu Ala Gly Lys Lys Phe Gly
    290                 295                 300

Cys Asn Glu Glu Val Ile Ala Glu Thr Glu Ala Tyr Phe Glu Ala Lys
305                 310                 315                 320

Pro Lys Glu Tyr Tyr Gln Asn Gly Ile Lys Lys Leu Glu Gly Arg Tyr
                325                 330                 335

Asn Arg Cys Ile Ala Leu Glu Gly Asn Tyr Val Glu
            340                 345


<210>  20
<211>  378
<212>  DNA
<213>  Artificial

<220>
<223>  IR/DR-R

<400>  20
aattccatca caaagctctg acctcaatcc tatagaaagg aggaatgagc caaaattcac      60

ccaacttatt gtgggaagct tgtggaaggc tactcgaaat gtttgaccca agttaaacaa     120

tttaaaggca atgctaccaa atactaattg agtgtatgtt aacttctgac ccactgggaa     180

tgtgatgaaa gaaataaaag ctgaaatgaa tcattctctc tactattatt ctgatatttc     240

acattcttaa aataaagtgg tgatcctaac tgaccttaag acagggaatc tttactcgga     300

ttaaatgtca ggaattgtga aaaagtgagt ttaaatgtat ttggctaagg tgtatgtaaa     360

cttccgactt caactgta                                                   378


<210>  21
<211>  354
<212>  DNA
<213>  Artificial

<220>
```

<223> IR/DR-L

<400> 21
ccttgaaata catccacagg tacacctcca attgactcaa atgatgtcaa ttagtctatc 60

agaagcttct aaagccatga catcattttc tggaattttc caagctgttt aaaggcacag 120

tcaacttagt gtatgtaaac ttctgaccca ctggaattgt gatacagtga attataagtg 180

aaataatctg tctgtaaaca attgttggaa aaatgacttg tgtcatgcac aaagtagatg 240

tcctaactga cttgccaaaa ctattgtttg ttaacaagaa atttgtggag tagttgaaaa 300

acgagtttta atgactccaa cttaagtgta tgtaaacttc cgacttcaac tgta 354

<210> 22
<211> 29
<212> DNA
<213> Artificial

<220>
<223> 5' outer repeat

<400> 22
gttcaagtcg gaagtttaca tacacttag 29

<210> 23
<211> 30
<212> DNA
<213> Artificial

<220>
<223> 5' inner repeat

<400> 23
cagtgggtca gaagtttaca tacactaagg 30

<210> 24
<211> 31
<212> DNA
<213> Artificial

<220>
<223> 3' inner repeat

<400> 24
cagtgggtca gaagttaaca tacactcaat t 31

<210> 25
<211> 30
<212> DNA
<213> Artificial

<220>
<223> 3' outer repeat

<400> 25

agttgaatcg gaagtttaca tacaccttag 30

<210> 26
<211> 30
<212> DNA
<213> Artificial

<220>
<223> preferred consensus direct repeat

<400> 26
caktgrgtcr gaagtttaca tacacttaag 30

<210> 27
<211> 26
<212> DNA
<213> Artificial

<220>
<223> inverted repeat sequence

<400> 27
gttgaagtcg gaagtttaca cttagg 26

<210> 28
<211> 31
<212> DNA
<213> Artificial

<220>
<223> inverted repeat sequence

<400> 28
ccagtgggtc aggaagttta catacactaa g 31

<210> 29
<211> 27
<212> DNA
<213> Artificial

<220>
<223> forward sequence used for PCR amplication from pCMV-SB

<400> 29
catgccatgg gaaaatcaaa agaaatc 27

<210> 30
<211> 26
<212> DNA
<213> Artificial

<220>
<223> reverse sequence used for PCR amplication from pCMV-SB

<400> 30
ccgctcgagc agtggcttct tccttg 26

<210> 31
<211> 25
<212> DNA
<213> Artificial

<220>
<223> primer EGFP-1U

<400> 31
caccctcgtg accaccctga cctac 25

<210> 32
<211> 25
<212> DNA
<213> Artificial

<220>
<223> primer EGFP-1L

<400> 32
cttgatgccg ttcttctgct tgtcg 25

<210> 33
<211> 30
<212> DNA
<213> Artificial

<220>
<223> primer HYG-1U

<400> 33
cgggcgtata tgctccccat tggtcttgac 30

<210> 34
<211> 30
<212> DNA
<213> Artificial

<220>
<223> primer TK-1L

<400> 34
tggtgtagat gttcgcgatt gtctcggaag 30

<210> 35
<211> 25
<212> DNA
<213> Artificial

<220>
<223> primer M13F

<400> 35
acgacgttgt aaaacgacgg ccagt 25

<210> 36
<211> 29
<212> DNA
<213> Artificial

<220>
<223> primer RMCE-DL1

<400> 36
gcatcgccat gggtcacgac gagatcctc 29

<210> 37
<211> 30
<212> DNA
<213> Artificial

<220>
<223> RRMCE-IL-1

<400> 37
aagtgagttt aaatgtattt ggctaaggtg 30

<210> 38
<211> 30
<212> DNA
<213> Artificial

<220>
<223> TgTP-2L

<400> 38
acacaggaaa cagctatgac catgattacg 30

<210> 39
<211> 25
<212> DNA
<213> Artificial

<220>
<223> primer neo-U1

<400> 39
gggtggagag gctattcggc tatga 25

<210> 40
<211> 25
<212> DNA
<213> Artificial

<220>
<223> primer neo-L1

<400> 40
tggatacttt ctcggcagga gcaag 25

<210> 41
<211> 23
<212> DNA
<213> Artificial

<220>
<223> probe

<220>
<221> misc_feature
<222> (23)..(23)
<223> connected to FITC

<400> 41
cggccgctct agcggtaccc tac 23

<210> 42
<211> 22
<212> DNA
<213> Artificial

<220>
<223> probe

<220>
<221> misc_feature
<222> (1)..(1)
<223> connected to LCRed640

<400> 42
gtaggggatc gacctcgagg gg 22

<210> 43
<211> 25
<212> DNA
<213> Artificial

<220>
<223> probe

<220>
<221> misc_feature
<222> (1)..(1)
<223> connected to FITC

<400> 43
gctgtgctcg acgttgtcac tgaag 25

<210> 44
<211> 24
<212> DNA
<213> Artificial

<220>
<223> probe

<220>
<221> misc_feature
<222> (1)..(1)
<223> connected to LCRed640

<400> 44
gggaagggac tggctgctat tggg 24

<210> 45
<211> 20
<212> DNA
<213> Artificial

<220>
<223> PCR primer

<400> 45
gttgggtcgt ttgttcggat 20

<210> 46
<211> 20
<212> DNA
<213> Artificial

<220>
<223> PCR primer

<400> 46
cgcgcaatta accctcacta 20

<210> 47
<211> 20
<212> DNA
<213> Artificial

<220>
<223> PCR primer

<400> 47
aatgaactgc aggacgaggc 20

<210> 48
<211> 20
<212> DNA
<213> Artificial

<220>
<223> PCR primer

<400> 48
atggatactt tctcggcagg 20

<210> 49
<211> 30

<212> DNA
<213> Artificial

<220>
<223> primer LCB2XL2

<400> 49
ttccaaaaga agtagagtgg agaaccagtg 30

<210> 50
<211> 23
<212> DNA
<213> Artificial

<220>
<223> primer PGK2

<400> 50
aggccacttg tgtagcgcca agt 23

<210> 51
<211> 30
<212> DNA
<213> Artificial

<220>
<223> primer LCB2XL1

<400> 51
ccaaccaaat acatttaaca tattctaggt 30

<210> 52
<211> 26
<212> DNA
<213> Artificial

<220>
<223> primer PGK4

<400> 52
gctgctaaag cgcatgctcc agactg 26

<210> 53
<211> 30
<212> DNA
<213> Artificial

<220>
<223> forward primer for amlication of SB transposase gene

<400> 53
aatagaactg tttggccata atgaccatcg 30

<210> 54
<211> 25
<212> DNA

<213> Artificial

<220>
<223> reverse primer for amlication of SB transposase gene

<400> 54
atccacataa ttttccttcc tcatg 25

<210> 55
<211> 30
<212> DNA
<213> Artificial

<220>
<223> forward primer for amlication of beta actin tranposase gene

<400> 55
cagggtgtga tggtgggaat gggtcagaag 30

<210> 56
<211> 30
<212> DNA
<213> Artificial

<220>
<223> reverse primer for amlication of beta actin tranposase gene

<400> 56
tacgtacatg gctggggtgt tgaaggtctc 30

<210> 57
<211> 30
<212> DNA
<213> Artificial

<220>
<223> primer beta geo

<400> 57
tgccagtttg aggggacgac gacagtatcg 30

<210> 58
<211> 30
<212> DNA
<213> Artificial

<220>
<223> MS specific primer

<400> 58
tggagtgagc tagaatcaga aagatgacac 30

<210> 59
<211> 30
<212> DNA
<213> Artificial

<220>
<223> M2L specific primer

<400> 59
gactttcaag accttcgacg caccgttcac 30

<210> 60
<211> 30
<212> DNA
<213> Artificial

<220>
<223> M4 specific primer

<400> 60
tcttcagcca caggctccca gacatgacag 30

<210> 61
<211> 30
<212> DNA
<213> Artificial

<220>
<223> N1 specific primer

<400> 61
gatatgaaga gctgtcagtt tgtagcagtc 30

<210> 62
<211> 34
<212> DNA
<213> Artificial

<220>
<223> Unmet-U

<400> 62
tacagttgaa gtcggaagtt tacatacact taag 34

<210> 63
<211> 34
<212> DNA
<213> Artificial

<220>
<223> Unmet-L

<400> 63
cttaagtgta tgtaaacttc cgacttcaac tgta 34

<210> 64
<211> 34
<212> DNA
<213> Artificial

<220>
<223> Met-U

<220>
<221> misc_feature
<222> (13)..(13)
<223> N is 5-methyl cytosine

<400> 64
tacagttgaa gtnggaagtt tacatacact taag 34

<210> 65
<211> 34
<212> DNA
<213> Artificial

<220>
<223> Met-L

<220>
<221> misc_feature
<222> (21)..(21)
<223> N is 5-methyl cytosine

<400> 65
cttaagtgta tgtaaacttc ngacttcaac tgta 34

<210> 66
<211> 25
<212> DNA
<213> Artificial

<220>
<223> forward primer for amylase 2.1 gene

<400> 66
ccttgtacgg gttggtggag gtcac 25

<210> 67
<211> 25
<212> DNA
<213> Artificial

<220>
<223> reverse primer for amylase 2.1 gene

<400> 67
cgccactcga acaggtggac aatag 25

<210> 68
<211> 24
<212> DNA
<213> Artificial

<220>

<223> primer

<400> 68
tgcgaggata agaacagaca ctac 24

<210> 69
<211> 24
<212> DNA
<213> Artificial

<220>
<223> primer

<400> 69
acagactcag aagcaaacgt aaga 24

<210> 70
<211> 24
<212> DNA
<213> Artificial

<220>
<223> forward primer sequence for IR/DR-L

<400> 70
gcacgggtgt tgggtcgttt gttc 24

<210> 71
<211> 27
<212> DNA
<213> Artificial

<220>
<223> reverse primer sequence for IR/DR-L

<400> 71
cttctaaagc catgacatca ttttctg 27

<210> 72
<211> 28
<212> DNA
<213> Artificial

<220>
<223> forward primer sequence for IR/DR-R

<400> 72
gaaggctact cgaaatgttt gacccaag 28

<210> 73
<211> 24
<212> DNA
<213> Artificial

<220>
<223> EGFP-1U primer

<400> 73
cacgctggtg accagcctga ccta 24

<210> 74
<211> 24
<212> DNA
<213> Artificial

<220>
<223> EGFP-1L primer

<400> 74
cttgatgccg ttctctgctt gtcg 24

<210> 75
<211> 24
<212> DNA
<213> Artificial

<220>
<223> SB-2U primer

<400> 75
tcctagagat gaacgtactt tggt 24

<210> 76
<211> 25
<212> DNA
<213> Artificial

<220>
<223> SB-1L primer

<400> 76
atccagataa ttttccttgc tcatg 25